(19) Europäisches Patentamt
European Patent Office
Office européen des brevets

(11) EP 1 756 305 B1

(12) **FASCICULE DE BREVET EUROPEEN**

(45) Date de publication et mention de la délivrance du brevet:
**01.06.2011 Bulletin 2011/22**

(21) Numéro de dépôt: **05741923.6**

(22) Date de dépôt: **17.03.2005**

(51) Int Cl.:
***C12Q 1/68*** *(2006.01)*

(86) Numéro de dépôt international:
**PCT/FR2005/000656**

(87) Numéro de publication internationale:
**WO 2005/093097 (06.10.2005 Gazette 2005/40)**

(54) **APTAMERES SELECTIONNES A PARTIR DE CELLULES VIVANTES TUMORALES ET LEURS APPLICATIONS**

AUS LEBENDIGEN TUMORZELLEN AUSGEWÄHLTE APTAMERE UND DEREN VERWENDUNG

APTAMERS SELECTED FROM LIVE TUMOR CELLS AND THE USE THEREOF

(84) Etats contractants désignés:
**AT BE BG CH CY CZ DE DK EE ES FI FR GB GR HU IE IS IT LI LT LU MC NL PL PT RO SE SI SK TR**

(30) Priorité: **17.03.2004 FR 0402774**

(43) Date de publication de la demande:
**28.02.2007 Bulletin 2007/09**

(73) Titulaire: **Commissariat à l'Énergie Atomique et aux Énergies Alternatives**
**75015 Paris (FR)**

(72) Inventeurs:
- **TAVITIAN, Bertrand**
**F-78210 SAINT-CYR L'ECOLE (FR)**
- **DUCONGE, Frédéric**
**F-92330 SCEAUX (FR)**
- **LIBRI, Domenico**
**F-91190 GIF-SUR-YVETTE (FR)**
- **DE FRANCISCIS, Vittorio**
**I-80134 NAPLES (IT)**
- **CERCHIA, Laura**
**I-80122 NAPLES (IT)**

(74) Mandataire: **Vialle-Presles, Marie José et al**
**Cabinet ORES**
**36, rue de St Pétersbourg**
**75008 Paris (FR)**

(56) Documents cités:
**WO-A-99/51777**
**WO-A-2004/011680**
**US-A- 5 580 737**
**US-A1- 2003 049 644**
**US-B1- 6 280 932**

- **ICHIHARA MASATOSHI ET AL: "RET and neuroendocrine tumors." CANCER LETTERS, vol. 204, no. 2, 20 février 2004 (2004-02-20), pages 197-211, XP002303439 ISSN: 0304-3835**
- **CHEN CHI-HONG B ET AL: "Inhibition of heregulin signaling by an aptamer that preferentially binds to the oligomeric form of human epidermal growth factor receptor-3." PROCEEDINGS OF THE NATIONAL ACADEMY OF SCIENCES OF THE UNITED STATES OF AMERICA, vol. 100, no. 16, 5 août 2003 (2003-08-05), pages 9226-9231, XP002303440 ISSN: 0027-8424**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

SEQUENCE LISTING

<110> COMMISSARIAT A L'ENERGIE ATOMIQUE
TAVITIAN Bertrand
DUCONGE Frédéric
LIBRI Domenico
DE FRANCISCIS Vittorio
CERCHIA Laura

<120> APTAMERES SELECTIONNES A PARTIR DE CELLULES VIVANTES TUMORALES ET LEURS APPLICATIONS

<130> F263/107ext

<160> 33

<170> PatentIn version 3.1

<210> 1
<211> 23
<212> RNA
<213> Artificial sequence

<220>
<223> R1
<400> 1
gggagacaag aauaaacgcu caa 23

<210> 2
<211> 24
<212> RNA
<213> Artificial sequence

<220>
<223> R2
<400> 2
aacgacagga ggcucacaac agga 24

<210> 3
<211> 50
<212> RNA
<213> Artificial sequence

<220>
<223> R de D4
<400> 3
gcgcgggaau aguauggaag gauacguaua ccgugcaauc cagggcaacg 50

<210> 4
<211> 50
<212> RNA
<213> Artificial sequence

<220>
<223> R de D12
<400> 4
gggcuucaua agcuacaccg gccaacgcag aaaugccuua agcccgaguu 50

<210> 5
<211> 50
<212> RNA
<213> Artificial sequence

<220>
<223> R de D14
<400> 5
ggccauagcg caccaccaag agcaaauccc uaagcgcgac ucgagugagc 50

<210> 6
<211> 50
<212> RNA
<213> Artificial sequence

<220>
<223> R de D20
<400> 6
gggccaaucg aagccgguaa uucccaaacu aacgugcaaa cugcacccgc 50

<210> 7
<211> 49
<212> RNA
<213> Artificial sequence

<220>
<223> R de D24
<400> 7
gcgguaugua gggaauagca cuuuuuuugc guauaccuac accgcagcg 49

<210> 8
<211> 50
<212> RNA
<213> Artificial sequence

<220>
<223> R de D30
<400> 8
aggcgagccc gaccacguca guaugcuaga caacaacgcc cgcgugguac 50

<210> 9
<211> 51
<212> RNA
<213> Artificial sequence

<220>
<223> R de D32
<400> 9
ccccgcuuuu ugacgugauc gaacgcguau caguaacguc agcagucgag c 51

<210> 10
<211> 51
<212> RNA
<213> Artificial sequence

<220>
<223> R de D33
<400> 10 51
caaagcgugu auucucguga gccgaccauc guugcgaaca uccccggaac g 51

<210> 11
<211> 48
<212> RNA
<213> Artificial sequence

<220>
<223> R de D42
<400> 11

gacccguaug aagguggcgc aggacacgac cgucugcaau gagcgagc 48

<210> 12
<211> 50
<212> RNA
<213> Artificial sequence

<220>
<223> R de D60
<400> 12
ccgaccugua cagcaguuag uuacacguuu gaaacaaccg gcguucgagc 50

<210> 13
<211> 50
<212> RNA
<213> Artificial sequence

<220>
<223> R de D76
<400> 13
ggcuuacacg gagaaacaag agagcggccc aaacuugauu gacaguggcc 50

<210> 14
<211> 49
<212> RNA
<213> Artificial sequence

<220>
<223> R de D71
<400> 14
ggcccuuaac gcaaaaacga aggaucaucg auugaucgcc uuaugggcu 49

<210> 15
<211> 48
<212> RNA
<213> Artificial sequence

<220>
<223> R de D87
<400> 15
ccgcggucug ugggacccuu caggaugaag cggcaaccca ugcgggcc 48

<210> 16
<211> 40
<212> DNA
<213> Artificial sequence

<220>
<223> amorce PCR
<400> 16
taatacgact cactataggg agacaagaat aaacgctcaa 40

<210> 17
<211> 24
<212> DNA
<213> Artificial sequence

<220>

<223> amorce PCR
<400> 17
tcctgttgtg agcctcctgt cgtt 24

<210> 18
<211> 24
<212> RNA
<213> Artificial sequence

<220>
<223> R1 + Z1
<400> 18
gggagacaag aauaaacgcu caag 24

<210> 19
<211> 30
<212> RNA
<213> Artificial sequence

<220>
<223> R1 + Z1
<400> 19
gggagacaag aauaaacgcu caagcgguau 30

<210> 20
<211> 39
<212> RNA
<213> Artificial sequence

<220>
<223> Z2 + R2
<400> 20
caauccaggg caacgaacga caggaggcuc acaacagga 39

<210> 21
<211> 33
<212> RNA
<213> Artificial sequence

<220>
<223> Z2 + R2
<400> 21
accgcagcga acgacaggag gcucacaaca gga 33

<210> 22
<211> 97
<212> RNA
<213> Artificial sequence

<220>
<223> aptamère: famille D4 (figure 11)
<400> 22

gggagacaag aauaaacgcu caagcgcggg aauaguaugg aaggauacgu auaccgugca 60
auccagggca acgaacgaca ggaggcucac aacagga 97

<210> 23
<211> 34
<212> RNA
<213> Artificial sequence

<220>
<223> aptamère: famille D4
<400> 23
cgcgggaaua guauggaagg auacguauac cgug 34

<210> 24
<211> 24
<212> RNA
<213> Artificial sequence

<220>
<223> aptamère
<400> 24
guagggaaua gcacguauac cuac 24

<210> 25
<211> 96
<212> RNA
<213> Artificial sequence

<220>
<223> aptamère: famille D24 (figure 12)
<400> 25

gggagacaag aauaaacgcu caagcgguau guagggaaua gcacuuuuuu ugcguauacc 60
uacaccgcag cgaacgacag gaggcucaca acagga 96

<210> 26
<211> 7
<212> RNA
<213> Artificial sequence

<220>
<223> Z1
<400> 26
gcgguau 7

<210> 27
<211> 15
<212> RNA
<213> Artificial sequence

<220>
<223> Z2
<400> 27
caauccaggg caacg 15

<210> 28
<211> 9
<212> RNA
<213> Artificial sequence

<220>
<223> Z2
<400> 28
accgcagcg 9

<210> 29
<211> 8
<212> RNA
<213> Artificial sequence

<220>
<223> boucle 1
<400> 29 8
uggaagga

<210> 30
<211> 7
<212> RNA
<213> Artificial sequence

<220>
<223> boucle 2
<400> 30
cuuuuuu 7

<210> 31
<211> 97
<212> RNA
<213> Artificial sequence

<220>
<223> aptamère: famille D30 (figure 13)
<400> 31

gggagacaag aauaaacgcu caaaggcgag cccgaccacg ucaguaugcu agacaacaac 60
gcccgcgugg uacaacgaca ggaggcucac aacagga 97

<210> 32
<211> 97
<212> RNA
<213> Artificial sequence

<220>
<223> aptamère: famille D12 (figure 14)
<400> 32

gggagacaag aauaaacgcu caagggcuuc auaagcuaca ccggccaacg cagaaaugcc 60
uuaagcccga guuaacgaca ggaggcucac aacagga 97

<210> 33
<211> 96
<212> RNA
<213> Artificial sequence

<220>
<223> aptamère: famille D71 (figure 15)
<400> 33

gggagacaag aauaaacgcu caaggcccuu aacgcaaaaa cgaaggauca ucgauugauc 60
gccuuauggg cuaacgacag gaggcucaca acagga 96

**Description**

La présente invention est relative à des aptamères sélectionnés à partir de cellules vivantes tumorales et à leurs applications dans le diagnostic et le traitement de certains cancers ainsi que d'autres pathologies.

Dans le domaine de l'oncologie, les méthodes diagnostiques non invasives par imagerie *in vivo* (radiographie, scanner X, IRM, gamma-scintigraphie, tomographie par émission de positons) sont rarement spécifiques d'un déterminant moléculaire (ou marqueur), caractéristique de la tumeur à diagnostiquer ou à traiter. Ceci contraste avec la précision des connaissances obtenues *in vitro,* qui décrivent de façon de plus en plus fine les anomalies moléculaires à l'origine des processus cancéreux, et conduit à une mauvaise adéquation des outils diagnostiques aux données actuelles de la science moléculaire. La même dichotomie entre *in vitro* et *in vivo* se retrouve souvent dans le domaine des thérapies anticancéreuses, ce qui conduit à des difficultés dans la mise au point de traitements avec un index thérapeutique (dose efficace/dose toxique) acceptable.

La recherche de ligands capables de reconnaître un déterminant moléculaire (ou marqueur) signant un type de tumeur particulier, ou bien un stade donné de son développement, ou encore signalant l'état métabolique d'une tumeur, est donc essentielle pour un meilleur suivi et une meilleure thérapie des cancers. Malheureusement, ces ligands sont généralement obtenus à partir de cibles purifiées et isolées hors de leur contexte biologique, et donc différentes des cibles placées dans leur environnement naturel.

Ainsi, le plus souvent, les ligands, efficaces en tube à essai, sont incapables d'interagir avec leur cible :

- parce qu'ils ne peuvent pas franchir les barrières tissulaires,
- parce qu'ils sont instables dans l'organisme, ou responsables de trop d'interactions non désirables avec d'autres biomolécules,
- parce que la structure native de la cible, placée hors de son environnement cellulaire naturel, n'a pas été conservée ou que certaines modifications essentielles de cette structure telles que : (i) modifications post-traductionnelles des protéines ou (ii) interactions avec d'autres protéines, ne peuvent être reproduites *in vitro.*

Ces deux dernières limitations sont particulièrement fréquentes dans le cas de cibles telles que les protéines transmembranaires comportant un segment lipophile inséré dans la membrane cellulaire lipidique ; ce segment lipophile n'est pas conservé *in vitro* alors que l'insertion membranaire de ces protéines détermine leur structure et est indispensable à leur activité.

D'autre part, se pose le problème de la spécificité avec laquelle les ligands disponibles reconnaissent les cibles identifiées dans les tumeurs. Il est donc important de pouvoir disposer de ligands spécifiques pour diagnostiquer et/ou traiter certains cancers, notamment ceux liés à la présence d'un récepteur à activité tyrosine kinase muté, conduisant à une activation constitutive ou à une sur-expression de ce récepteur.

La médecine moléculaire a donc besoin de nouvelles sondes de reconnaissance moléculaire :

- spécifiques,
- ajustables, et
- faciles à produire à un coût raisonnable.

La recherche pharmacologique a mis en place de nouvelles stratégies afin de découvrir de nouveaux ligands efficaces contre des cibles identifiées dans les tumeurs :

- les banques combinatoires de petites molécules permettent d'augmenter les chances de trouver un ligand contre une protéine particulière (exemple : une sous-classe de banques combinatoires, les faux substrats inhibiteurs d'enzymes comme ceux inhibant les MMP (*matrix metalloproteases*)). Leur inconvénient majeur est qu'elles sont triées *in vitro.* Par ailleurs, leur sélectivité n'est pas garantie, d'où la difficulté d'obtenir, avec ces agents, un composé suffisamment sélectif pour être efficace et dépourvu d'effets secondaires. On n'obtient qu'exceptionnellement des composés spécifiques d'une forme anormale de protéine, ce qui entraîne une fixation non spécifique et des effets indésirables et conduit à un mauvais index thérapeutique.
- les anticorps monoclonaux sont d'excellents agents de ciblage spécifique et ont été récemment appliqués à des fins thérapeutiques (exemple : Herceptin dans le cancer du sein) ; cependant ils restent très malaisés à utiliser *in vivo* du fait de leur taille, idiotypie, et immunogénicité et sont extrêmement coûteux à produire et à optimiser. D'autre part, les anticorps monoclonaux atteignent leurs limites lorsqu'il s'agit de reconnaître une mutation ponctuelle affectant un seul acide aminé sur une protéine. En particulier, il n'existe pas d'anticorps monoclonal capable d'identifier l'une des formes anormales de la protéine Ret.
- les aptamères, qui constituent un moyen de diagnostic et de thérapie alternatif et qui présentent un certain nombre d'avantages par rapport aux anticorps, comme illustré dans le Tableau I ci-après.

| | Aptamères | Anticorps |
|---|---|---|
| Taille | 9-15 kDa | >150 kDa |
| discrimination* | +++ | +/++ |
| affinité | 5-100 nM | 0,1-100 nM |
| Source | synthétique | Animal |
| Prix | ↘ | ↗ |
| *théophylline vs. Caféine, par exemple. | | |

Une méthode de sélection d'aptamères, se liant spécifiquement et ayant une forte affinité pour des cibles prédéfinies, a été décrite au début des années 1990, sous le nom de méthode SELEX (***S**ystematic **E**volution of **L**igands by **Ex**ponential enrichment*). Cette méthode fonctionne par cycles itératifs de sélection-amplification ; cette méthode ainsi qu'un certain nombre de perfectionnements et d'applications de cette méthode sont décrits notamment dans les Brevets américains suivants: US 5,270,163 ; US 5,475,096 ; US 5,496,938 ; US 5,567,588 ; US 5,580,737 ; US 5,637,459 ; US 5,660,985 ; US 5,683,867 ; US 5,707,796, US 5,763,177 et US 5,789,157.

Brièvement, le principe de la méthode SELEX implique la sélection, à partir d'un mélange d'acides nucléiques comprenant des séquences aléatoires, et par réitérations successives d'étapes de liaison, de séparation et d'amplification, de molécules d'acides nucléiques (aptamères) présentant une affinité de liaison et une spécificité définies pour une cible donnée. Ainsi, en partant d'un mélange d'acides nucléiques aléatoires, la méthode SELEX comprend plus précisément les étapes suivantes :

- mise en contact du mélange d'acides nucléique avec l'élément cible (polymères naturels ou synthétiques : protéines, polysaccharides, glycoprotéines, hormones, récepteurs, antigènes, anticorps, surfaces cellulaires ; petites molécules : médicament, métabolites, cofacteurs, substrats, analogues d'états de transition ; tissus et notamment cellules entières ; virus, etc...), dans des conditions favorables à la liaison,
- séparation des séquences non liées
- dissociation des complexes acide nucléique-élément cible
- amplification des acides nucléiques dissociés, pour obtenir un mélange enrichi en ligands (aptamères) et
- réitération des étapes de liaison, de séparation, de dissociation et d'amplification, le nombre de fois désirées.

Parmi les Brevets américains précités :

- le Brevet US 5,270,163 décrit le principe initial de la méthode SELEX ;
- le Brevet US 5,580,737 décrit une application de cette méthode, comprenant une étape de contre sélection, à la sélection d'aptamères aptes à discriminer entre deux substances de structure proches, à savoir la caféine et la théophylline ;
- le Brevet US 5,660,985 décrit une application de cette méthode à la sélection d'aptamères modifiés comportant des nucléotides modifiés au niveau des pyrimidines (modification en position 5 ou en position 2') ;
- le Brevet US 5,789,157 décrit une application de cette méthode à la sélection d'aptamères aptes à se lier à des protéines présentes à la surface de tissus et notamment de cellules ; ce Brevet préconise en particulier d'appliquer la méthode SELEX, à des cibles particulières, à savoir des tissus et notamment des cellules tumorales, qui sont considérées comme des cibles plus complexes que celles mises en oeuvre dans les autres Brevets cités et qui sont généralement des cibles moléculairement identifiées (protéines etc..). Ce Brevet US 5,789,157 précise en outre qu'il est possible d'utiliser une contre sélection (ou sélection négative), avant, pendant ou après la mise en oeuvre de la méthode SELEX. La sélection négative plus précisément décrite dans le Brevet US 5,580,737, permet de discriminer entre des types de tissus différents mais néanmoins très proches. Par exemple, la sélection négative peut être utilisée pour identifier les ligands qui présentent une spécificité élevée pour des cellules tumorales et non vis-à-vis des cellules normales correspondantes. Ce Brevet prévoit également le cas où les acides nucléiques ligands d'un type cellulaire qui exprime un certain récepteur peut être contre sélectionné avec une lignée cellulaire construite de telle sorte qu'elle n'exprime pas ledit récepteur ; toutefois, l'utilisation d'une telle stratégie peut être difficile à mettre en oeuvre si la protéine induit un changement phénotypique important de la cellule.
- le Brevet US 6,232,071 décrit une application de cette méthode à la sélection d'aptamères spécifiques de la ténascine C.

De manière surprenante, le Demandeur a trouvé qu'il était possible d'obtenir des aptamères spécifiques de récepteurs cellulaires et plus particulièrement de marqueurs tumoraux, en mettant en oeuvre le procédé dénommé SELEX, sur des cellules cibles vivantes, dans certaines conditions.

En conséquence, selon un premier aspect, la présente invention a pour objet un procédé d'identification de ligands ou aptamères spécifiques d'un récepteur membranaire à activité tyrosine kinase (RPTK pour *receptor protein-tyrosine kinase*) exprimé sous une forme activée par des cellules (et ce quelle que soit l'origine ou la cause de l'activation) ou non (de préférence sous une forme activée), à partir d'un mélange d'acides nucléiques, lequel procédé comprend au moins les étapes suivantes :

(a) la mise en contact d'un mélange d'acides nucléiques avec des cellules n'exprimant pas ledit récepteur membranaire à activité tyrosine kinase ou l'exprimant sous une forme non activée, (cellules $C_N$), lesdites cellules présentant le même type cellulaire que des cellules exprimant le même récepteur membranaire à activité tyrosine kinase mais sous une forme activée, du fait de l'existence d'au moins une mutation dans le domaine extracellulaire (cellules $C_{Te}$) ;
(b) la récupération d'un premier sous-ensemble S1 d'acides nucléiques qui ne se lient pas aux cellules $C_N$, lors de l'étape (a) ;
(c) la mise en contact dudit premier sous-ensemble S1 avec des cellules $C_i$, présentant le même type cellulaire que les cellules $C_{Te}$, mais exprimant ledit récepteur membranaire à activité tyrosine kinase, muté dans sa partie intracellulaire, lesdites cellules $C_i$ présentant un phénotype du même type que celui des cellules $C_{Te}$ ;
(d) la récupération d'un deuxième sous-ensemble S2 d'acides nucléiques qui ne se lient pas aux cellules $C_i$ lors de l'étape (c) ;
(e) la mise en contact du deuxième sous-ensemble S2 avec les cellules $C_{Te}$ ;
(f) la récupération des acides nucléiques qui se lient auxdites cellules $C_{Te}$, c'est-à-dire ceux présentant une affinité élevée vis-à-vis des cellules exprimant ledit récepteur membranaire à activité tyrosine kinase muté dans le domaine extracellulaire (récepteur activé), après dissociation des complexes cellules-acides nucléiques ;
(g) l'amplification desdits acides nucléiques à affinité élevée pour les cellules exprimant ledit récepteur membranaire à activité tyrosine kinase muté dans le domaine extracellulaire (récepteur activé), de manière à obtenir un mélange d'acides nucléiques, enrichi en acides nucléiques ayant une affinité élevée pour lesdites cellules $C_{Te}$ et
(h) l'identification des ligands ou aptamères spécifiques des cellules exprimant des récepteurs membranaires à activité tyrosine kinase (RPTK pour *receptor protein-kinase tyrosine*) sous une forme activée, à partir du mélange obtenu en (g).

De manière surprenante, un tel procédé même s'il comprend des étapes d'exclusion d'aptamères se liant à des formes non activées de RPTK, permet de sélectionner des aptamères spécifiques de RPTK, à savoir, soit des aptamères capables de se lier audit RPTK et d'inhiber l'activité dudit RPTK (activation de la cascade de kinases), soit des aptamères capables uniquement de se lier audit RPTK (intérêt dans des applications en imagerie).

Définitions

- Récepteurs à activité tyrosine kinase (RPTK)
  Les récepteurs membranaires à activité tyrosine kinase (RPTK pour *receptor protein-tyrosine kinase*) constituent une très grande famille de protéines. Il existe actuellement plus de 90 gènes connus codant pour des protéines tyrosine kinases (PTK ou *PKT*), dans le génome humain (Blume-Jensen P. et al., Nature, 2001, 411, 355-365): 58 codent pour des RPTK transmembranaires réparties dans 20 familles et 32 codent pour des PKT cytoplasmiques. Parmi les récepteurs humains à activité tyrosine kinase (RPTK), impliqués dans des cancers, on peut citer les familles suivantes : EGFR (*Epithelial Growth Factor Receptor*), InsulinR (*Insulin Receptor*), PDGFR (*Platelet-derived Growth Factor Receptor*), VEGFR (*Vascular Endothelial Growth Factor Receptor*), FGFR (*Fibroblast Growth Factor Receptor*), NGFR (*Nerve Growth Factor Receptor*), HGFR (*Hepatocyte Growth Factor Receptor*), EPHR (*Ephrin Receptor*), AXL (Tyro 3 PTK), TIE (*Tyrosine Kinase Receptor in endothelial cells*), RET (*Rearranged During Transfection*), ROS (RPTK exprimé dans certaines cellules épithéliales) et LTK (*Leukocyte Tyrosine Kinase*). Dans la suite, le terme RPTK utilisé sans autre précision implique n'importe quel récepteur (activé ou non ; dans une forme activée ou non).
- RPTK non activé
  Dans sa forme normale, ledit récepteur n'est pas activé ; on n'observe une activation qu'après stimulation convenable des cellules exprimant ledit récepteur normal (RPTK activé après stimulation).
- RPTK muté dans le domaine extracellulaire (récepteur activé)

Dans certaines formes anormales, dues à des mutations dans le domaine extracellulaire du récepteur à activité tyrosine kinase, (une ou plusieurs mutations ponctuelles, insertions, délétions et/ou réarrangements), on observe une activation constitutive ou une surexpression du récepteur ; un tel récepteur activé muté dans la partie extracellulaire, est activateur constitutif de la cascade de kinases.

- RPTK muté dans le domaine intracellulaire
  Un tel récepteur peut activer certaines cascades intracellulaires, ; il n'est pas considéré, au sens de la présente invention, comme un récepteur activé ; par contre, il est inclus dans la définition des récepteurs dans ou sous une forme activée.
- RPTK dans ou sous une forme activée
  On entend par récepteur dans ou sous une forme activée, un RPTK activateur de la cascade de kinases, quelle qu'en soit la raison :

  . activation par stimulation par un facteur de croissance (activation normale, dans certaines conditions)
  . activation constitutive ou surexpression du récepteur, du fait de l'existence d'une ou plusieurs mutations, soit dans la partie extracellulaire, soit dans la partie intracellulaire dudit récepteur.

De manière surprenante, les conditions particulières du procédé selon l'invention permettent effectivement de sélectionner et d'identifier des ligands ou aptamères spécifiques du ou des récepteurs membranaires à activité tyrosine kinase présélectionnés, c'est-à-dire se liant audit récepteur et en outre, parmi ceux-ci, de sélectionner ceux capables d'inhiber lesdits récepteurs dans leur forme activée. En effet, la sélection des aptamères avec des cellules $C_N$, $C_{Te}$ et $C_i$, telles que définies ci-dessus, permet effectivement d'obtenir, notamment après réitération des étapes a) à g), des aptamères spécifiques des cellules exprimant des récepteurs membranaires à activité tyrosine kinase (RPTK) présélectionnés.

Conformément à l'invention, plusieurs cycles d'étapes (a) à (g) peuvent avantageusement être réitérés en utilisant les mélanges enrichis en ligands ou aptamères du cycle précédent, jusqu'à l'obtention d'au moins un aptamère dont l'affinité, définie par sa constante de dissociation (Kd), est mesurable et convient pour une utilisation pharmaceutique.

Egalement conformément à l'invention :

. la banque combinatoire d'acides nucléiques de départ est avantageusement constituée d'oligonucléotides comprenant des séquences aléatoires, du même type que celles décrites dans les Brevets SELEX précités. Elle contient au moins $10^2$ acides nucléiques, de préférence entre $10^9$ et $10^{15}$ acides nucléiques; les acides nucléiques constituant ladite banque combinatoire sont de préférence des séquences d'acides nucléiques naturelles (ARN ou ADN) ou modifiées (par exemple pyrimidines modifiées par un atome de fluor en position 2' du ribose) constituées de séquences aléatoires comprenant respectivement à leurs extrémités 5' et 3' des séquences fixes permettant une amplification par PCR, de préférence les oligonucléotides de séquences SEQ ID NO :1 et SEQ ID NO :2, ou un fragment d'au moins 8 nucléotides de ces oligonucléotides. Lesdites séquences aléatoires contiennent chacune entre 10 et 1000 nucléotides, de préférence 50 nucléotides.

. les aptamères sélectionnés sont définis par leur séquence primaire ainsi que par leur structure secondaire ; cette dernière se présente soit sous forme de boucle en épingle à cheveux, soit sous forme de structures plus complexes (par exemple : pseudo-noeud, triple hélice, quartet de Guanine...). Ils sont avantageusement constitués de préférence par des séquences d'acides nucléiques de 20 à 100 nucléotides.

De manière avantageuse, les conditions décrites ci-dessus permettent d'identifier des ligands ou aptamères contre des déterminants moléculaires (ou marqueurs) de pathologies, susceptibles d'être effectivement efficaces dans les conditions même de leur utilisation future, c'est-à-dire *in vivo.*

Ainsi, en sélectionnant contre une cible particulière, à savoir les cellules $C_N$, $C_i$ et $C_{Te}$, telles que définies ci-dessus, on obtient effectivement des aptamères qui reconnaissent, de manière spécifique, les cellules exprimant un récepteur membranaire à activité tyrosine kinase présélectionné, notamment sous sa forme activée.

Selon un mode de mise en oeuvre avantageux dudit procédé, l'identification des ligands ou aptamères spécifiques des cellules $C_{Te}$ selon l'étape (h) comporte une évaluation de l'activité biologique desdits aptamères sur lesdites cellules $C_{Te}$.

Les activités biologiques, qui sont avantageusement évaluées, dépendent du récepteur sélectionné, elles sont notamment les suivantes :

(a) inhibition ou activation de l'auto-phosphorylation du récepteur (RPTK),
(b) inhibition ou activation de la cascade d'activation de kinases,
(c) inhibition de la phosphorylation du RPTK normal de cellules normales (cellules $C_N$) activées par stimulation convenable (facteur de croissance approprié, par exemple),

(d) réversion du phénotype associé à une activation du RPTK.

Au sens de la présente invention, les termes suivants sont considérés comme équivalents : fragment d'acide nucléique, oligonucléotide, ligand ou aptamère.

Selon un deuxième aspect, la présente invention a pour objet des ligands ou aptamères, caractérisés en ce qu'ils sont spécifiques des cellules exprimant un récepteur membranaire à activité tyrosine kinase (RPTK) sous une forme activée ou non (de préférence sous une forme activée), notamment un RPTK muté dans le domaine extracellulaire et sont susceptibles d'être identifiés par le procédé d'identification d'aptamères, tel que défini ci-dessus.

Selon un mode de réalisation avantageux dudit aptamère, il est spécifique des cellules exprimant un récepteur membranaire à activité tyrosine kinase (RPTK) sous une forme activée ou non, sélectionné notamment dans le groupe constitué par les récepteurs membranaires suivants, donnés à titre d'exemples non limitatifs : EGFR (*Epithelial Growth Factor Receptor*), InsulinR ((*Insulin Receptor*), PDGFR (*Platelet-derived Growth Factor Receptor*), VEGFR (*Vascular Endothelial Growth Factor Receptor*), FGFR (*Fibroblast Growth Factor Receptor*), NGFR (*Nerve Growth Factor Receptor*), HGFR (*Hepatocyte Growth Factor Receptor*), EPHR (*Ephrin Receptor*), AXL (Tyro 3 PTK), TIE (*Tyrosine Kinase Receptor in endothelial cells*), RET (*Remoranged During Transfection*), ROS (RPTK exprimé dans certaines cellules épithéliales) et LTK (*Leukocyte Tyrosine Kinase*).

Selon une disposition avantageuse de ce mode de réalisation, ledit aptamère reconnaît en particulier le récepteur Ret activé par mutation au niveau d'une cystéine localisée dans le domaine extracellulaire, de préférence au niveau des codons 609, 611, 618, 620 ou 634.

Selon une modalité préférée de cette disposition, ledit aptamère est susceptible d'être identifié par un procédé, tel que défini ci-dessus, qui comprend :

(a) la mise en contact d'un mélange d'acides nucléiques avec des cellules $C_N$ n'exprimant pas de récepteur Ret sous une forme activée,
(b) la récupération d'un premier sous-ensemble S1 d'acides nucléiques qui ne se lient pas auxdites cellules $C_N$, lors de l'étape (a),
(c) la mise en contact dudit premier sous-ensemble S1 avec des cellules $C_i$ exprimant un récepteur Ret, muté dans son domaine intracellulaire, notamment le récepteur muté $Ret^{M918T}$,
d) la récupération d'un deuxième sous-ensemble S2 d'acides nucléiques qui ne se lient pas auxdites cellules $C_i$,
e) la mise en contact du deuxième sous-ensemble S2 avec des cellules $C_{Te}$ exprimant un récepteur Ret activé par mutation dans le domaine extracellulaire, lequel récepteur est sélectionné dans le groupe constitué par les récepteurs Ret mutés portant une mutation sur l'une des cystéines localisées dans le domaine extracellulaire, de préférence au niveau des Cys609, Cys611, Cys618, Cys620 ou Cys 634, de préférence le récepteur $Ret^{C634Y}$,
f) la récupération des acides nucléiques liés auxdites cellules $C_{Te}$, c'est-à-dire présentant à la fois une affinité élevée et une spécificité de liaison pour les cellules exprimant un récepteur Ret muté (récepteur activé) tel que défini à l'étape e),
g) l'amplification desdits acides nucléiques obtenus à l'étape f), de manière à obtenir un mélange d'acides nucléiques, enrichi en acides nucléiques ayant une affinité élevée pour les cellules $C_{Te}$,
h) la répétition des étapes a)-g), jusqu'à l'obtention d'au moins un aptamère dont l'affinité pour les cellules $C_{Te}$, définie par sa constante de dissociation (Kd), est mesurable et convient pour une activité pharmacologique, et
i) l'identification des aptamères spécifiques des cellules exprimant un récepteur Ret dans sa forme activée, sélectionné à partir du mélange obtenu en h).

Le cycle d'obtention des aptamères, selon l'invention, appliqué au récepteur Ret, est illustré à la figure 1.

L'oncogène Ret (*rearranged during transfection*) code pour une forme anormale d'une protéine de surface de type récepteur de la famille des tyrosine-kinases ; ce proto-oncogène est localisé au niveau du chromosome 10q11.2. Les mutations dans le proto-oncogène Ret sont associées à des maladies disparates, notamment la maladie de Hirschsprung et les néoplasies endocrines multiples de type II (*multiple endocrine neoplasia type* II ou MEN 2), qui incluent la MEN de type 2A (MEN 2A), la MEN de type 2B (MEN 2B) et le carcinome thyroïdien médullaire familial (*familial medullary thyroid cancer* ou FMTC). La MEN 2A est caractérisée par un carcinome thyroïdien médullaire, un phéochromocytome et une hyperplasie parathyroïdienne (hyperparathyroïdie primaire). La MEN 2B est caractérisée par une forme particulièrement agressive du cancer médullaire de la thyroïde, un phéochromocytome, des neurogliomes mucosaux multiples et des ganglionévromatoses intestinales. Une forme tronquée de *ret* code pour une protéine intracellulaire associée au cancer papillaire de la thyroïde (PTC).

Les oncogènes sont des formes mutées des proto-oncogènes, qui sont des protéines normales dont la fonction est de contrôler la croissance et la division des cellules, notamment après activation par des facteurs de croissance appropriés (de type, par exemple, du GDNF pour le proto-oncogène codant pour le récepteur Ret). Certaines mutations de ces proto-oncogènes conduisent à des formes actives en permanence de ces protéines, même en l'absence de stimulation par le ou les facteurs de croissance habituels (dérégulation). Cette activation constitutive (permanente) conduit à une stimulation permanente de la croissance et de la division cellulaire, et à terme à une cancérisation. On parle alors d'oncogène activateur de tumeurs pour la forme mutée du proto-oncogène. Les oncogènes peuvent induire une cancérisation, par exemple, par une surproduction de facteurs de croissance, ou par une inondation de la cellule par des signaux de réplication, ou par une stimulation incontrôlée des voies intermédiaires, ou par une croissance désordonnée des cellules, liée à un taux élevé de facteurs de transcription. Certains oncogènes sont transmis de générations en générations, lorsque le proto-oncogène mute dans les cellules germinales. Ceci implique une prédisposition tumorale héritée et dominante. Par exemple, la néoplasie endocrine multiple de type II (*multiple endocrine neoplasia type* II ou MEN 2) est le résultat d'une transmission germinale de l'oncogène Ret activé.

Des mutations dans les exons 10 et 11 du proto-oncogène sont observées dans plus de 95 % des cas de MEN 2A et dans plus de 80 % des cas de MTC ; la plupart de ces mutations sont localisées au niveau de cinq cystéines conservées, localisées dans le domaine extracellulaire (codons 609, 611, 618, 620 et 634). Ces mutants du récepteur Ret forment spontanément des homodimères actifs à la surface de la cellule, qui induisent des changements morphologiques et biochimiques, conduisant à un phénotype de type phéochromocytome, dépendant du récepteur Ret dans les syndromes MEN2 ; les mutations au niveau du codon 634 sont les plus fréquentes dans MEN 2A. L'activation du récepteur Ret peut être suivie à l'aide de la cascade de phosphorylation (Jhiang SM, Oncogene, 2000, 19, 5590-5597 ; Califano D et al, PNAS, 1996, 93, 7933-7937).

En ce qui concerne MEN 2B, une mutation ponctuelle dans l'exon 16 du proto-oncogène au niveau du codon 918 du gène *ret* a été identifiée dans environ 95 % des cas ; cette mutation entraîne la substitution d'une thréonine par une méthionine, au niveau du domaine catalytique du récepteur Ret. Cette mutation entraîne l'activation du récepteur sous la forme d'un monomère.

De manière surprenante, la sélection des aptamères avec des cellules $C_N$, $C_{Te}$ et $C_i$, telles que définies ci-dessus, permet effectivement d'obtenir, après réitération des étapes a) à g), des aptamères spécifiques de la forme humaine des récepteurs Ret et notamment des récepteurs Ret sous leur forme activée, par exemple les récepteurs mutés $Ret^{C634Y}$, exprimés par les cellules $C_{Te}$, les récepteurs normaux, activés ou non, ou des récepteurs mutés dans la partie intracellulaire (voir définitions).

Conformément à l'invention :

- les cellules $C_N$ sont notamment des cellules PC12 sauvages (référence ECACC N° 88022) ou des cellules NIH 3T3 sauvages (référence ECACC N° 93061524),
- les cellules $C_i$ et $C_{Te}$ sont obtenues en introduisant un oncogène porteur d'une mutation, respectivement intracellulaire ou extracellulaire (figure 2), dans des cellules $C_N$ en culture de façon à ce que celles-ci expriment l'oncogène ; dans le contexte de l'invention, on obtient un phénotype transformé (figure 3) similaire dans les deux cas. Les cellules PC12 passent d'une forme ronde peu adhérente à une forme allongée avec des pseudo-neurites qui présente de bonnes capacités d'adhérence (Califano D et al, PNAS, 1996, 93, 7933-7937).

On peut alors réaliser sélection et contre sélection sur des phénotypes quasiment identiques qui ne différent que par la localisation cellulaire de la mutation oncogénique (figure 1) ; plus précisément, les cellules $C_i$ obtenues sont dénommées cellules PC12/MEN 2B (ou NIH/MEN 2B) et les cellules $C_{Te}$ obtenues sont dénommées cellules PC 12/MEN 2A (ou NIH/MEN 2A).

De manière avantageuse, les conditions décrites ci-dessus permettent d'identifier des ligands ou aptamères contre des déterminants moléculaires (ou marqueurs) de pathologies, qui seront effectivement efficaces dans les conditions même de leur utilisation future, c'est-à-dire *in vivo.*

Ainsi en sélectionnant une cible particulière, à savoir les cellules $C_N$, $C_i$ et $C_{Te}$, telles que définies ci-dessus, on obtient effectivement des aptamères qui reconnaissent, de manière spécifique, les cellules exprimant la forme humaine du récepteur Ret sous une forme activée ou non, de préférence sous une forme activée.

L'identification d'un aptamère spécifique des cellules exprimant une forme humaine du récepteur Ret dans sa forme activée ou non, tel que défini ci-dessus, comporte avantageusement une étape (j) supplémentaire d'évaluation de son activité biologique sur lesdites cellules $C_{Te}$.

Les activités biologiques qui sont avantageusement évaluées sont les suivantes :

(a) inhibition ou activation de l'auto-phosphorylation du récepteur Ret,
(b) inhibition ou activation de la cascade d'activation de kinases Erk, protéine intracellulaire en aval de Ret dans la cascade,
(c) inhibition de la phosphorylation du récepteur Ret normal des cellules PC 12 activées par le GDNF et réversion du phénotype qui lui est associé,
(d) réversion du phénotype associé à l'activation du RPTK surexprimant l'oncogène ($Ret^{C634Y}$).

Ledit aptamère est susceptible d'être obtenu par un procédé d'identification tel que précisé ci-dessus et est sélectionné dans le groupe constitué par les aptamères de formule (I) :

$R_1$-R-$R_2$, (I),

dans laquelle :

$R_1$ représente 5' GGGAGACAAGAAUAAACGCUCAA 3' (SEQ ID NO :1) ou un fragment de 1 à 23 nucléotides de ladite SEQ ID NO :1 ;
$R_2$ représente 5' AACGACAGGAGGCUCACAACAGGA 3' (SEQ ID NO :2) ou un fragment de 1 à 24 nucléotides de ladite SEQ ID NO :2 et
R représente une séquence aléatoire de 10 à 1000 nucléotides, de préférence de 50 nucléotides.

Selon une disposition avantageuse de ce mode de réalisation, R est de préférence sélectionné parmi les séquences suivantes :

**D4** 5'GCGCGGGAAUAGUAUGGAAGGAUACGUAUACCGUGCAAUCCAGGGCAACG 3' (SEQ ID NO3)
**D12** 5'GGGCUUCAUAAGCUACACCGGCCAACGCAGAAAUGCCUUAAGCCCGAGUU 3' (SEQ ID NO:4)
**D14** 5'GGCCAUAGCGCACCACCAAGAGCAAAUCCCUAAGCGCGACUCGAGUGAGC 3' (SEQ ID NO.5)
**D20** 5'GGGCCAAUCGAAGCCGGUAAUUCCCAAACUAACGUGCAAACUGCACCCGC 3' (SEQ ID NO:6)
**D24** 5'GCGGUAUGUAGGGAAUAGCACUUUUUUUGCGUAUACCUACACCGCAGCG 3' (SEQ ID NO:7)
**D30** 5'AGGCGAGCCCGACCACGUCAGUAUGCUAGACAACAACGCCCGCGUGGUAC 3' (SEQ ID NO.8)
**D32** 5'CCCCGCUUUUUGACGUGAUCGAACGCGUAUCAGUAACGUCAGCAGUCGAGC 3' (SEQ ID NO9)
**D33** 5'CAAAGCGUGUAUUCUCGUGAGCCGACCAUCGUUGCGAACAUCCCCGGAACG 3'(SEQ ID NO :10)
**D42** 5'GACCCGUAUGAAGGUGGCGCAGGACACGACCGUCUGCAAUGAGCGAGC 3' (SEQ ID NO:11)
**D60** 5'CCGACCUGUACAGCAGUUAGUUACACGUUUGAAACAACCGGCGUUCGAGC 3' (SEQ ID NO:12)
**D76** 5'GGCUUACACGGAGAAACAAGAGAGCGGCCCAAACUUGAUUGACAGUGGCC 3' (SEQ ID NO.13)
**D71** 5'GGCCCUUAACGCAAAAACGAAGGAUCAUCGAUUGAUCGCCUUAUGGGCU 3' (SEQ ID NO:14)
**D87** 5'CCGCGGUCUGUGGGACCCUUCAGGAUGAAGCGGCAACCCAUGCGGGCC 3' (SEQ ID NO:15)

Les aptamères préférés dont les R sont tels que définis ci-dessus sont représentés par les séquences SEQ ID NO :22 (D4 ; figure 11), SEQ ID NO :25 (D24 ; figure 12), SEQ ID NO :31 (D30 ; figure 13), SEQ ID NO :32 (D12 ; figure 14), SEQ ID NO :33 (D71 ; figure 15).

Conformément à l'invention, dans lesdits aptamères, les riboses des purines sont porteurs, comme c'est le cas dans l'ARN naturel, d'une fonction hydroxyle (OH) sur le carbone en position 2', tandis que les riboses des pyrimidines portent un atome de fluor sur le carbone en position 2'. Cette modification de la position 2' est connue pour conférer aux acides nucléiques une plus grande résistance vis-à-vis des nucléases.

Les séquences des amorces utilisées pour mettre en oeuvre l'étape (g) d'amplification du mélange d'acides nucléiques de formules $R_1$-R-$R_2$, dans lesquels $R_1$ représente la SEQ ID NO:1 et $R_2$ représente la SEQ ID NO :2 sont avantageusement les suivantes :

- Amorce sens (amorce P10) : TAATACGACTCACTATAGGGAGACAAGAATAAACGCTCAA (SEQ ID NO:16)
- Amorce anti-sens (amorce P30) : TCCTGTTGTGAGCCTCCTGTCGTT (SEQ ID NO: 17).

La prédiction de structure secondaire et tertiaire des aptamères sélectionnés est réalisée à l'aide du logiciel RNAstructure écrit par David H. Mathews : http://rna.chem.rochester.edu. L'algorithme utilisé par ce logiciel est basé sur les recherches décrites dans la publication : D.H. Mathews et al., J. Mol. Biol., 1999, 288, 911-940. Les mêmes prédictions peuvent être obtenues en utilisant l'algorithme **mfold,** disponible sur le site du laboratoire de Michael Zuker : http://bioinfo.math.rpi.edu/-zukerm/ L'algorithme utilisé par ce logiciel est également basé sur les recherches décrites dans la publication D.H. Mathews et al., précitée.

Parmi les aptamères décrits ci-dessus, certains présentent une structure commune, définie par la formule II ci-après :

5' $R_4X_6X_5X_4X_3$GGAAUAG$X_2X_1R_3X'_1X'_2$CGUAUAC$X'_3X'_4X'_5X'_6R_5$ 3' (II),

dont la structure secondaire est représentée à la figure 10, et dans laquelle :

- les riboses des purines sont porteurs d'un groupement OH en 2' et les riboses des pyrimidines sont porteurs d'un atome de fluor en 2',
- **$R_3$** est présent ou absent et représente un renflement (ou boucle) apical comprenant :
  - une chaîne carbonée linéaire ou ramifiée sélectionnée dans le groupe constitué par des groupes alkyles en $C_6$-$C_{30}$ ou des groupes aryles en $C_6$-$C_{30}$ ;
  - un polymère tel que du PEG ou du PEI ou autre.
  - des groupements fonctionnels tels que biotine, streptavidine, peroxydase...
  - d'autres molécules d'intérêt comme par exemple, des principes actifs, des étiquettes de marquage notamment fluorescentes ou des chélateurs pour radio isotopes
  - une séquence nucléotidique naturelle (ADN ou ARN) ou modifiée (par exemple : 2'fluoro, 2'O-méthyle, PNA, LNA...) ; de préférence $R_3$ représente les renflements ou boucles (1) à (4) suivants :

Boucle (1) : 5'UGGAAGGA 3' (SEQ ID NO :29)
Boucle (2) : 5' CUUUUUU 3' (SEQ ID NO :30)
Boucle (3) 5'GNPuA 3'
Boucle (4) 5'UNCG 3',

dans lesquels les riboses des purines sont porteurs d'une fonction hydroxyle sur le carbone en position 2', tandis que les riboses des pyrimidines portent un atome de fluor sur le carbone en position 2',

- **$X_1$, $X'_1$, $X_2$, $X'_2$, $X_3$, $X'_3$, $X_4$, $X'_4$, $X_5$, $X'_5$, $X_6$ et $X'_6$** représentent Py ou Pu avec de préférence :

$X_1$-$X'_1$ correspondant à C-G, A-U, G-C ou U-A
$X_2$-$X'_2$ correspondant à C-G, A-U, G-C ou U-A
$X_3$-$X'_3$ correspondant à C-G, A-U, G-C ou U-A
$X_4$-$X'_4$ correspondant à C-G, A-U, G-C ou U-A
$X_5$-$X'_5$ correspondant à C-G, A-U, G-C ou U-A
$X_6$-$X'_6$ correspondant à C-G, A-U, G-C ou U-A
N correspondant à G ou C ou A ou U
**Pu** correspondant à G ou A, dans lesquels les riboses sont porteurs d'un groupement OH en 2' (chimie ARN naturelle)
**Py** correspond à U ou C, dans lesquels les riboses sont porteurs d'un atome de fluor en 2' et

- **$R_4$** et **$R_5$** sont présents ou absents et représentent :

. une séquence nucléotidique naturelle (ADN ou ARN), ou modifiée (par exemple : 2'Fluoro, 2'O-méthyle, PNA, LNA...), comprenant entre 1 et plusieurs milliers de nucléotides, de préférence entre 1 et 39 nucléotides ; une partie de ladite séquence nucléotidique ou ladite séquence comprenant de préférence l'une des séquences suivantes :

$R_4$

5'-$R_1$-$Z_1$-3', avec $Z_1$=G: 5'GGGAGACAAGAAUAAACGCUCAAG 3' (SEQ ID NO: 18) ou
5'-$R_1$-$Z_1$-3', avec $Z_1$=GCGGUAU (SEQ ID NO :26) :
5'GGGAGACAAGAAUAAACGCUCAAGCGGUAU (SEQ ID NO :19), et

R5 :

5'-$Z_2$-$R_2$-3', avec $Z_2$= CAAUCCAGGGCAACG (SEQ ID NO :27) :
5' CAAUCCAGGGCAACGAACGACAGGAGGCUCACAACAGGA 3' (SEQ ID NO: 20) ou
5'-$Z_2$-$R_2$-3', avec $Z_2$=ACCGCAGCG (SEQ ID NO :28) :

5'ACCGCAGCGAACGACAGGAGGCUCACAACAGGA 3' (SEQ ID NO 21);

. une chaîne carbonée linéaire ou ramifiée sélectionnée dans le groupe constitué par des groupes alkyles en $C_6$-$C_{30}$ ou des groupes aryles en $C_6$-$C_{30}$ ;
. un polymère tel que du PEG ou du PEI ou autre.
. des groupements fonctionnels tels que biotine, streptavidine, peroxydase...

. d'autres molécules d'intérêt comme par exemple, des principes actifs, des étiquettes de marquage notamment fluorescentes ou des chélateurs pour radio isotopes.

Parmi les aptamères présentant une structure telle que définie à la formule II et à la figure 10, on observe des différences de propriétés comme précisé ci-après :

La structure de formule II, dans laquelle $R_3$, $R_4$ et $R_5$ sont absents, est suffisante pour la liaison au récepteur $Ret^{C634Y}$, alors que la structure de formule II, dans laquelle $R_3$, $R_4$ et $R_5$ sont présents, présente soit uniquement des propriétés de liaison, soit à la fois des propriétés de liaison et d'inhibition ; ces propriétés variant en fonction de $R_3$, $R_4$ et $R_5$ :

- lorsque $R_3$ représente 5' UGGAAGGA 3' (SEQ ID NO :29 : boucle (1)), $R_4$ représente la SEQ ID NO :18 et $R_5$ représente la SEQ ID NO :20, l'aptamère présentant une telle structure (famille D4) possède à la fois des propriétés de liaison audit récepteur Ret dans sa forme activée et des propriétés d'inhibition de l'activité dudit récepteur. La structure secondaire de ce produit est représentée à la figure 11 ; il s'agit des produits de la famille D4, dans laquelle $R_4$ et $R_5$ comprennent un nombre suffisant de nucléotides entraînant une inhibition de l'activité du récepteur Ret. Un aptamère préféré répondant à cette définition comprend de 5' en 3', successivement les séquences suivantes : SEQ ID NO :1+SEQ ID NO :3+SEQ ID NO :2 (SEQ ID NO :22) (D4 : figure 5A et figure 11), en référence à la formule I.
- Lorsque $R_3$ représente 5' CUUUUUU 3' (SEQ ID NO :30 : boucle (2)), 5' GNPuA 3' (boucle 3) ou 5' UNCG 3' (boucle 4), $R_4$ comprend de 1 à 30 nucléotides sélectionnés dans la SEQ ID NO :19 ou de 1 à 24 nucléotides sélectionnés dans la SEQ ID NO :18 et $R_5$ comprend de 1 à 33 nucléotides sélectionnés dans la SEQ ID NO :21 ou de 1 à 39 nucléotides sélectionnés dans la SEQ ID NO :20, l'aptamère présentant une telle structure ne possède que des propriétés de liaison audit récepteur Ret dans sa forme activée et notamment au récepteur Ret muté dans son domaine extracellulaire. Dans le cas où $R_3$ représente 5' CUUUUUU 3' (boucle 2), $R_4$ représente la SEQ ID NO : 19 et $R_5$ représente la SEQ ID NO :21, la structure secondaire de ce produit est représentée à la figure 12 ; le produit préféré est représenté par la SEQ ID NO :25 et appartient à la famille D24, dans laquelle $R_4$ et $R_5$ comprennent au minimum un nucléotide. Cet aptamère comprend successivement de 5' en 3' : SEQ ID NO :1 + SEQ ID NO :7 + SEQ ID NO :2, en référence à la formule I.
- lorsque $R_3$ représente 5' UGGAAGGA 3' (boucle 1) et en l'absence de $R_4$ et de $R_5$, l'aptamère présentant une telle structure ne possède que des propriétés de liaison audit récepteur Ret dans sa forme activée. Un aptamère préféré répondant à cette dernière définition correspond à une partie de la SEQ ID NO :3 de la famille D4 (SEQ ID NO :23).

Les différences entre les définitions de $R_1$ et $R_2$ dans la formule 1 $R_1$-R-$R_2$ et de $R_4$ et $R_5$ dans la formule II, proviennent de la non superposition entre la séquence consensus de la figure 10 et la définition de R (séquences aléatoires) dans la formule I.

Les figures 5A et 10 à 12 montrent les liens entre les deux formules (I et II).

Les ligands ou aptamères selon l'invention peuvent avantageusement être mis en oeuvre dans les applications suivantes :

- en tant que réactif de diagnostic : aussi bien *in vitro* (trousses diagnostiques ; marqueur histologique ; puces) qu'*in vivo* (agent de contraste pour l'imagerie ; radiopharmaceutique). En effet, les aptamères selon l'invention qui ont une capacité de liaison spécifique à un récepteur Ret dans sa forme activée ou non sont adaptés à la détection de cellules exprimant ledit récepteur sous forme activée ou non ; en particulier lorsque le récepteur Ret est muté dans son domaine extracellulaire, lesdits aptamères sont particulièrement adaptés à la détection de cellules anormales exprimant ledit récepteur ; dans le cas où le récepteur sous sa forme activée n'est pas muté, on suivra alors une différence de niveau d'expression de la protéine.
- en tant que médicament, notamment comme anticancéreux ; notamment en ce qui concerne les aptamères qui présentent à la fois une capacité de liaison au récepteur Ret et une action inhibitrice vis-à-vis, notamment du récepteur muté $Ret^{C634Y}$.

En effet, de manière surprenante :

- certains des aptamères sélectionnés ont uniquement une activité de liaison et seront de préférence utilisés comme réactifs de diagnostic.
- d'autres aptamères sélectionnés présentent, en outre, une activité d'inhibition de la transformation oncogénique médiée par l'oncogène humain Ret muté notamment au niveau d'une cystéine dans le domaine extracellulaire, et un effet d'inhibition de l'activité de la protéine normale Ret après stimulation par son propre ligand (famille D4 ou séquences dérivées de la formule II avec des modifications chimiques). Les aptamères présentant une telle activité pourront être utilisés soit comme agents diagnostiques soit comme médicaments. Pour leur caractérisation, quatre tests peuvent avantageusement être réalisés, comme précisé ci-dessus :

(a) inhibition ou activation de l'auto-phosphorylation de Ret,
(b) inhibition ou activation de la cascade d'activation de kinases Erk, protéine intracellulaire en aval de Ret dans la cascade,
(c) inhibition de la phosphorylation de Ret activée par le GDNF
(d) réversion du phénotype associé à l'activation de Ret (notamment $Ret^{C634Y}$),

La présente invention a également pour objet un réactif de diagnostic d'une tumeur, caractérisé en ce qu'il est constitué par au moins un aptamère tel que défini ci-dessus.

Selon un mode de réalisation avantageux dudit réactif, il correspond à un aptamère de formule II, telle que définie ci-dessus :

$5'R_4X_6X_5X_4X_3GGAAUAGX_2X_1R_3X'_1X'_2CGUAUACX'_3X'_3X'_5X'_6R_53'$,

dans laquelle $R_3$, $R_4$ et $R_5$ sont absents.

Selon une disposition avantageuse de ce mode de réalisation, ledit réactif correspond à un aptamère de séquence :

5'GUAGGGAAUAGCACGUAUACCUAC 3'(**SEQ ID NO :24**),

dans lequel $X_1$-$X'_1$ = A-U, $X_2$-$X'_2$ = C-G, $X_3$-$X'_3$ = G-C, $X_4$-$X'_4$ = A-U, $X_5$-$X'_5$ = U-A et $X_6$-$X'_6$ = G-C.

Selon un autre mode de réalisation avantageux dudit réactif, il correspond à un aptamère de formule II, dans laquelle $R_3$ représente 5' CUUUUUU 3' (boucle (2)), $R_4$ représente la séquence SEQ ID NO :19 et $R_5$ représente la séquence SEQ ID NO :21 ; cet aptamère correspond à SEQ ID NO :25, et comprend successivement de 5' en 3', en référence à la formule I : SEQ ID NO :1 + SEQ ID NO :7 + SEQ ID NO :2, comme précisé ci-dessus.

La présente invention a également pour objet un réactif de diagnostic ou de détection du récepteur Ret sous une forme activée ou non, caractérisé en ce qu'il est constitué par au moins un aptamère tel que défini ci-dessus.

La présente invention a en outre pour objet un médicament, caractérisé en ce qu'il comprend un aptamère tel que défini ci-dessus qui présente à la fois une capacité de liaison à un récepteur RPTK et une action inhibitrice vis-à-vis dudit récepteur sous une forme activée.

La présente invention a également pour objet un médicament destiné au traitement d'une tumeur, caractérisé en ce qu'il comprend un aptamère tel que défini ci-dessus qui présente à la fois une capacité de liaison à un récepteur RPTK activé et notamment à un récepteur muté dans le domaine extracellulaire et notamment au récepteur Ret muté, par exemple au niveau d'une des cystéines localisées dans le domaine extracellulaire (codons 609, 611, 618, 620 et 634) et une action inhibitrice vis-à-vis de ce récepteur activé.

Selon un mode de réalisation avantageux dudit médicament, il correspond à un aptamère de la famille des aptamères D4, tels que définis ci-dessus.

La présente invention a également pour objet une composition pharmaceutique, caractérisée en ce qu'elle comprend un aptamère tel que défini ci-dessus, qui présente à la fois une capacité de liaison à un récepteur RPTK et une action inhibitrice vis-à-vis dudit récepteur sous sa forme activée.

La présente invention a en outre pour objet une composition pharmaceutique, caractérisée en ce qu'elle comprend :

- un aptamère tel que défini ci-dessus, qui présente à la fois une capacité de liaison à un récepteur RPTK muté dans le domaine extracellulaire et notamment au récepteur Ret muté, par exemple au niveau d'une des cystéines localisées dans le domaine extracellulaire (codons 609, 611, 618, 620 et 634) et une action inhibitrice vis-à-vis de ce récepteur muté,
- une autre molécule anticancéreuse et
- au moins un véhicule pharmaceutiquement acceptable.

La présente invention a également pour objet l'utilisation d'un aptamère qui présente à la fois une capacité de liaison à un récepteur RPTK et une action inhibitrice ou non vis-à-vis de ce récepteur RPTK pour le criblage de produits interagissant avec le récepteur RPTK et pouvant l'inhiber ou non.

La présente invention a également pour objet l'utilisation d'un aptamère qui présente à la fois une capacité de liaison à un récepteur RPTK dans sa forme activée et notamment au récepteur Ret muté au niveau d'une des cystéines localisées dans le domaine extracellulaire (codons 609, 611, 618, 620 et 634) et une action inhibitrice ou non vis-à-vis de ce récepteur RPTK muté pour le criblage de produits interagissant avec le récepteur RPTK et pouvant l'inhiber ou non.

La présente invention a, en outre, pour objet, un procédé de criblage de produits interagissant avec un récepteur RPTK ou des cibles formant un complexe avec le RPTK (sous une forme activée ou non), lequel procédé est caractérisé en ce qu'il comprend :

- la mise en contact de cellules exprimant des RPTK sous une forme activée ou non avec la substance à tester,
- l'ajout, dans des conditions convenables, d'un aptamère, éventuellement marqué, tel que défini ci-dessus, avant la substance à tester, en même temps ou après, et
- l'évaluation de la fixation compétitive entre l'aptamère et la substance à tester (par exemple : par mesure de radioactivité, de fluorescence, de luminescence, de résonance plasmonique de surface, BRET, FRET, ou toute autre technique de mise en évidence d'une interaction moléculaire).

Conformément à l'invention, après identification des substances se liant de manière compétitive avec l'aptamère aux cellules présentant des RPTK sous une forme activée, l'effet de ces substances sur l'activité biologique desdits cellules peut être évalué pour trouver des substances qui inhibent ou activent lesdites activités biologiques des cellules exprimant des RPTK sous une forme activée.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- Figure 1 : sélection schématique des aptamères spécifiques des cellules PC12 MEN 2A :

  . étapes a) et b) : contre sélection
  Une banque combinatoire d'ARNs 2'-F-Py est incubée avec des cellules PC12 sauvages (PC12 wt) en suspension ; les séquences non liées sont récupérées par centrifugation et incubées avec des cellules PC 12 MEN 2B ; les séquences non liées, présentes dans le surnageant sont récupérées et incubées avec des cellules PC12 MEN 2A.
  . étape c) : sélection
  Les séquences non liées sont éliminées par plusieurs lavages des cellules et les séquences liées sont récupérées par extraction au phénol.
  . étapes d) et e) : amplification
  Les séquences sélectionnées sont amplifiées par RT-PCR et transcription *in vitro* avant un nouveau cycle de sélection.

- Figure 2 : structure des différents récepteurs Ret : Ret normal ; Ret MEN 2A ($Ret^{C634Y}$) ; Ret MEN 2B ($Ret^{M918T}$).
- Figure 3 : représentation du phénotype des cellules PC12 transfectées de manière stable avec un vecteur d'expression comportant la séquence codant le récepteur muté $Ret^{C634Y}$ humain (PC12 MEN 2A) ou le récepteur muté $Ret^{M918T}$ (PC12/MEN 2B).
- Figure 4 : représentation schématique de l'activation du récepteur Ret dépendant du GDNF.
- Figure 5 (A) : comparaison de la prédiction de la structure secondaire des aptamères D4 et D24. La prédiction de structure secondaire est réalisée à l'aide du logiciel **RNAstructure** écrit par David H. Mathews. http://rna.chem.rochester.edu. L'algorithme est basé sur les recherches décrites dans D.H. Mathews et al. (Journal of Molecular Biology, 1999, 288, 911-940, précité).
  Les mêmes prédictions peuvent être obtenues en utilisant l'algorithme mfold, disponible sur le site http://bioinfo.math.rpi.edu/~zukerm/. Ce dernier algorithme est basé sur les recherches décrites dans la publication au nom de D.H. Mathews et al., précitée. La structure consensus est en caractère gras (B) : courbe de liaison de l'aptamère D4 avec des cellules PC12 MEN 2A ; l'aptamère D4 est radiomarqué avec du $^{32}P$ et incubé à différentes concentrations avec des monocouches de cellules. Après plusieurs lavages, l'aptamère lié est quantifié. Il est tenu compte du bruit de fond, en soustrayant pour chaque point obtenu la valeur obtenue avec un aptamère D4 déstructuré (D4Sc) de séquence brouillée (i.e. comportant les mêmes nucléotides, mais dans un ordre différent). Une analyse Scatchard (insert) est utilisée pour évaluer la constante de liaison et le nombre de cibles.
- Figure 6 : effets des différents aptamères sélectionnés sur l'activité du récepteur $Ret^{C634Y}$ : (A) les cellules PC12 MEN 2A sont soit non traitées, soit traitées pendant 16 heures avec 150 nM de l'aptamère indiqué ou du pool (banque combinatoire) d'ARN de départ ; (B) les cellules PC 12 MEN 2A sont traitées pendant une heure avec des doses croissantes de D4 (produit de formule II) (gauche) ou avec 200 nM de produit D4 pendant les temps d'incubation indiqués (droite). Les lysats cellulaires sont soumis à une analyse par immunoblotting (ou immunoempreinte) avec des anticorps anti-Ret (Tyr-phosphorylée) ou des anticorps anti (phospho) Erk, comme indiqué. Pour confirmer la charge égale, les membranes de transfert sont soumises à une nouvelle analyse en présence des anticorps anti-Ret total et anti-Erk total précités. Les cellules contrôle non traitées sont indiquées par un « C ». Les valeurs de phosphorylation, en prenant la valeur 1 pour le contrôle ont été calculées à l'aide du programme NIH Image, basé sur la somme des deux bandes spécifiques de Erk. Les déviations standard sont obtenues à partir de quatre expériences indépendantes.

- Figure 7 : effet de l'aptamère D4 sur l'activité du récepteur Ret sauvage ($Ret^{wt}$) et du récepteur muté $Ret^{M918T}$. (A) les cellules PC12 transfectées de manière à exprimer de façon stable le récepteur Ret non muté (PC12/wt) sont traitées pendant 10 min avec du GDNF (50 ng/ml et du GFR$\alpha$1 soluble (1,6 nM), ou 5 min avec du NGF (100 ng/ml) ainsi qu'avec, simultanément 200 nM soit d'aptamère D4, soit du pool d'ARN de départ. (B) les cellules PC12 MEN 2B sont déprivées de sérum pendant 6 heures puis traitées pendant 1 heure avec 200 nM d'aptamère D4 ou de pool d'ARN de départ. Les lysats cellulaires sont analysés par immunoempreinte avec les anticorps suivants : anticorps anti- Ret (Tyr-phosphorylée) ou anticorps anti(phospho) Erk, comme indiqué. Les déviations standard sont obtenues à partir de cinq expériences indépendantes.
- Figure 8 : l'aptamère D4 inhibe la différenciation des cellules PC12 transfectées de manière à exprimer de façon stable le récepteur Ret non muté et le co-récepteur GFR$\alpha$1 (PC12-$\alpha$1/wt) induite par le GDNF. Les cellules sont soit non stimulées (A) ou stimulées par du GDNF seul (B) ou stimulées par du GDNF en présence de l'aptamère D4 ou de l'aptamère D4 déstructuré (D4Sc) (C et D, respectivement). Après 48 heures de traitement au GDNF, le pourcentage d'extension des prolongements est calculé. Les données sont exprimées comme le pourcentage de cellules comportant des prolongements rapporté au nombre total de cellules comptées. Chaque expérience est répétée au moins trois fois (E) et les lysats cellulaires sont analysés par immunoempreinte avec des anticorps anti-VGF qui est un marqueur de la différenciation induite par l'activation de Ret par le GDNF (F).
- Figure 9 : l'aptamère D4 modifie la morphologie des cellules NIH/MEN 2A transformées. Les lignées cellulaires indiquées sont ensemencées à densité égale sur des plaques de culture comprenant 12 puits. Un jour après l'ensemencement, 3 $\mu$M de D4 ou de D4 déstructuré sont ajoutés dans le milieu et les cellules sont maintenues en culture pendant 72 heures en ajoutant 3 $\mu$M de chaque aptamère chaque 24 heures. Etant donné la ½ vie de l'aptamère dans du sérum à 15 %, ce protocole assure la présence continue d'au moins 200 nM d'aptamère dans le milieu. Les cellules sont photographiées à l'aide d'un microscope à contraste de phase.
- Figures 10 à 15 : structure secondaire des aptamères suivants : formule II (figure 10); D4 (figure 11) ; D24 (figure 12); D30 (figure 13); D12 (figure 14) et D71 (figure 15),
- Figure 16 : Criblage d'aptamères interagissant avec RET sur les cellules PC12 MEN 2A par fixation compétitive avec l'aptamère D4. L'aptamère D4 est radiomarqué avec du $^{32}P$ et incubé à 50nM avec des monocouches de cellules PC12 MEN 2A en présence de 400nM de différents aptamères. Après plusieurs lavages, la quantité d'aptamère D4 lié est quantifiée. Il est tenu compte du bruit de fond, en soustrayant pour chaque point obtenu la valeur obtenue avec un aptamère D4 déstructuré (D4Sc) de séquence brouillée (i.e. comportant les mêmes nucléotides, mais dans un ordre différent).
- Figure 17 : Fixation compétitive de l'aptamère E38 sur les cellules PC12 MEN 2A en présence dune gamme d'aptamère D4. L'aptamère E38 est radiomarqué avec du $^{32}P$ et incubé à 100nM avec des monocouches de cellules PC12 MEN 2A en présence d'une concentration croissante d'aptamère D4. Après plusieurs lavages, la quantité d'aptamère E38 lié est quantifiée. Il est tenu compte du bruit de fond, en soustrayant pour chaque point obtenu la valeur obtenue avec un aptamère D4 déstructuré (D4Sc) de séquence brouillée (i.e. comportant les mêmes nucléotides, mais dans un ordre différent).

Il doit être bien entendu, toutefois, que ces exemples sont donnés uniquement à titre d'illustration de l'objet de l'invention, dont ils ne constituent en aucune manière une limitation.

**EXEMPLE 1 : Préparation d'une banque combinatoire d'ARNs 2'-F-Py**

Pour obtenir l'ARN 2'F-Py, il faut réaliser une transcription *in vitro* à partir d'une matrice d'ADN double brin obtenue selon 3 méthodes :

**1. Avant la sélection, par l'amplification PCR de la séquence ADN :**

B2S0 : 5'TCCTGTTGTGAGCCTCCTGTCGTT-N-TTGAGCGTTTATTCTTGTCTCCC3',

où N représente une séquence aléatoire de 50 nucléotides

**1er cycle de PCR :**

* *Hybridation*

5'TCCTGTTGTGAGCCTCCTGTCGTT-N-TTGAGCGTTTATTCTTGTCTCCC3' (B2S0)
(amorce P10) 3'AACTCGCAAATAAGAACAGAGGGATATCACTCAGCATAAT5'
(SEQ ID NO :16)

* *Elongation*

5'TCCTGTTGTGAGCCTCCTGTCGTT-N-TTGAGCGTTTATTCTTGTCTCCC**TATAGTGAGTCGTATTA**3'

**3'AGGACAACACTCGGAGGACAGCAA-M-**AACTCGCAAATAAGAACAGAGGGATATCACTCAGCATAAT5',

où le texte en gras représente la séquence polymérisée et M représente la séquence complémentaire de N.

**2ème cycle de PCR :**

* *Dénaturation*

5'TCCTGTTGTGAGCCTCCTGTCGTT-N-TTGAGCGTTTATTCTTGTCTCCCTATAGTGAGTCGTATTA3'

3'AGGACAACACTCGGAGGACAGCAA-M-AACTCGCAAATAAGAACAGAGGGATATCACTCAGCATAAT5'

* *Hybridation*

5'TCCTGTTGTGAGCCTCCTGTCGTT-N-TTGAGCGTTTATTCTTGTCTCCCTATAGTGAGTCGTATTA3'
3'AACTCGCAAATAAGAACAGAGGGATATCACTCAGCATAAT5'

(amorce P10 : SEQ ID NO :16)

5'TCCTGTTGTGAGCCTCCTGTCGTT3' (Amorce P30 : SEQ ID NO :17)
3'AGGACAACACTCGGAGGACAGCAA-M-AACTCGCAAATAAGAACAGAGGGATATCACTCAGCATAAT5'

* *Elongation*

5'TCCTGTTGTGAGCCTCCTGTCGTT-N-TTGAGCGTTTATTCTTGTCTCCCTATAGTGAGTCGTATTA3'
**3'AGGACAACACTCGGAGGACAGCAA-M-**AACTCGCAAATAAGAACAGAGGGATATCACTCAGCATAAT5'

5'TCCTGTTGTGAGCCTCCTGTCGTT**-N-TTGAGCGTTTATTCTTGTCTCCCTATAGTGAGTCGTATTA3'**
3'AGGACAACACTCGGAGGACAGCAA-M-AACTCGCAAATAAGAACAGAGGGATATCACTCAGCATAAT5'

le texte en gras représentant la séquence polymérisée et M représente la séquence complémentaire de N.

Ce deuxième cycle de PCR est répété 15 à 30 fois afin d'obtenir une l'ADN double brin qui sera transcrit *in vitro* en ARN 2'F-Py.

**2. Pendant la sélection, par l'amplification RT-PCR des ARN 2'F-Py sélectionnés, de formule $R_1$-R- $R_2$, telle Que définie ci-dessus :**

5'GGGAGACAAGAAUAAACGCUCAAR-AACGACAGGAGGCUCACAACAGGA3', où R représente la séquence des ARN 2'F-Py sélectionnés.

**Réverse transcription (RT) :**

* *Hybridation*

```
5'GGGAGACAAGAAUAAACGCUCAA-R-AACGACAGGAGGCUCACAACAGGA3'(ARN 2'F-Py)
                         3' TTGCTGTCCTCCGAGTGTTGTCCT5'(amorce P30)
```

* *Elongation*

```
5'GGGAGACAAGAAUAAACGCUCAA-R-AACGACAGGAGGCUCACAACAGGA3'
3'CCCTCTGTTCTTATTTGCGAGTT-S-TTGCTGTCCTCCGAGTGTTGTCCT5',
```

où le texte en gras représente la séquence polymérisée et S représente la séquence complémentaire de R

**1ème cycle de PCR :**

* *Dénaturation*

3'CCCTCTGTTCTTATTTGCGAGTT-S-TTGCTGTCCTCCGAGTGTTGTCCT5'

(ADNc de l'ARN2' F-Py)

* *Hybridation*

```
5'TAATACGACTCACTATAGGGAGACAAGAATAAACGCTCAA3' (amorce P10)
                 3'CCCTCTGTTCTTATTTGCGAGTT-S-TTGCTGTCCTCCGAGTGTTGTCCT5'
```

* *Elongation*

5'TAATACGACTCACTATAGGGAGACAAGAATAAACGCTCAA**-R-AACGACAGGAGGCTCACAACAGGA3'**
3'ATTATGCTGAGTGATATCCCTCTGTTCTTATTTGCGAGTT-S-TTGCTGTCCTCCGAGTGTTGTCCT5'

où le texte en gras représente la séquence polymérisée et S représente la séquence complémentaire de R.

**2ème cycle de PCR :**

* *Dénaturation*

5'TAATACGACTCACTATAGGGAGACAAGAATAAACGCTCAA-R-AACGACAGGAGGCTCACAACAGGA3'

3'ATTATGCTGAGTGATATCCCTCTGTTCTTATTTGCGAGTT-S-TTGCTGTCCTCCGAGTGTTGTCCT5'

* *Hybridation*

5'TAATACGACTCACTATAGGGAGACAAGAATAAACGCTCAA-R-AACGACAGGAGGCTCACAACAGGA3'
(amorce P30) 3'TTGCTGTCCTCCGAGTGTTGTCCT5'

5'TAATACGACTCACTATAGGGAGACAAGAATAAACGCTCAA3' (amorce P10)
3'ATTATGCTGAGTGATATCCCTCTGTTCTTATTTGCGAGTT-S-TTGCTGTCCTCCGAGTGTTGTCCT5'

* *Elongation*

5'TAATACGACTCACTATAGGGAGACAAGAATAAACGCTCAA-R-AACGACAGGAGGCTCACAACAGGA3'
**3'ATTATGCTGAGTGATATCCCTCTGTTCTTATTTGCGAGTT-S**-TTGCTGTCCTCCGAGTGTTGTCCT5'

5'TAATACGACTCACTATAGGGAGACAAGAATAAACGCTCAA**-R-AACGACAGGAGGCTCACAACAGGA3'**
3'ATTATGCTGAGTGATATCCCTCTGTTCTTATTTGCGAGTT-S-TTGCTGTCCTCCGAGTGTTGTCCT5'

où le texte en gras représente la séquence polymérisée et S représente la séquence complémentaire de R.

Ce deuxième cycle de PCR est répété 15 à 30 fois afin d'obtenir une l'ADN double brin qui sera transcrit *in vitro* en ARN 2'F-Py.

<u>**3. Après la sélection, par l'amplification PCR des aptamères à partir de plasmide où ils ont été clonés**</u>

Les plasmides contiennent la séquence :

5'TAATACGACTCACTATAGGGAGACAAGAATAAACGCTCAA**-R-AACGACAGGAGGCTCACAACAGGA3'**
3'ATTATGCTGAGTGATATCCCTCTGTTCTTATTTGCGAGTT-S-TTGCTGTCCTCCGAGTGTTGTCCT5'

où R représente la séquence ADN spécifique de l'aptamère et S la séquence complémentaire à R.

**1ème cycle de PCR :**

* *Dénaturation*

5'TAATACGACTCACTATAGGGAGACAAGAATAAACGCTCAA-R-AACGACAGGAGGCTCACAACAGGA3'

3'ATTATGCTGAGTGATATCCCTCTGTTCTTATTTGCGAGTT-S-TTGCTGTCCTCCGAGTGTTGTCCT5'

* *Hybridation*

5'TAATACGACTCACTATAGGGAGACAAGAATAAACGCTCAA-R-AACGACAGGAGGCTCACAACAGGA3'<br>
(amorce P30) 3'TTGCTGTCCTCCGAGTGTTGTCCT5'

5'TAATACGACTCACTATAGGGAGACAAGAATAAACGCTCAA3' (amorce P10)<br>
3'ATTATGCTGAGTGATATCCCTCTGTTCTTATTTGCGAGTT-S-TTGCTGTCCTCCGAGTGTTGTCCT5'

* *Elongation*

5'TAATACGACTCACTATAGGGAGACAAGAATAAACGCTCAA-R-AACGACAGGAGGCTCACAACAGGA3'<br>
**3'ATTATGCTGAGTGATATCCCTCTGTTCTTATTTGCGAGTT-S**-TTGCTGTCCTCCGAGTGTTGTCCT5'

5'TAATACGACTCACTATAGGGAGACAAGAATAAACGCTCAA**-R-AACGACAGGAGGCTCACAACAGGA3'**<br>
3'ATTATGCTGAGTGATATCCCTCTGTTCTTATTTGCGAGTT-S-TTGCTGTCCTCCGAGTGTTGTCCT5'

où le texte en gras représente la séquence polymérisée et S représente la séquence complémentaire de R
Ce cycle de PCR est répété 15 à 30 fois afin d'obtenir une l'ADN double brin qui sera transcrit in vitro en ARN 2'F-Py.

**Transcription *in vitro* :**

Un des deux brins de l'ADN amplifié par PCR sert de matrice pour la transcription *in vitro* des ARN 2'F-Py double brin. La séquence soulignée correspond à la région du promoteur de l'ARN polymérase du phage T7

5'TAATACGACTCACTATAGGGAGACAAGAATAAACGCTCAA-R-AACGACAGGAGGCTCACAACAGGA3'<br>
3'ATTATGCTGAGTGATATCCCTCTGTTCTTATTTGCGAGTT-S-TTGCTGTCCTCCGAGTGTTGTCCT5'

/ GGGAGACAAGAATAAACGCTCAA-R-AACGACAGGAGGCTCACAACAGGA3'

5'TAATACGACTCACTATA**GGGAGACAAGAAUAAACGCUCAA-R-AACGACAGGAGGCUCACAACAGGA3'**<br>
3'ATTATGCTGAGTGATATCCCTCTGTTCTTATTTGCGAGTT-S-TTGCTGTCCTCCGAGTGTTGTCCT5'

Dans les ARN2'F-Py, la séquence complémentaire de l'amorce P30 se trouve à l'extrémité 3':

5'AACGACAGGAGGCUCACAACAGGA3' ($R_2$=SEQ ID NO :2)

Et une partie de séquence identique à l'amorce P10 se trouve à l'extrémité 5':

5'GGGAGACAAGAAUAAACGCUCAA3' ($R_1$= SEQ ID NO :1).

**EXEMPLE 2 : Matériel et méthodes**

*- Culture cellulaire et analyse en immunoblot*

Les conditions de croissance des cellules PC12 et des lignées cellulaires dérivées ont été décrites par D'Alessio A. et al. (Endocrinology 2003; 144, 10, 4298-4305).

Les cellules NIH/MEN2A et NIH/MEN2B sont obtenues à partir de cellules NIH3T3, transfectées de manière stable avec des vecteurs d'expression pour $Ret^{C634Y}$ et $Ret^{M918T}$. Pour évaluer les effets des aptamères selon l'invention sur l'activité Ret, des cellules (160.000 cellules/3,5 cm de plaque) ont été déprivées en sérum pendant 2 heures, puis traitées avec la quantité indiquée dans les figures (200 nM) d'aptamère ou d'un pool d'ARN primaire après une courte étape de dénaturation-renaturation.

Lorsque cela est indiqué, 100 ng/ml de NGF 2,5 S (*Nerve Growth Factor;* Upstate Biotechnology Inc., Lake Placid), 50 ng/ml de GNDF (Promega) ou 1,6 nM de chimère GFRα1-FC (R1D Systems Ltd, UK) sont ajoutés dans le milieu de culture.

Les extraits cellulaires et l'analyse en immunoblot sont réalisés comme décrit dans Cerchia L. et al. (Biochem. J. 2003, 372, 897-903).

Les anticorps primaires utilisés sont les suivants : anticorps anti-Ret (C-19), anticorps anti-VGF (R-15), anticorps anti-ERKI (C-16) (Santa Cruz Biotechnology Inc., Santa Cruz CA), anticorps anti-Ret (Tyr phosphorylée) (Cell Signaling), anticorps monoclonaux anti-phospho44/42 MAP kinase (E10) (Cell Signaling). Pour les immunoblots illustrés dans les figures, l'analyse statistique a été réalisée sur au moins quatre expériences indépendantes.

*- Test d'extension des prolongements cellulaires*

Des cellules PC12-α1/wt sont ensemencées à densité égale sur des plaques de culture comprenant 12 puits. Pour évaluer les effets de l'aptamère D4 sur la différenciation cellulaire, les cellules sont prétraitées pendant 6 heures avec 400 nM d'aptamère D4 ou d'aptamère D4 déstructuré, puis incubées avec 50 ng/ml de GNDF et les aptamères appropriés à une concentration finale de 3 μM. Après une stimulation par le GDNF de 24 heures, 3 μM d'aptamère D4 ou d'aptamère D4 déstructuré sont à nouveau ajoutés aux cellules et la stimulation est poursuivie jusqu'à 48 heures. Au moins 15 champs aléatoires sont photographiés 24 heures et 48 heures après la stimulation au GDNF en utilisant un microscope à contraste de phase et 50 cellules par cadre sont comptées ; la présence ou l'absence d'extension des prolongements est enregistrée. On considère qu'il existe une extension des prolongements lorsque l'on observe un processus d'extension d'un diamètre supérieur au double du diamètre du corps cellulaire.

*- SELEX ex vivo*

Le cycle SELEX est réalisé essentiellement comme décrit antérieurement (Tuerk C et al., Science 1990, 249, 4968, 505-510; Ellington AD et al., Nature, 1990, 346, 6287, 818-22). La transcription est réalisée en présence de 1 mM de 2'-F pyrimidines et d'une forme mutante d'ARN polymérase T7 ($T7^{Y639F}$) (Padilla, R et al., Nucleic Acids Res, 1999, 27, 6, 1561-1563), pour augmenter les rendements. Les ARNs 2'-F-Py sont utilisés en raison de leur résistance à la dégradation par les nucléases sériques.

La complexité de l'échantillon initial est d'environ $10^{14}$ séquences différentes. La banque d'ARNs 2'-F-Py (1-5 nmoles) contenant 50 nucléotides de séquences aléatoires sont chauffés à 85°C pendant 5 min dans 3 ml de RPMI 1640, refroidis rapidement dans de la glace, pendant 2 min, puis réchauffés jusqu'à 37°C avant leur incubation avec les cellules.

Deux étapes de contre sélection sont réalisées à chaque cycle.

Pour éviter la sélection d'aptamères reconnaissant, de manière non-spécifique la surface cellulaire, la banque combinatoire d'ARNs initiaux est d'abord incubée 30 min à 37°C avec 5 x $10^6$ cellules PC12 (référence ECACC N° 88022) et les séquences non liées sont récupérées par centrifugation. Ces dernières séquences sont ensuite incubées avec 5 x $10^6$ cellules PC12 MEN B2 adhérentes, exprimant un récepteur Ret muté dans le domaine intracellulaire ($Ret^{M918T}$) et les séquences non-liées sont récupérées pour la phase de sélection. Cette étape permet de sélectionner des séquences qui reconnaissent spécifiquement les cellules PC 12 MEN 2A exprimant le récepteur Ret muté au niveau du domaine extracellulaire ($Ret9^{C634Y}$).

Les séquences récupérées sont incubées avec 5 x $10^6$ cellules PC 12 MEN 2A pendant 30 min à 37°C en présence d'ARN de compétition non spécifique (ARN total de levure, Sigma) et récupérées après plusieurs lavages par extraction totale de l'ARN (Trizol, Sigma).

Après une amplification par RT-PCR à l'aide du couple d'amorces suivant :

amorce sens : 5' TAATACGACTCACTATAGGGAGACAAGAATAAACGCTCAA 3' (SEQ ID NO : 16)
amorce antisens : 5' TCCTGTTGTGAGCCTCCTGTCGTT 3' (SEQ ID NO :17)

et une transcription *in vitro* par la polymérase T7 mutante selon les conditions décrites dans Padilla, R et al. (N.A.R., 1999, précité), en utilisant un tampon modifié (40 mM Tris pH 7,5 ; 6 mM $MgCl_2$ ; 4 mM NaCl ; 2 mM spermidine ; 10 mM DTT), le processus est réitéré. Toutes les incubations avec les cellules sont effectuées à 37°C dans du milieu de culture RPMI 1640, pour approcher au maximum les conditions physiologiques.

Pendant le processus de sélection, la pression de sélection est augmentée, en augmentant le nombre de lavages, la quantité d'ARN de compétition non spécifique et en diminuant le temps d'incubation et le nombre de cellules exposées aux aptamères comme illustré au Tableau II ci-après.

Tableau II : Conditions utilisées pour les cycles successifs de sélection

| N° du cycle | Nombre (millions) de cellules | Nanomoles d'ARN 2'Fpy | Volume (ml) d'incubation | Concentration (nM) d'ARN 2'Fpy | Durée (min) d'incubation | Nombre de lavages | Compétiteur ajouté |
|---|---|---|---|---|---|---|---|
| S1 | 5 | 1 | 3 | 333 | 30 | 1 | 0 |
| S2 | 5 | 3 | 3 | 1000 | 30 | 1 | 0 |
| S3 | 5 | 3 | 3 | 1000 | 30 | 2 | 0 |
| S4 | 5 | 0.5 | 3 | 167 | 30 | 2 | 0 |
| S5 | 5 | 1,5 | 3 | 500 | 15 | 3 | 0 |
| S6 | 5 | 4 | 3 | 1333 | 15 | 4 | 0 |
| S7 | 5 | 1,3 | 3 | 433 | 15 | 3 | 0 |
| S8 | 5 | 2,3 | 3 | 767 | 15 | 3 | 0 |
| S9 | 5 | 2,5 | 3 | 833 | 15 | 3 | 0 |
| S10 | 5 | 10 | 3 | 3333 | 15 | 3 | 150μg ARN t |
| S11 | 5 | 5,8 | 3 | 1933 | 15 | 3 | 150μg ARN t |
| S12 | 5 | 2 | 3 | 667 | 15 | 4 | 150μg ARN t |
| S13 | 2 | 1 | 3 | 333 | 15 | 5 | 150μg ARN t |
| S14 | 2 | 1 | 3 | 333 | 15 | 5 | 150μg ARN t |
| S15 | 2 | 1 | 3 | 333 | 15 | 5 | 150μg ARN t |

Pour suivre l'évolution du *pool,* l'apparition de sites de restriction comportant 4 bases dans la population a été analysée par RFLP et révèle l'émergence de séquences sélectionnées pendant le processus SELEX qui correspondent à des sites de restriction spécifiques (Bartel DP et al., Science, 1993, 261, 5127, 1411-8).

Après quinze cycles de sélection, les séquences sont clonées à l'aide du kit de clonage TOPO-TA (Invitrogen) et analysées par séquençage.

- *Tests de liaison*

La liaison des différents aptamères (ou du pool initial, comme contrôle) aux cellules PC12 MEN 2A est réalisée dans des plaques comportant 24 puits (expériences réalisées en triplicat), avec de l'ARN marqué au 5'-$^{32}$P. $10^5$ cellules par puits sont incubées avec différentes concentrations d'aptamères dans 200 μl de RPMI pendant 10 min à 37°C en présence de 100 μg/ml de polyinosine, en tant que compétiteur non-spécifique. Après plusieurs lavages, les séquences liées sont récupérées dans 350 μl de SDS à 0,6 % et la quantité de radioactivité récupérée est rapportée au nombre de cellules en mesurant la teneur en protéine dans chaque puits.

Les constantes de dissociation (Kd) et le nombre de cibles (Cmax) pour chaque aptamère sont déterminés :

* par analyse Scatchard selon l'équation suivante :

$$[\text{aptamère lié}]/[\text{aptamère}] = -(1/Kd) \times [\text{aptamère lié}] + [Cmax]/Kd$$

* ou par analyse selon la méthode de Lineweaver-Burk, selon l'équation suivante :

$$1/[\text{aptamère lié}]=Kd/([Cmax] \times [\text{aptamère}]) + 1/[Cmax].$$

La liaison des séquences individuelles aux différentes lignées cellulaires est réalisée dans les mêmes conditions, mais à une seule concentration de 50 nM.

**EXEMPLE 3 : Résultats**

**- SELEX *ex vivo* : sélection des aptamères**

Pour identifier les aptamères qui reconnaissent le récepteur Ret, un protocole SELEX modifié a été mis en oeuvre, comme précisé à l'exemple 1 :

- le procédé est mis en oeuvre sur des cellules intactes, à savoir une lignée cellulaire dérivée des cellules PC 12 (PC 12 MEN 2A), exprimant le récepteur mutant humain $Ret^{C634Y}$.
- des ARNs 2'-F-Pyrimidines sont utilisés, pour augmenter la résistance des aptamères aux nucléases présentes dans les liquides biologiques
- deux étapes de contre sélection sont mises en oeuvre à chaque cycle : (1) sur des cellules PC12 qui n'expriment pas le récepteur Ret humain et (2) sur des cellules PC 12 MEN 2B, qui expriment un allèle muté au niveau du domaine de tyrosine kinase intracellulaire du récepteur Ret ($Ret^{M918T}$) pour optimiser la sélection d'aptamères spécifiques du récepteur Ret.

Le contexte utilisé a permis de sélectionner des aptamères capables de cibler des épitopes de surface spécifiques des cellules PC 12 MEN 2A ainsi que des aptamères aptes à reconnaître la portion extracellulaire du récepteur Ret.

Cette technique a l'avantage d'éviter l'utilisation de récepteur Ret recombinant.

69 clones sont obtenus et séquencés, à partir du pool de séquences obtenues au 15$^{ème}$ tour et liées aux cellules PC 12 MEN 2A de manière saturable avec un Kd d'environ 100 nM. Les résultats sont illustrés au Tableau III ci-après

Tableau III : Résultats de la sélection (les séquences sont données sans les régions fixes R1 (SEQ ID NO :1) et R2 (SEQ ID NO :2) qui les encadrent et permettent une amplification par RT-PCR)

| Code de la séquence (nombre de clones) | SEQUENCE (R) | liaison à 100nM sur PC12 MEN 2A | Kd sur PC12 MEN2A (nM) | nombre de cibles/ cellule (x1000) |
|---|---|---|---|---|
| **D14(23)** | GGCCATAGCGCACCACCAAGAGCAAAT<br>CCCTAAGCGCGACTCGAGTGAGC | - | | |
| **D12(21)** | GGGCUUCAUAAGCUACACCGGCCAAC<br>GCAGAAAUGCCUUAAGCCCGAGUU | + | **67 ± 7** | **102 ± 25** |
| **D30(7)** | AGGCGAGCCCGACCACGTCAGTATGC<br>T<br>AGACAACAACGCCCGCGTGGTAC | + | **74 ± 25** | **212 ± 27** |
| **D71(5)** | GGCCCUUAACGCAAAAACGAAGGAUC<br>A<br>UCGAUUGAUCGCCUUAUGGGCU | + | **53 ± 7** | **248 ± 42** |
| **D42(4)** | GACCCGUAUGAAGGUGGCGCAGGACA<br>CGACCGUCUGCAAUGAGCGAGC | - | | |
| **D20(2)** | GGGCCAATCGAAGCCGGTAATTCCCAA<br>ACTAACGTGCAAACTGCACCCGC | - | | |
| **D76** | GGCTTACACGGAGAAACAAGAGAGCG<br>G<br>CCCAAACTTGATTGACAGTGGCC | - | | |
| **D60** | CCGACCTGTACAGCAGTTAGTTACACG<br>TTTGAAACAACCGGCGTTCGAGC<br>CCCCGCTTTTTGACGTGATCGAACGCG | - | | |

(suite)

| Code de la séquence (nombre de clones) | SEQUENCE (R) | liaison à 100nM sur PC12 MEN 2A | Kd sur PC12 MEN2A (nM) | nombre de cibles/ cellule (x1000) |
|---|---|---|---|---|
| **D32** | TATCAGTAACGTCAGCAGTCGAGC CAAAGCGTGTATTCTCGTGAGCCGACC | - | | |
| **D33** | ATCGTTGCGAACATCCCCGGAACG CCGCGGTCTGTGGGACCCTTCAGGATG | - | | |
| **D87** | AAGCGGCAACCCATGCGGGCC GCGGTATGTAGGGAATAGCACTTTTTTT | - | **32 ± 5** | **102 ± 58** |
| **D24** | GCGTATACCTACACCGCAGCG GCGCGGGAATAGTATGGAAGGATACGT | + | **35 ± 3** | **110 ± 47** |
| **D4** | ATACCGTGCAATCCAGGGCAACG | + | | |

Deux séquences (D14 et D12) dominent la sélection et constituent plus de 50 % des clones. Quatre autres séquences sont moins abondantes (25 % des clones) et les autres sont présentes seulement une fois (sept séquences).
Du fait de la complexité de la cible (cellules entières vivantes), aucune similarité n'a été observée entre les différentes séquences d'aptamères sauf en ce qui concerne les clones D24 et D4 qui partagent certains motifs et une structure commune (voir figure 5A, formule II et figures 11 et 12).
La capacité de liaison de chaque aptamère aux cellules PC12 MEN 2A a été testée.
Toutes les séquences retrouvées plus d'une fois ont ainsi été testées ainsi que des séquences présentes en moindre quantité (dont D4 et D24). En dépit de son abondance, D14 ne lie pas les cellules PC12 MEN 2A, de manière significative au dessus du bruit de fond.
D'autres séquences lient les cellules PC12 MEN 2A, avec un Kd compris entre 30 et 70 nM.
La plupart des aptamères ne lient pas les cellules PC12 parentales, les cellules de carcinome de vessie de rat (NBTII) et les cellules humaines HeLa.

**- Action de l'aptamère D4 sur le récepteur Ret**

L'activation du récepteur Ret normal dans les cellules normales (*Cn*) se fait par l'interaction avec le co-récepteur GRF-alpha pour plusieurs facteurs trophiques, dont le plus répandu est le GDNF (*Glial Derived Growth Factor*) (figure 4). Dans les cellules exprimant la forme normale de Ret, la cascade n'est activée qu'en présence de GDNF. L'inhibition de la phosphorylation de Ret par le GDNF dans ces cellules en présence de D4 est la preuve que D4 interagit avec la voie de signalisation Ret, ce que confirme l'absence d'activité de D4 sur l'activation par un autre facteur trophique, le NGF, dont l'activité sur Erk n'est pas médiée par Ret. Le fait que D4 n'ait pas d'activité non plus sur l'activation de Ret et Erk dans les cellules MEN 2B dans lesquelles la mutation est intracellulaire suggère que l'interaction entre D4 et Ret concerne la partie extracellulaire de Ret, peut être au niveau du site de dimérisation. Toutefois, ceci n'est pas prouvé.
De façon très remarquable, l'activité de D4 conduit à une réversion du phénotype transformé sur les cellules dans lesquelles Ret est constitutivement activé. Ces cellules en culture prennent un morphotype « *neuronal-like* » avec extension axonales. D4, mais pas D4Sc qui est un D4 déstructuré, de même composition chimique mais sans ordonnancement de sa séquence en une structure active, induit une réduction très significative du nombre d'extensions axonales et ramène le phénotype cellulaire à un phénotype de cellules non activées, aussi bien dans une lignée neuroendocrine (PC 12) que dans une lignée fibroblastique (NIH 3T3).
Le récepteur mutant $Ret^{C634Y}$, exprimé par les cellules PC12 MEN 2A, forme des homodimères à la surface cellulaire qui conduit à l'activation constitutive de son activité tyrosine kinase (Santoro M et al., Science, 1995, 20, 267, 5196, 381-383) et induit plusieurs cascades de signalisation en aval, y compris l'activation de la kinase Erk (Colucci-D'Amato et al., J. Biol. Chem., 2000, 275, 19306-19314 ; Jhiang SM, Oncogene, 2000, 19, 5590-5597). En utilisant comme système cellulaire *in vitro* des cellules PC12 exprimant l'oncogène $Ret^{C634Y}$, la capacité de chaque aptamère à inhiber l'autophosphorylation du récepteur $Ret^{C634Y}$ et la signalisation en aval dépendante du récepteur (figure 6) est évaluée.
Les cellules PC12 MEN 2A sont incubées une nuit avec l'un des aptamères suivants : D4 (SEQ ID NO :3), D12 (SEQ ID NO :4), D30 (SEQ ID NO :8) et D71 (SEQ ID NO :14), à une concentration finale de 150 nM. Les lysats cellulaires sont analysés par immunoempreinte avec des anticorps anti-(Tyr-phosphorylée) Ret (figure 6B) ou des anticorps anti-phospho-Erk (figure 6B). Comme cela a déjà été démontré (Colucci et al. précité), les taux de récepteur Ret et de protéine Erk phosphorylés sont constitutivement élevés dans les cellules PC12 MEN 2A non traitées, en raison de la présence de l'allèle actif $Ret^{C634Y}$. Quelques uns des aptamères testés inhibent l'auto-phosphorylation du récepteur $Ret^{C634Y}$ et la phosphorylation de la protéine Erk qui en découle, en comparaison avec la banque comminatoire de départ et avec d'autres aptamères (figure 6A). En utilisant ces conditions expérimentales, l'aptamère D4 se révèle l'inhibiteur le plus efficace et a donc été utilisé pour les études suivantes.

Dans ces conditions, une expérience dose-réponse (figure 6B, gauche) révèle qu'une concentration de 200 nM d'aptamère D4 est suffisante pour inhiber l'autophosphorylation du récepteur $Ret^{C634Y}$ jusqu'à 70 % et pour réduire de manière très importante la phosphorylation de la protéine Erk. Un traitement d'une heure des cellules avec 200 nM d'aptamère D4 est suffisant pour inhiber de manière significative l'autophosphorylation du récepteur $Ret^{C634Y}$ et pour complètement abolir la phosphorylation de la protéine Erk (figure 6B, droite).

Dans toutes les expériences, l'inhibition de la phosphorylation de la protéine Erk est plus rapide et plus quantitative que l'inhibition de la phosphorylation du récepteur $Ret^{C634Y}$, sans doute en raison de sensibilités différentes des deux processus pour modifier l'activité tyrosine kinase du récepteur Ret.

La structure secondaire prédite de l'aptamère D4 est illustrée à la figure 5A ainsi que celle de l'aptamère D24. La comparaison des deux structures suggère que la liaison aux cellules n'est pas dépendante de la séquence de la queue ou de la boucle apicale. En effet, si l'on remplace la boucle apicale par une boucle extrastable comprenant quatre nucléotides (UUGC) ou en supprimant les nucléotides représentés par $R_4$ et $R_5$, tels que définis ci-dessus, on n'observe aucune différence significative dans la liaison aux cellules PC12 MEN 2A. Toutefois, c'est l'aptamère D4 complet qui est le produit le plus actif dans l'inhibition de la voie de signalisation induite par le récepteur $Ret^{C634Y}$. Un ARN 2'-F-Py de composition identique mais avec une séquence déstructurée (D4Sc) est inefficace aussi bien pour la liaison et que l'inhibition.

L'aptamère D4 reconnaît les cellules PC12 MEN 2A, avec un Kd estimé de 35 nM (figure 5B) ; de plus, il ne reconnaît ni les cellules PC12 parentales, ni les cellules de rat NBTII, ni les cellules humaines HeLa qui n'expriment pas le récepteur RET.

Dans la mesure où l'aptamère D4 a été sélectionné sur des cellules exprimant le récepteur mutant $Ret^{C634Y}$, on a essayé de déterminer si l'aptamère D4 pourrait également inhiber le récepteur Ret sauvage. Dans ce but, une lignée cellulaire dérivée des cellules PC12 exprimant le récepteur Ret sauvage (PC12/wt) a été utilisée. Les cellules ont été stimulées avec un mélange contenant du GDNF et du GFRα1 soluble et soient traitées avec l'aptamère D4 ou avec la banque combinatoire de départ, comme contrôle négatif. Comme illustré à la figure 7A, seul l'aptamère D4 (et non la banque combinatoire d'ARN contrôle) inhibe, de manière importante, la phosphorylation du récepteur Ret induite par le GDNF (Figure 7A) et de la protéine Erk (Figure 7B). Un effet inhibiteur similaire a été observé avec les cellules PC12-α1/wt, une lignée cellulaire dérivée des cellules PC12 qui exprime de manière stable à la fois le récepteur Ret humain et le GFRα1. Il est important de noter que l'aptamère D4 est inactif sur la voie de signalisation induite par le NGF sur le récepteur de la tyrosine kinase, TrkA. Après stimulation par le NGF des cellules, le traitement par l'aptamère D4 ne modifie pas la quantité de phospho-Erk, ce qui indique que l'inhibition induite par l'aptamère D4 de la phosphorylation de la protéine Erk est spécifique de la voie de signalisation intracellulaire du récepteur Ret stimulée par le GDNF (figure 7).

Bien que l'aptamère D4 lie les cellules PC12 MEN 2B, le traitement de ces cellules avec 200 nM de D4 pendant une heure (figure 7B) n'interfère pas avec la voie de signalisation induite par le récepteur monomérique $Ret^{M918T}$. Ceci confirme que l'inhibition de la phosphorylation de la protéine Erk par l'aptamère D4 est spécifique de la forme activée par dimérisation du récepteur Ret. Les activités kinase et biologique du récepteur $Ret^{M918T}$ bien que constitutives, répondent à une stimulation par le GDNF en présence de GFRα1 (Carlomagno F et al., Endocrinology, 1998, 139, 8, 3613-3619). Cependant, conformément à l'inhibition de l'activité du récepteur Ret sauvage (Ret wt) par l'aptamère D4, le traitement des cellules PC12 MEN 2B avec l'aptamère D4 abolit la sur-stimulation de la phosphorylation du récepteur Ret et de la protéine Erk, dépendante du GDNF. Ces expériences montrent que l'aptamère D4 inhibe à la fois les activités du récepteur Ret et de la protéine Erk par action directe sur le récepteur Ret et non par action sur d'autres cibles cellulaires, par exemple la tyrosine phosphatase.

**- Effets biologiques de l'aptamère D4 sur la différenciation et la transformation cellulaires, dépendantes du récepteur Ret**

L'observation que l'inhibition se produit dans tous les cas où la dimérisation du récepteur sauvage (wt) ou mutant est nécessaire à la voie de signalisation, indique également que la dimérisation est la cible de l'action de l'aptamère D4.

Dans ce but, l'extension axonale (ou crête neurale) a été mesurée comme le reflet de la différenciation dans les cellules PC12-α1/wt après stimulation par le GDNF (voir exemple 1).

Les cellules sont traitées avec 50 ng/ml de GDNF et le pourcentage de cellules contenant des extensions axonales est déterminé 24 et 48 heures après le traitement comme précisé à l'exemple 1. Comme illustré à la figure 8, les cellules présentent des processus d'extension axonale en réponse à une exposition de deux jours au GDNF (figure 8B) par rapport aux cellules contrôles non-stimulées (figure 8A). Le traitement des cellules avec l'aptamère D4 (figure 8C), mais non avec l'aptamère D4 déstructuré (D4Sc) utilisé comme contrôle (figure 8D), inhibe de manière significative le nombre et la longueur des axones, peut-être en empêchant la formation d'un complexe fonctionnel entre le récepteur Ret et GDNF/GFR$\alpha$1. Pour évaluer biochimiquement la différenciation, les taux de VGF dans des extraits cellulaires ont été déterminés après 48 heures de traitement. *Vgf* est un gène précoce qui est rapidement induit par le NGF et le GDNF dans les cellules PC12 (Salton SR, Mt Sinai J Med., 2003, 70, 2, 93-100). On observe (figure 8F) que dans les cellules traitées par le GDNF, l'expression de VGF est stimulée et conformément avec les effets phénotypiques rapportés ci-dessus, le traitement avec l'aptamère D4 mais non le traitement avec l'aptamère D4 déstructuré maintient des taux de VGF proches des taux basaux.

Après expression du récepteur $Ret^{C634Y}$ ou du récepteur $Ret^{M918T}$, les cellules NIH3T3 sont transformées et présentent des changements importants dans leur morphologie (Santoro et al., Science, 1995 précité). Les cellules NIH/MEN 2A et NIH/MEN 2B, qui expriment les récepteurs Ret mutants de manière stable, sont traitées avec l'aptamère D4 pendant 72 heures et les modifications morphologiques induites par cet aptamère sont analysées. Comme illustré à la figure 9, les cellules NIH/MEN 2A et les cellules NIH/MEN 2B ont une forme en fuseau, de longues protrusions et ont une apparence hautement réfringente (figures 9B et 9E respectivement). Les cellules NIH/MEN 2A traitées par l'aptamère D4 retrouvent une morphologie polygonale et plate similaire à celle des cellules parentales NIH3T3 (figure 9C), tandis qu'on n'observe aucun changement morphologique dans les cellules NIH/MEN 2B (figure 9F) ou dans les cellules NIH-Ras. Ceci est en accord avec les résultats précédents qui montrent que la voie de signalisation induite par le récepteur $Ref^{634Y}$ mais pas celle induite par le récepteur $Ret^{M918T}$ est inhibée par l'aptamère D4.

Par ailleurs, le traitement avec l'aptamère D4 déstructuré n'a aucun effet sur les différentes lignées cellulaires (figure 9D). En conséquence, un traitement de 72 heures des cellules NIH/MEN 2A avec l'aptamère D4 inhibe l'étendue des effets de l'activation de la protéine Erk par la phosphorylation à la fois du récepteur $Ret^{C634Y}$ et de la protéine Erk.

**<u>EXEMPLE 4 :</u> Utilisation de l'aptamère D4 pour cribler des produits interagissant avec le récepteur Ret ou des cibles formant un complexe avec la protéine Ret sur les cellules PC12 MEN 2A**

Afin de valider l'utilisation de l'aptamère D4 pour cribler des molécules interagissant avec le récepteur Ret ou des cibles formant un complexe avec ladite protéine sur les Cellules PC12 MEN 2A, deux approches ont été utilisées :

1-l'aptamère D4 radiomarqué avec du $^{32}P$ a été incubé à 50nM avec des monocouches de cellules PC12 MEN 2A en présence de 400nM de différents aptamères sélectionnés soit sur cellules, soit contre la protéine recombinante Ret isolée. Après plusieurs lavages, la quantité d'aptamère D4 lié est quantifiée. En comparant le taux de fixation de l'aptamère D4 en fonction des molécules utilisées, on peut constater que seul l'aptamère D12 et surtout l'aptamère E38, dont la cible est la récepteur Ret, ont été capables de diminuer la fixation de l'aptamère D4 sur les cellules. On peut donc conclure que ces 2 aptamères se fixent sur une cible en rapport avec la protéine Ret, soit directement sur ladite protéine au même site ou non que l'aptamère D4, soit sur une autre cible présente dans un complexe avec Ret.

2- l'aptamère E38 radiomarqué avec du $^{32}P$, a été incubé à 100nM avec des monocouches de cellules PC12 MEN 2A en présence d'une concentration croissante d'aptamère D4. Après plusieurs lavages, la quantité d'aptamère E38 lié aux cellules a été quantifiée. On peut constater une inhibition croissante de la fixation de l'aptamère E38 en fonction de la concentration d'aptamère D4. On peut donc conclure que l'aptamère E38 se fixe sur une cible en rapport avec la protéine Ret, soit directement sur ladite protéine au même site ou non que l'aptamère D4, soit sur une autre cible présente dans un complexe avec Ret.

## Revendications

**1.** Procédé d'identification de ligands ou aptamères spécifiques d'un récepteur membranaire à activité tyrosine kinase (RPTK pour *receptor protein-tyrosine kinase*), exprimé sous une forme activée ou non, par des cellules, à partir d'un mélange d'acides nucléiques, lequel procédé comprend au moins les étapes suivantes :

(a) la mise en contact d'un mélange d'acides nucléiques avec des cellules n'exprimant pas ledit récepteur membranaire à activité tyrosine kinase ou l'exprimant sous une forme non activée, (cellules $C_N$), lesdites cellules présentant le même type cellulaire que des cellules exprimant le même récepteur membranaire à activité tyrosine kinase mais sous une forme activée, du fait de l'existence d'une mutation dans le domaine extracellulaire (cellules $C_{Te}$) ;
(b) la récupération d'un premier sous-ensemble S1 d'acides nucléiques qui ne se lient pas aux cellules $C_N$, lors de l'étape (a) ;
(c) la mise en contact dudit premier sous-ensemble S1 avec des cellules $C_i$, présentant le même type cellulaire que les cellules $C_{Te}$, mais exprimant ledit récepteur membranaire à activité tyrosine kinase, muté dans sa partie intracellulaire, lesdites cellules $C_i$ présentant un phénotype du même type que celui des cellules $C_{Te}$ ;
(d) la récupération d'un deuxième sous-ensemble S2 d'acides nucléiques qui ne se lient pas aux cellules $C_i$ lors de l'étape (c) ;
(e) la mise en contact du deuxième sous-ensemble S2 avec les cellules $C_{Te}$ ;
(f) la récupération des acides nucléiques qui se lient auxdites cellules $C_{Te}$, c'est-à-dire ceux présentant une affinité élevée vis-à-vis des cellules exprimant ledit récepteur membranaire à activité tyrosine kinase muté dans le domaine extracellulaire, après dissociation des complexes cellules-acides nucléiques :
(g) l'amplification desdits acides nucléiques à affinité élevée pour les cellules exprimant ledit récepteur membranaire à activité tyrosine kinase muté dans le domaine extracellulaire, de manière à obtenir un mélange d'acides nucléiques, enrichi en acides nucléiques ayant une affinité élevée pour lesdites cellules $C_{Te}$ et
(h) l'identification des ligands ou aptamères spécifiques des cellules exprimant des récepteurs membranaires à activité tyrosine kinase (RPTK pour *receptor protein-tyrosine kinase*) sous une forme activée, à partir du mélange obtenu en (g).

**2.** Procédé selon la revendication 1, **caractérisé en ce que** l'on répète les étapes (a)-(g), en utilisant les mélanges enrichis en ligands ou aptamères du cycle précédent, jusqu'à l'obtention d'au moins un aptamère, dont l'affinité définie par sa constante de dissociation (Kd), est mesurable et convient pour une utilisation pharmaceutique.

**3.** Procédé selon la revendication 1 ou la revendication 2, **caractérisé en ce que** la banque combinatoire d'acides nucléiques de départ contient au moins $10^2$ acides nucléiques, de préférence entre $10^9$ et $10^{15}$ acides nucléiques et est avantageusement constituée d'acides nucléiques comprenant des séquences aléatoires comprenant respectivement à leurs extrémités 5' et 3', des séquences fixes permettant une amplification par PCR, de préférence les séquences SEQ ID NO :1 et SEQ ID NO: 2 ou un fragment d'au moins 8 nucléotides de ces séquences.

**4.** Procédé selon l'une quelconque des revendications 1 à 3, **caractérisé en ce que** lesdites séquences aléatoires contiennent chacune entre 10 et 1000 nucléotides, de préférence 50 nucléotides, et sont avantageusement des ADN, des ARN ou des acides nucléiques modifiés.

**5.** Procédé selon l'une quelconque des revendications 1 à 4, **caractérisé en ce que** l'identification des ligands ou aptamères spécifiques des cellules $C_{Te}$ selon l'étape (h) comporte une évaluation de l'activité biologique desdits aptamères sur lesdites cellules $C_{Te}$.

**6.** Procédé selon la revendication 5, **caractérisé en ce que** lesdites activités biologiques qui sont avantageusement évaluées sont les suivantes :

(a) inhibition ou activation de l'auto-phosphorylation du RPTK,
(b) inhibition ou activation de la cascade d'activation de kinases,
(c) inhibition de la phosphorylation du RPTK normal de cellules $C_N$ activées par une stimulation convenable, et
(d) réversion du phénotype associé à une activation du RPTK.

**7.** Procédé selon l'une quelconque des revendications 1 à 6, **caractérisé en ce que** ledit RPTK est sélectionné dans le groupe constitué par les récepteurs membranaires suivants : EGFR *(Epithelial Growth Factor Receptor),* InsulinR *(Insulin Receptor),* PDGFR *(Platelet-derived Growth Factor Receptor),* VEGFR *(Vascular Endothelial Growth Factor Receptor),* FGFR *(Fibroblast Growth Factor Receptor),* NGFR *(Nerve Growth Factor Receptor),* HGFR *(Hepatocyte Growth Factor Receptor),* EPHR *(Ephrin Receptor),* AXL (Tyro 3 PTK), TIE *(Tyrosine Kinase Receptor in endothelial cells),* RET *(Rearranged During Transfection),* ROS (RPTK exprimé dans certaines cellules épithéliales) et LTK *(Leukocyte Tyrosine Kinase).*

**8.** Procédé selon l'une quelconque des revendications 1 à 7, **caractérisé en ce que** ledit aptamère ou ledit ligand reconnaît un récepteur Ret sous une forme activée et notamment le récepteur Ret activé par mutation au niveau d'une cystéine localisée dans le domaine extracellulaire, de préférence au niveau des codons 609, 611, 618, 620 ou 634, ledit procédé comprenant :

(a) la mise en contact d'un mélange d'acides nucléiques avec des cellules $C_N$ n'exprimant pas de récepteur Ret sous une forme activée,
(b) la récupération d'un premier sous-ensemble S1 d'acides nucléiques qui ne se lient pas auxdites cellules $C_N$ lors de l'étape (a),
(c) la mise en contact dudit premier sous-ensemble S1 avec des cellules $C_i$ exprimant un récepteur Ret muté dans son domaine intracellulaire, notamment le récepteur muté $Ret^{M918T}$,
d) la récupération d'un deuxième sous-ensemble S2 d'acides nucléiques qui ne se lient pas auxdites cellules $C_i$,
e) la mise en contact du deuxième sous-ensemble S2 avec des cellules $C_{Te}$ exprimant un récepteur Ret activé par mutation dans le domaine extracellulaire, lequel récepteur est sélectionné dans le groupe constitué par les récepteurs Ret mutés portant une mutation sur l'une des cystéines localisées dans le domaine extracellulaire, de préférence au niveau des Cys609, Cys611, Cys618, Cys620 ou Cys 634, de préférence le récepteur $Ret^{C634Y}$,
f) la récupération des acides nucléiques liés auxdites cellules $C_{Te}$, c'est-à-dire présentant à la fois une affinité élevée et une spécificité de liaison pour les cellules exprimant un récepteur Ret muté tel que défini à l'étape e),
g) l'amplification desdits acides nucléiques obtenus à l'étape f), de manière à obtenir un mélange d'acides nucléiques, enrichi en acides nucléiques ayant une affinité élevée pour les cellules $C_{Te}$,
h) la répétition des étapes a)-g), jusqu'à l'obtention d'au moins un aptamère dont l'affinité pour les cellules $C_{Te}$, définie par sa constante de dissociation (Kd), est mesurable et convient pour une activité pharmacologique, et
i) l'identification des aptamères spécifiques des cellules exprimant un récepteur Ret dans sa forme activée, sélectionné à partir du mélange obtenu en h).

**9.** Procédé selon la revendication 8, **caractérisé en ce que** :

- les cellules $C_N$ sont notamment des cellules PC12 sauvages (référence ECACC N° 88022) ou des cellules NIH 3T3 sauvages (référence ECACC N° 93061524),
- les cellules $C_i$ et $C_{Te}$ sont obtenues en introduisant un oncogène porteur d'une mutation respectivement intracellulaire et extracellulaire dans des cellules $C_N$ en culture de façon à ce que celles-ci expriment l'oncogène.

**10.** Aptamère, **caractérisé en ce qu'**il est susceptible d'être obtenu par un procédé d'identification tel que défini aux revendications 1 à 9, **en ce qu'**il est spécifique des cellules exprimant un récepteur à activité tyrosine kinase sous une forme activée et **en ce qu'**il est sélectionné dans le groupe constitué par les aptamères de formule (I) :

$$R_1\text{-}R\text{-}R_2 \qquad (I),$$

dans laquelle :

$R_1$ représente 5' GGGAGACAAGAAUAAACGCUCAA 3' (SEQ ID NO:1) ou un fragment de 1 à 23 nucléotides de ladite SEQ ID NO: 1 ;

$R_2$ représente 5' AACGACAGGAGGCUCACAACAGGA 3' (SEQ ID NO :2) ou un fragment de 1 à 24 nucléotides de ladite SEQ ID NO :2 et
R représente une séquence aléatoire de 10 à 1000 nucléotides, de préférence de 50 nucléotides.

**11.** Aptamère selon la revendication 10, **caractérisé en ce que** R est de préférence sélectionné parmi les séquences suivantes :

| | |
|---|---|
| **D4** | 5'GCGCGGGAAUAGUAUGGAAGGAUACGUAUACCGUGCAAUCCAGGGCAACG 3' (SEQ ID NO :3) |
| **D12** | 5'GGGCUUCAUAAGCUACACCGGCCAACGCAGAAAUGCCUUAAGCCCGAGUU 3' (SEQ ID NO4) |
| **D14** | 5'GGCCAUAGCGCACCACCAAGAGCAAAUCCCUAAGCGCGACUCGAGUGAGC 3' (SEQ ID NO.5) |
| **D20** | 5'GGGCCAAUCGAAGCCGGUAAUUCCCAAACUAACGUGCAAACUGCACCCGC 3' (SEQ ID NO:6) |
| **D24** | 5'GCGGUAUGUAGGGAAUAGCACUUUUUUUGCGUAUACCUACACCGCAGCG 3' (SEQ ID NO:7) |
| **D30** | 5'AGGCGAGCCCGACCACGUCAGUAUGCUAGACAACAACGCCCGCGUGGUAC 3' (SEQ ID NO:8) |
| **D32** | 5'CCCCGCUUUUUGACGUGAUCGAACGCGUAUCAGUAACGUCAGCAGUCGAGC 3' (SEQ 1D NO: 9) |
| **D33** | 5'CAAAGCGUGUAUUCUCGUGAGCCGACCAUCGUUGCGAACAUCCCCGGAACG 3' (SEQ ID NO:10) |
| **D42** | 5'GACCCGUAUGAAGGUGGCGCAGGACACGACCGUCUGCAAUGAGCGAGC 3' (SEQ ID NO:11) |
| **D60** | 5'CCGACCUGUACAGCAGUUAGUUACACGUUUGAAACAACCGGCGUUCGAGC 3' (SEQ ID NO:12) |
| **D76** | 5'GGCUUACACGGAGAAACAAGAGAGCGGCCCAAACUUGAUUGACACUG GCC 3' (SEQ ID NO :13) |
| **D71** | 5'GGCCCUUAACGCAAAAACGAAGGAUCAUCGAUUGAUCGCCUUAUGGGCU 3' (SEQ ID NO:14) |
| **D87** | 5'CCGCGGUCUGUGGGACCCUUCAGGAUGAAGCGGCAACCCAUGCGGGCC 3' (SEQ ID NO:15) |

**12.** Aptamère selon la revendication 10 ou la revendication 11, **caractérisé en ce que** les riboses des purines sont porteurs d'une fonction hydroxyle sur le carbone en position 2', tandis que les riboses des pyrimidines portent un atome de fluor sur le carbone en position 2'.

**13.** Aptamère selon l'une quelconque des revendications 10 à 12, **caractérisé en ce qu'**il présente l'une des séquences suivantes : SEQ ID NO :31-33.

**14.** Aptamère selon l'une quelconque des revendications 10 à 12, **caractérisé en ce qu'**il présente la formule II suivante :

5' $R_4X_6X_5X_4X_3$GGAAUAG$X_2X_1R_3X'_1X'_2$CGUAUAC$X'_3X'_4X'_5X'_6R_5$ 3' (II),

dont la structure secondaire est représentée à la figure 10, et dans laquelle :

- les riboses des purines sont porteurs d'un groupement OH en 2' et les riboses des pyrimidines sont porteurs d'un atome de fluor en 2',
- $\mathbf{R_3}$ est présent ou absent et représente un renflement (ou boucle) apical comprenant

. une chaîne carbonée linéaire ou ramifiée sélectionnée dans le groupe constitué par des groupes alkyles en $C_6$-$C_{30}$ ou des groupes aryles en $C_6$-$C_{30}$ ;
. un polymère tel que du PEG ou du PEI ou autre.
. des groupements fonctionnels tels que biotine, streptavidine, peroxydase,
. d'autres molécules d'intérêt comme par exemple, des principes actifs, des étiquettes de marquage notamment fluorescentes ou des chélateurs pour radio isotopes
. une séquence nucléotidique naturelle ou modifiée ; de préférence $R_3$ représente les renflements ou boucles (1) à (4) suivants :

Boucle (1) : 5'UGGAAGGA 3' (SEQ ID NO :29)
Boucle (2) : 5' CUUUUUU 3' (SEQ ID NO:30)
Boucle (3) 5'GNPuA 3'
Boucle (4) 5'UNCG 3',

dans lesquels les riboses des purines sont porteurs d'une fonction hydroxyle sur le carbone en position 2', tandis que les riboses des pyrimidines portent un atome de fluor sur le carbone en position 2',
- $\mathbf{X_1, X'_1, X_2, X'_2, X_3, X'_3, X_4, X'_4, X_5, X'_5, X_6}$ **et** $\mathbf{X'_6}$ représentent Py ou Pu avec de préférence :

$X_1$ -$X'_1$ correspondant à C-G, A-U, G-C ou U-A
$X_2$-$X'_2$ correspondant à C-G, A-U, G-C ou U-A
$X_3$-$X'_3$ correspondant à C-G, A-U, G-C ou U-A
$X_4$-$X'_4$ correspondant à C-G, A-U, G-C ou U-A
$X_5$-$X'_5$ correspondant à C-G, A-U, G-C ou U-A
$X_6$-$X'_6$ correspondant à C-G, A-U, G-C ou U-A
N correspondant à G ou C ou A ou U
**Pu** correspondant à G ou A, dans lesquels les riboses sont porteurs d'un groupement OH en 2'
**Py** correspond à U ou C, dans lesquels les riboses sont porteurs d'un atome de fluor en 2' et

- **$R_4$** et **$R_5$** sont présents ou absents et représentent :

. une séquence nucléotidique naturelle ou modifiée, comprenant entre 1 et plusieurs milliers de nucléotides, de préférence entre 1 et 39 nucléotides ; une partie de ladite séquence nucléotidique ou ladite séquence comprenant de préférence l'une des séquences suivantes :

**$R_4$** à

5'-$R_1$-$Z_1$-3', avec $Z_1$=G: 5'GGGAGACAAGAAUAAACGCUCAAG 3' (SEQ ID NO : 18) ou 5'-$R_1$-$Z_1$-3', avec $Z_1$=GCGGUAU (SEQ ID NO :26) :
5'GGGAGACAAGAAUAAACGCUCAAGCGGUAU (SEQ ID NO :19), et

**$R_5$:**

5'-$Z_2$-$R_2$-3', avec $Z_2$= CAAUCCAGGGCAACG (SEQ ID NO :27) :
5' CAAUCCAGGGCAACGAACGACAGGAGGCUCACAACAGGA 3' (SEQ ID NO: 20) ou
5'-$Z_2$-$R_2$-3', avec Z2=ACCGCAGCG (SEQ ID NO :28) :
5' ACCGCAGCGAACGACAGGAGGCUCACAACAGGA 3' (SEQ ID NO 21);
5'GGGAGACAAGAAUAAACGCUCAAG 3' (SEQ ID NO: 18) ou
5'GGGAGACAAGAAUAAACGCUCAAGCGGUAU (SEQ ID NO:19), pour $R_4$ et
5' CAAUCCAGGGCAACGAACGACAGGAGGCUCACAACAGGA 3' (SEQ ID NQ 20) ou
5, ACCGCAGCGAACGACAGGAGGCUCACAACAGGA 3'(SEQ ID NO 21), pour $R_5$ ;

. une chaîne carbonée linéaire ou ramifiée sélectionnée dans le groupe constitué par des groupes alkyles en $C_6$-$C_{30}$ ou des groupes aryles en $C_6$-$C_{30}$ ;
. un polymère tel que du PEG ou du PEI ou autre.
. des groupements fonctionnels tels que biotine, streptavidine, peroxydase,
. d'autres molécules d'intérêt comme par exemple, des principes actifs, des étiquettes de marquage notamment fluorescentes ou des chélateurs pour radio isotopes

**15.** Aptamère selon la revendication 14, **caractérisé en ce que** $R_3$ représente 5' UGGAAGGA 3' (boucle (1)), $R_4$ représente la SEQ ID NO :18 et $R_5$ représente la SEQ ID NO :20, l'aptamère présentant une telle structure (famille D4) possédant à la fois des propriétés de liaison audit récepteur Ret dans sa forme activée et des propriétés d'inhibition de l'activité dudit récepteur.

**16.** Aptamère selon la revendication 15, **caractérisé en ce qu'**il présente la séquence SEQ ID NO :22.

**17.** Aptamère selon la revendication 14, **caractérisé en ce que** $R_3$ représente 5' CUUUUUU 3' (boucle (2)), 5' GNPuA 3' (boucle (3)) ou 5' UNCG 3' (boucle (4)), $R_4$ comprend de 1 à 30 nucléotides sélectionnés dans la SEQ ID NO : 19 ou de 1 à 24 nucléotides sélectionnés dans la SEQ ID NO :18 et $R_5$ comprend de 1 à 33 nucléotides de la SEQ ID NO :21 ou de 1 à 39 nucléotides sélectionnés dans la SEQ ID NO :20, l'aptamère présentant une telle structure ne possédant que des propriétés de liaison audit récepteur Ret dans sa forme activée et notamment au récepteur Ret muté dans son domaine extracellulaire.

**18.** Aptamère selon la revendication 17, **caractérisé en ce que** $R_3$ représente 5' CUUUUUU 3' (boucle (2)), $R_4$ représente la SEQ ID NO :19 et $R_5$ représente la SEQ ID NO :21.

**19.** Aptamère selon la revendication 17 ou la revendication 18, **caractérisé en ce qu'**il présente la SEQ ID NO : 25.

**20.** Aptamère selon la revendication 14, **caractérisé en ce que** $R_3$ représente 5' UGGAAGGA 3' (boucle (1)), $R_4$ et $R_5$ sont absents, l'aptamère présentant une telle structure ne possédant que des propriétés de liaison audit récepteur Ret dans sa forme activée et **en ce qu'**il présente la séquence SEQ ID NO :23.

**21.** Réactif de diagnostic d'une tumeur, **caractérisé en ce qu'**il est constitué par un aptamère selon l'une quelconque des revendications 10 à 20.

**22.** Réactif selon la revendication 21, **caractérisé en ce qu'**il correspond à un aptamère de formule II :

$5'R_4X_6X_5X_4X_3GGAAUAGX_2X_1R_3X'_1X'_2CGUAUACX'_3X'_4X'_5X'_5R_5,3'$, dans laquelle $R_3$, $R_4$ et $R_5$ sont absents.

**23.** Réactif selon la revendication 22, **caractérisé en ce qu'**il correspond à un aptamère de séquence :

<u>5'GUAGGGAAUAGCACGUAUACCUAC 3'</u>(SEQ ID NO :24).

**24.** Réactif selon la revendication 21, **caractérisé en ce qu'**il correspond à un aptamère de formule II, dans laquelle $R_3$ représente 5' CUUUUUU 3' et **en ce qu'**il correspond à la séquence SEQ ID NO :25.

**25.** Réactif de diagnostic ou de détection du récepteur Ret sous une forme activée ou non, **caractérisé en ce qu'**il est constitué par au moins un aptamère selon l'une quelconque des revendications 10 à 20.

**26.** Médicament, **caractérisé en ce qu'**il comprend un aptamère selon l'une quelconque des revendications 10 à 20 qui présente à la fois une capacité de liaison à un récepteur RPTK et une action inhibitrice vis-à-vis dudit récepteur sous une forme activée.

**27.** Médicament destiné au traitement d'une tumeur, **caractérisé en ce qu'**il comprend un aptamère selon l'une quelconque des revendications 10 à 20, qui présente à la fois une capacité de liaison à un récepteur RPTK activé et notamment au récepteur muté dans le domaine extracellulaire et notamment au récepteur Ret muté au niveau d'une des cystéines localisées dans le domaine extracellulaire (codons 609, 611, 618, 620 et 634) et une action inhibitrice vis-à-vis de ce récepteur muté.

**28.** Médicament selon la revendication 26 ou la revendication 27, **caractérisé en ce qu'**il correspond à un aptamère de la famille des aptamères D4, tels que définis aux revendications 11, 14 ou 15.

**29.** Composition pharmaceutique, **caractérisée en ce qu'**elle comprend un aptamère selon l'une quelconque des revendications 10 à 20, qui présente à la fois une capacité de liaison à un récepteur RPTK et une action inhibitrice vis-à-vis dudit récepteur sous sa forme activée.

**30.** Composition pharmaceutique, **caractérisée en ce qu'**elle comprend :

- un aptamère selon l'une quelconque des revendications 10 à 20, qui présente à la fois une capacité de liaison à un récepteur RPTK activé et notamment à un récepteur muté dans le domaine extracellulaire et notamment au récepteur Ret muté au niveau d'une des cystéines localisées dans le domaine extracellulaire (codons 609, 611, 618, 620 et 634) et une action inhibitrice vis-à-vis de ce récepteur muté,
- une autre molécule anticancéreuse et
- au moins un véhicule pharmaceutiquement acceptable.

**31.** Utilisation d'un aptamère qui présente à la fois une capacité de liaison à un récepteur RPTK et une action inhibitrice vis-à-vis de ce récepteur RPTK pour le criblage de produits interagissant avec le récepteur RPTK et pouvant l'inhiber ou non.

**32.** Utilisation d'un aptamère qui présente à la fois une capacité de liaison à un récepteur RPTK activé et notamment au récepteur Ret muté au niveau d'une des cystéines localisées dans le domaine extracellulaire (codons 609, 611, 618, 620 et 634) et une action inhibitrice vis-à-vis de ce récepteur RPTK activé pour le criblage de produits interagissant avec ledit récepteur RPTK.

**33.** Procédé de criblage de produits interagissant avec un récepteur RPTK ou des cibles formant un complexe avec ledit RPTK sous une forme activée ou non, lequel procédé est **caractérisé en ce qu'**il comprend :

- la mise en contact de cellules exprimant des RPTK sous une forme activée ou non avec la substance à tester,
- l'ajout, dans des conditions convenables, d'un aptamère selon l'une quelconque des revendications 10 à 20, avant, en même temps ou après la substance à tester,
- l'évaluation de la fixation compétitive entre l'aptamère et la molécule à tester (par exemple : par mesure de radioactivité, de fluorescence, de luminescence, de résonance plasmonique de surface, BRET, FRET, ou toute autre technique de mise en évidence d'une interaction moléculaire).

**34.** Procédé selon la revendication 33, **caractérisé en ce qu'**après identification des substances se liant de manière compétitive avec l'aptamère aux cellules présentant des RPTK, l'effet de ces substances sur l'activité biologique desdits cellules peut être évalué pour trouver des substances qui inhibent ou activent lesdites activités biologiques ces cellules présentant des RPTK.

## Claims

**1.** Method for identifying ligands or aptamers specific for a membrane receptor protein-tyrosine kinase (RPTK), expressed in an activated or nonactivated form, by cells, using a mixture of nucleic acids, which method comprises at least the following steps:

(a) bringing a mixture of nucleic acids into contact with cells not expressing said membrane receptor protein-tyrosine kinase or expressing it in a nonactivated form ($C_N$ cells), said cells having the same cell type as cells expressing the same membrane receptor protein-tyrosine kinase but in an activated form, due to the existence of a mutation in the extracellular domain ($C_{Te}$ cells);
(b) recovering a first subset S1 of nucleic acids which do not bind to the $C_N$ cells, in step (a);
(c) bringing said first subset S1 into contact with $C_i$ cells, having the same cell type as the $C_{Te}$ cells, but expressing said membrane receptor protein-tyrosine kinase mutated in its intracellular part, said $C_i$ cells exhibiting a phenotype of the same type as that of the $C_{Te}$ cells;
(d) recovering a second subset S2 of nucleic acids which do not bind to the $C_i$ cells in step (c);
(e) bringing the second subset S2 into contact with the $C_{Te}$ cells;
(f) recovering the nucleic acids which bind to said $C_{Te}$ cells, i.e. those exhibiting a high affinity with respect to the cells expressing said membrane receptor protein-tyrosine kinase mutated in the extracellular domain, after dissociation of the cell-nucleic acid complexes;
(g) amplifying said nucleic acids with high affinity for the cells expressing said membrane receptor protein-tyrosine kinase mutated in the extracellular domain, so as to obtain a mixture of nucleic acids, enriched in nucleic acids having a high affinity for said $C_{Te}$ cells, and
(h) identifying the ligands or aptamers specific for the cells expressing membrane receptor protein-tyrosine kinases (RPTKs) in an activated form, from the mixture obtained in (g).

**2.** Method according to Claim 1, **characterized in that** steps (a)-(g) are repeated using the mixtures enriched in ligands or aptamers from the preceding cycle, until at least one aptamer is obtained, the affinity of which, defined by its dissociation constant (Kd), can be measured and is suitable for pharmaceutical use.

**3.** Method according to Claim 1 or Claim 2, **characterized in that** the starting nucleic acid combinatorial library contains at least $10^2$ nucleic acids, preferably between $10^9$ and $10^{15}$ nucleic acids, and advantageously consists of nucleic acids comprising random sequences comprising, respectively at their 5' and 3' ends, fixed sequences for PCR amplification, preferably the sequences SEQ ID NO:1 and SEQ ID NO:2 or a fragment of at least 8 nucleotides of these sequences.

**4.** Method according to any one of Claims 1 to 3, **characterized in that** said random sequences each contain between 10 and 1000 nucleotides, preferably 50 nucleotides, and are advantageously DNAs, RNAs or modified nucleic acids.

**5.** Method according to any one of Claims 1 to 4, **characterized in that** the identification of the ligands or aptamers specific for the $C_{Te}$ cells according to step (h) comprises an evaluation of the biological activity of said aptamers on said $C_{Te}$ cells.

**6.** Method according to Claim 5, **characterized in that** said biological activities which are advantageously evaluated are the following:

(a) inhibition or activation of the auto-phosphorylation of the RPTK,
(b) inhibition or activation of the kinase activation cascade,
(c) inhibition of the phosphorylation of the normal RPTK of $C_N$ cells activated by suitable stimulation, and
(d) reversion of the phenotype associated with activation of the RPTK.

**7.** Method according to any one of Claims 1 to 6, **characterized in that** said RPTK is selected from the group consisting of the following membrane receptors:

EGFR (Epithelial Growth Factor Receptor), InsulinR (Insulin Receptor), PDGFR (Platelet-derived Growth Factor Receptor), VEGFR (Vascular Endothelial Growth Factor Receptor), FGFR (Fibroblast Growth Factor Receptor), NGFR (Nerve Growth Factor Receptor), HGFR (Hepatocyte Growth Factor Receptor), EPHR (Ephrin Receptor), AXL (Tyro 3 PTK), TIE (Tyrosine Kinase Receptor in endothelial cells), RET (Rearranged During Transfection), ROS (RPTK expressed in certain epithelial cells) and LTK (Leukocyte Tyrosine Kinase).

**8.** Method according to any one of Claims 1 to 7, **characterized in that** said aptamer or ligand recognises a Ret receptor in an activated form, and in particular the Ret receptor activated by mutation at a cysteine located in the extracellular domain, preferably at codons 609, 611, 618, 620 or 634, said method comprising:

(a) bringing a mixture of nucleic acids into contact with $C_N$ cells not expressing any Ret receptor in an activated form,
(b) recovering a first subset S1 of nucleic acids which do not bind to said $C_N$ cells, in step (a),
(c) bringing said first subset S1 into contact with $C_i$ cells expressing a Ret receptor, mutated in its intracellular domain, in particular the mutated receptor $Ret^{M918T}$,
(d) recovering a second subset S2 of nucleic acids which do not bind to said $C_i$ cells,
(e) bringing the second subset S2 into contact with $C_{Te}$ cells expressing a Ret receptor activated by mutation in the extracellular domain, which receptor is selected from the group consisting of mutated Ret receptors carrying a mutation on one of the cysteines located in the extracellular domain, preferably at Cys609, Cys611, Cys618, Cys620 or Cys634, preferably the $Ret^{C634Y}$ receptor,
(f) recovering the nucleic acids bound to said $C_{Te}$ cells, i.e. exhibiting both a high affinity and a binding specificity for the cells expressing a mutated Ret receptor as defined in step (e),
(g) amplifying said nucleic acids obtained in step (f), so as to obtain a mixture of nucleic acids, enriched in nucleic acids having a high affinity for the $C_{Te}$ cells,
(h) repeating steps (a)-(g), until at least one aptamer is obtained, the affinity of which for the $C_{Te}$ cells, defined by its dissociation constant (Kd), is measurable and suitable for a pharmacological activity, and
(i) identifying the aptamers specific for the cells expressing a Ret receptor in its activated form, selected from the mixture obtained in (h).

**9.** Method according to Claim 8, **characterized in that**:

- the $C_N$ cells are in particular wild-type PC12 cells (reference ECACC No. 88022) or wild-type NIH 3T3 cells (reference ECACC No. 93061524),
- the $C_i$ and $C_{Te}$ cells are obtained by introducing an oncogene bearing a mutation, respectively intracellular and extracellular, in $C_N$ cells in culture in such a way that the latter express the oncogene.

**10.** Aptamer, **characterized in that** it can be obtained by means of a method of identification as defined in Claims 1 to 9, **in that** it is specific for cells expressing a receptor protein-tyrosine kinase in an activated form and **in that** it is selected from the group consisting of the aptamers of formula (I):

$$R_1\text{-}R\text{-}R_2 \qquad (I),$$

in which:

$R_1$ represents 5' GGGAGACAAGAAUAAACGCUCAA 3' (SEQ ID NO:1) or a fragment of 1 to 23 nucleotides of said SEQ ID NO:1;
$R_2$ represents 5' AACGACAGGAGGCUCACAACAGGA 3' (SEQ ID NO:2) or a fragment of 1 to 24 nucleotides of said SEQ ID NO:2, and
R represents a random sequence of 10 to 1000 nucleotides, preferably of 50 nucleotides.

**11.** Aptamer according to Claim 10, **characterized in that** R is preferably selected from the following sequences:

| | |
|---|---|
| **D4** | 5'GCGCGGGAAUAGUAUGGAAGGAUACGUAUACCGUGCAAUCCAGGCAACG 3' (SEQ ID NO:3) |
| **D12** | 5'GGGCUUCAUAAGCUACACCGGAACGCAGAAAUGCCUUAAGCCCGAGUU 3' (SEQ ID NO:4) |
| **D14** | 5'GGCCAUAGCGCACCACCAAGAGCAAAUCCCUAAGCGCGACUCGAGUGAGC 3' (SEQ ID NO:5) |
| **D20** | 5'GGGCCAAUCGAAGCCGGUAACUCCCAAACUAACGUGCAAACUGCACCCGC 3' (SEQ ID NO:6) |
| **D24** | 5'GCGGUAUGUAGGGAAUAGCACUUUUUUGCGUAUACCUACACCGCAGCG 3' (SEQ JD NO:7) |
| **D30** | 5'AGGCGAGCCCGACCACGUCAGUAUGCUAGACAACAACGCCCGCGUGGUAC 3' (SEQ ID NO:8) |
| **D32** | 5'CCCCGCUUUUGACGUGAUCGAACGCGUAUCAGUAACGUCAGCAGUCGAGC 3' (SEQ ID NO:9) |
| **D33** | 5'CAAAGCGUGUAUUCUCGUGAGCCGACCAUCGUUGCGAACAUCCCCGGAACG 3' (SEQ ID NO :10) |
| **D42** | 5'GACCCGUAUGAAGGUGGCGCAGGACACGACCGUCUGCAAUGAGCGAGC 3' (SEQ ID NO:11) |
| **D60** | 5'CCGACCUGUAACAGCAGUUAGUUACACGUUUGAAACAACCGGCGUUCGAGC 3' (SEQ ID NO:12) |
| **D76** | 5'GGCUUACACGGAGAAACAAGAGAGCGGCCCAAACUUGAUUGACAGUGGCC 3' (SEQ ID NO:13) |
| **D71** | 5'GGCCCUUAACGCAAAAACGAAGGAUCAUCGAUUGAUCGCCUUAUGGGCU 3' (SEQ ID NO:14) |
| **D87** | 5'CCGCGGUCUGUGGGACCCUUCAGGAUGAAGCGGCAACCCAUGCGGGCC 3' (SEQ ID NO:15) |

**12.** Aptamer according to Claim 10 or Claim 11, **characterized in that** the riboses of the purines bear a hydroxyl function on the carbon in the 2'-position, while the riboses of the pyrimidines bear a fluorine atom on the carbon in the 2'-position.

**13.** Aptamer according to any one of Claims 10 to 12, **characterized in that** it has one of the following sequences: SEQ ID NOs:31-33.

**14.** Aptamer according to any one of Claims 10 to 12, **characterized in that** it has formula II below:

5' $R_4X_6X_5X_4X_3$GGAAUAG$X_2X_1R_3X'_1X'_2$CGUAUAC$X'_3X'_4X'_5X'_6R_5$3' (II),

the secondary structure of which is represented in figure 10, and in which:

- the riboses of the purines bear an OH group in the 2'-position and the riboses of the pyrimidines bear a fluorine atom in the 2'-position,
- **$R_3$** is present or absent and represents an apical bulge (or loop) comprising:
    - a linear or branched carbon chain selected from the group consisting of $C_6$-$C_{30}$ alkyl groups or $C_6$-$C_{30}$ aryl groups;
    - a polymer such as PEG or PEI, or the like;
    - functional groups such as biotin, streptavidin, peroxidase, etc.;
    - other molecules of interest such as, for example, active ingredients, labeling tags, in particular fluorescent tags, or chelating agents for radioisotopes;
    - a natural or modified nucleotide sequence; preferably, $R_3$ represents the following bulges or loops (1) to (4):

      loop (1): 5' UGGAAGGA 3' (SEQ ID NO:29)
      loop (2): 5' CUUUUUU 3' (SEQ ID NO:30)
      loop (3): 5' GNPuA 3'
      loop (4): 5' UNCG 3',

    in which the riboses of the purines bear a hydroxyl function on the carbon in the 2'-position, while the riboses of the pyrimidines bear a fluorine atom on the carbon in the 2'-position,

- **$X_1$, $X'_1$, $X_2$, $X'_2$, $X_3$, $X'_3$, $X_4$, $X'_4$, $X_5$, $X'_5$, $X_6$ and $X'_6$** represent Py or Pu with, preferably:

  $X_1$-$X'_1$ corresponding to C-G, A-U, G-C or U-A
  $X_2$-$X'_2$ corresponding to C-G, A-U, G-C or U-A
  $X_3$-$X'_3$ corresponding to C-G, A-U, G-C or U-A
  $X_4$-$X'_4$ corresponding to C-G, A-U, G-C or U-A

$X_5$-$X'_5$ corresponding to C-G, A-U, G-C or U-A
$X_6$-$X'_6$ corresponding to C-G, A-U, G-C or U-A
N corresponding to G or C or A or U,
**Pu** corresponding to G or A, in which the riboses bear an OH group in the 2'-position,
**Py** corresponds to U or C, in which the riboses bear a fluorine atom in the 2'-position, and

- $\mathbf{R_4}$ and $\mathbf{R_5}$ are present or absent and represent:

. a natural or modified nucleotide sequence, comprising between 1 and several thousand nucleotides, preferably between 1 and 39 nucleotides; a part of said nucleotide sequence or said sequence preferably comprising one of the following sequences:

$R_4$ :

5'-$R_1$-$Z_1$-3', with $Z_1$=G:

5' GGGAGACAAGAAUAAACGCUCAAG 3' (SEQ ID NO:18) or
5'-$R_1$-$Z_1$-3', with $Z_1$=GCGGUAU (SEQ ID NO: 26) :

5' GGGAGACAAGAAUAAACGCUCAAGCGGUAU (SEQ ID NO:19), and

$R_5$ :

5'-$Z_2$-$R_2$-3', with $Z_2$=CAAUCCAGGGCAACG (SEQ ID NO:27):

5' CAAUCCAGGGCAACGAACGACAGGAGGCUCACAACAGGA 3' (SEQ ID NO:20) or
5'-$Z_2$-$R_2$-3', with $Z_2$=ACCGCAGCG (SEQ ID NO:28) 5' ACCGCAGCGAACGACAGGAGGCUCACAACAGGA 3' (SEQ ID NO:21),
5' GGGAGACAAGAAUAAACGCUCAAG 3' (SEQ ID NO:18) or
5' GGGAGACAAGAAUAAACGCUCAAGCGGUAU (SEQ ID NO:19), for $R_4$ and
5' CAAUCCAGGGCAACGAACGACAGGAGGCUCACAACAGGA 3' (SEQ ID NO: 20) or
5' ACCGCAGCGAACGACAGGAGGCUCACAACAGGA 3' (SEQ ID NO:21) for $R_5$;

. a linear or branched carbon chain selected from the group consisting of $C_6$-$C_{30}$ alkyl groups or $C_6$-$C_{30}$ aryl groups;
. a polymer such as PEG or PEI, or the like;
. functional groups such as biotin, streptavidin, peroxidase;
. other molecules of interest such as, for example, active ingredients, labeling tags, in particular fluorescent tags, or chelating agents for radioisotopes.

**15.** Aptamer according to Claim 14, **characterized in that** $R_3$ represents 5' UGGAAGGA 3' (loop (1)), $R_4$ represents SEQ ID NO:18 and $R_5$ represents SEQ ID NO:20, the aptamer exhibiting such a structure (family D4) has both properties of binding to said Ret receptor in its activated form and properties of inhibition of the activity of said receptor.

**16.** Aptamer according to Claim 15, **characterized in that** it has the sequence SEQ ID NO:22.

**17.** Aptamer according to Claim 14, **characterized in that** $R_3$ represents 5' CUUUUUU 3' (loop (2)), 5' GNPuA 3' (loop (3)) or 5' UNCG 3' (loop (4)), $R_4$ comprises from 1 to 30 nucleotides selected from SEQ ID NO:19 or from 1 to 24 nucleotides selected from SEQ ID NO:18 and $R_5$ comprises from 1 to 33 nucleotides of SEQ ID NO:21 or from 1 to 39 nucleotides selected from SEQ ID NO:20, the aptamer exhibiting such a structure having only properties of binding to said Ret receptor in its activated form, and in particular to the Ret receptor mutated in its extracellular domain.

**18.** Aptamer according to Claim 17, **characterized in that** $R_3$ represents 5' CUUUUUU 3' (loop (2)), $R_4$ represents SEQ ID NO:19 and $R_5$ represents SEQ ID NO: 21.

**19.** Aptamer according to Claim 17 or Claim 18, **characterized in that** it has SEQ ID NO:25.

**20.** Aptamer according to Claim 14, **characterized in that** $R_3$ represents 5' UGGAAGGA 3' (loop (1)), $R_4$ and $R_5$ are absent, the aptamer exhibiting such a structure having only properties of binding to said Ret receptor in its activated form, and **in that** it has the sequence SEQ ID NO:23.

**21.** Reagent for diagnosing a tumor, **characterized in that** it consists of an aptamer according to any one of Claims 10 to 20.

**22.** Reagent according to Claim 21, **characterized in that** it corresponds to an aptamer of formula II:

5' $R_4X_6X_5X_4X_3$GGAAUAG$X_2X_1R_3X'_1X'_2$CGUAUAC$X'_3X'_4X'_5X'_6R_5$3', in which $R_3$, $R_4$ and $R_5$ are absent.

**23.** Reagent according to Claim 22, **characterized in that** it corresponds to an aptamer of sequence: 5' GUAGGGAAUAGCACGUAUACCUAC 3' (SEQ ID NO:24).

**24.** Reagent according to Claim 21, **characterized in that** it corresponds to an aptamer of formula II, in which $R_3$ represents 5' CUUUUUU 3' and **in that** it corresponds to the sequence SEQ ID NO:25.

**25.** Reagent for diagnosing or detecting the Ret receptor in an activated or nonactivated form, **characterized in that** it consists of at least one aptamer according to any one of Claims 10 to 20.

**26.** Medicament, **characterized in that** it comprises an aptamer according to any one of claims 10 to 20, which has both an ability to bind to an RPTK receptor and an inhibitory action with respect to said receptor in an activated form.

**27.** Medicament for use in the treatment of a tumor, **characterized in that** it comprises an aptamer according to any one of Claims 10 to 20, which has both an ability to bind to an activated RPTK receptor, and in particular to the receptor mutated in the extracellular domain, and in particular to the Ret receptor mutated at one of the cysteines located in the extracellular domain (codons 609, 611, 618, 620 and 634), and an inhibitory action with respect to this mutated receptor.

**28.** Medicament according to Claim 26 or Claim 27, **characterized in that** it corresponds to an aptamer of the aptamer family D4, as defined in Claim 11, 14 or 15.

**29.** Pharmaceutical composition, **characterized in that** it comprises an aptamer according to any one of Claims 10 to 20, which has both an ability to bind to an RPTK receptor and an inhibitory action with respect to said receptor in its activated form.

**30.** Pharmaceutical composition, **characterized in that** it comprises:

- an aptamer according to any one of Claims 10 to 20, which has both an ability to bind to an activated RPTK receptor, and in particular to a receptor mutated in the extracellular domain, and in particular to the Ret receptor mutated at one of the cysteines located in the extracellular domain (codons 609, 611, 618, 620 and 634), and an inhibitory action with respect to this mutated receptor,
- another anticancer molecule, and
- at least one pharmaceutically acceptable vehicle.

**31.** Use of an aptamer which has both an ability to bind to an RPTK receptor and an inhibitory action with respect to this RPTK receptor, for screening products which interact with the RPTK receptor and which may or may not inhibit it.

**32.** Use of an aptamer which has both an ability to bind to an activated RPTK receptor, and in particular to the Ret receptor mutated at one of the cysteines located in the extracellular domain (codons 609, 611, 618, 620 and 634), and an inhibitory action with respect to this activated RPTK receptor, for screening products which interact with said RPTK receptor.

**33.** Method for screening products which interact with an RPTK receptor or targets which form a complex with said RPTK in an activated or nonactivated form, which method is **characterized in that** it comprises:

- bringing cells expressing RPTKs in an activated or nonactivated form into contact with the substance to be tested,

- adding, under suitable conditions, an aptamer according to any one of Claims 10 to 20, before, at the same time as or after the substance to be tested,
- evaluating the competitive binding between the aptamer and the molecule to be tested (for example: by measuring radioactivity, fluorescence, luminescence, surface plasmon resonance, BRET, FRET, or any other technique for demonstrating a molecular interaction).

**34.** Method according to Claim 33, **characterized in that**, after identification of the substances which bind competitively with the aptamer to the cells exhibiting RPTKs, the effect of these substances on the biological activity of said cells can be evaluated in order to find substances which inhibit or activate said biological activities of the cells exhibiting RPTKs.

**Patentansprüche**

**1.** Verfahren zur Identifizierung von Liganden oder Aptameren, die für einen Membranrezeptor mit Tyrosinkinase-Aktivität (RPTK für receptor protein-tyrosine kinase = Rezeptor-Proteinkinase), der in aktivierter oder nicht-aktivierter Form durch Zellen exprimiert wird, spezifisch sind, aus einem Gemisch von Nukleinsäuren, wobei das Verfahren wenigstens die folgenden Stufen umfasst:

(a) In-Kontakt-Bringen eines Gemisches von Nukleinsäuren mit Zellen, die den Membranrezeptor mit Tyrosinkinase-Aktivität nicht exprimieren oder ihn in nicht-aktivierter Form exprimieren, (Zellen $C_N$), wobei die Zellen denselben Zelltyp aufweisen wie Zellen, die denselben Membranrezeptor mit Tyrosinkinase-Aktivität exprimieren, aber in aktivierter Form, und zwar aufgrund des Vorliegens einer Mutation in der extrazellulären Domäne (Zellen $C_{Te}$);
(b) Wiedergewinnen einer ersten Untergruppe S1 von Nukleinsäuren, die während der Stufe (a) nicht an die Zellen $C_N$ binden;
(c) In-Kontakt-Bringen der ersten Untergruppe S1 mit Zellen $C_i$, die denselben Zelltyp wie die Zellen $C_{Te}$ darstellen, allerdings den Membranrezeptor mit Tyrosinkinase-Aktivität, der in seinem intrazellulären Teil mutiert ist, exprimieren, wobei die Zellen $C_i$ den Phänotyp desselben Typs wie der der Zellen $C_{Te}$ zeigen;
(d) Wiedergewinnen einer zweiten Untergruppe S2 von Nukleinsäuren, die während der Stufe (c) nicht an die Zellen $C_i$ binden;
(e) In-Kontakt-Bringen der zweiten Untergruppe S2 mit den Zellen $C_{Te}$:
(f) Wiedergewinnen der Nukleinsäuren, die an die Zellen $C_{Te}$ binden, das heißt der, die eine erhöhte Affinität gegenüber den Zellen aufweisen, die den Membranrezeptor mit Tyrosinkinase-Aktivität exprimieren, der in seinem extrazellulären Teil mutiert ist, nach Dissoziierung der Komplexe Zellen-Nukleinsäuren;
(g) Amplifikation der Nukleinsäuren mit erhöhter Affinität für die Zellen, die den Membranrezeptor mit Tyrosinkinase-Aktivität exprimieren, der in der extrazellulären Domäne mutiert ist, derart, dass ein Gemisch von Nukleinsäuren erhalten wird, das an Nukleinsäuren angereichert ist, die eine erhöhte Affinität für die Zellen $C_{Te}$ haben, und
(h) Identifizierung der spezifischen Liganden oder Aptameren der Zellen, die Membranrezeptoren mit Tyrosinkinase-Aktivität (RPTK für receptor protein-tyrosine kinase, Rezeptor-Proteinkinase) in aktivierter Form exprimieren, aus einem in (g) erhaltenen Gemisch.

**2.** Verfahren gemäß Anspruch 1, **dadurch gekennzeichnet, dass** man die Stufen (a)-(g) unter Verwendung der Gemische, die an Liganden oder Aptameren des vorangehenden Zyklus angereichert sind, bis zum Erhalt wenigstens eines Aptamers, dessen Affinität, die durch seine Dissoziationskonstante (Kd) definiert wird, messbar ist und das für eine pharmazeutische Verwendung geeignet ist, wiederholt.

**3.** Verfahren gemäß Anspruch 1 oder Anspruch 2, **dadurch gekennzeichnet, dass** die kombinatorische Bank von Ausgangs-Nukleinsäuren wenigstens $10^2$ Nukleinsäuren, vorzugsweise zwischen $10^9$ und $10^{15}$ Nukleinsäuren enthält und vorteilhafterweise aus Nukleinsäuren besteht, die zufällige Sequenzen umfassen, die an ihren 5'- und 3'-Enden feststehende Sequenzen, die eine Amplifikation durch PCR zulassen, vorzugsweise die Sequenzen SEQ ID NO:1 und SEQ ID NO:2 oder ein Fragment von wenigstens 8 Nucleotiden dieser Sequenzen, umfassen.

**4.** Verfahren gemäß einem der Ansprüche 1 bis 3, **dadurch gekennzeichnet, dass** die zufälligen Sequenzen jeweils zwischen 10 und 1000 Nucleotide, vorzugsweise 50 Nucleotide, enthalten und vorzugsweise DNA, RNA oder modifizierte Nukleinsäuren sind.

**5.** Verfahren gemäß einem der Ansprüche 1 bis 4, **dadurch gekennzeichnet, dass** die Identifizierung der spezifischen Liganden oder Aptamere der Zellen $C_{Te}$ gemäß Stufe (h) eine Evaluierung der biologischen Aktivität der Aptamere auf den Zellen $C_{Te}$ umfasst.

**6.** Verfahren gemäß Anspruch 5, **dadurch gekennzeichnet, dass** die biologischen Aktivitäten, die vorteilhafterweise evaluiert werden, die folgenden sind:

(a) Inhibierung oder Aktivierung der Auto-Phosphorylierung der RPTK,
(b) Inhibierung oder Aktivierung der Aktivierungskaskade von Kinasen,
(c) Inhibierung der Phosphorylierung der normalen RPTK von Zellen $C_N$, die durch eine zweckmäßige Stimulierung aktiviert wurden, und
(d) Reversion des Phänotyps, der mit einer Aktivierung der RPTK assoziiert ist.

**7.** Verfahren gemäß einem der Ansprüche 1 bis 6, **dadurch gekennzeichnet, dass** die RPTK aus der Gruppe ausgewählt wird, die durch die folgenden Membranrezeptoren gebildet wird: EGFR (Epithelial Growth Factor Receptor, epithelialer Wachstumsfaktor-Rezeptor), InsulinR (Insulin Receptor, Insulinrezeptor), PDGFR (Platelet-derived Growth Factor Receptor, Blutplättchen-Wachstumsfaktor-Rezeptor), VEGFR (Vascular Endothelial Growth Factor Receptor, vaskulärer epithelialer Wachstumsfaktor-Rezeptor), FGFR (Fibroblast Growth Factor Receptor, Fibroblasten-Wachstumsfaktor-Rezeptor), NGFR (Nerve Growth Factor Receptor, Nerven-Wachstumsfaktor-Rezeptor), HGFR (Hepatocyte Growth Factor Receptor, Hepatozyten-Wachstumsfaktor-Rezeptor), EPHR (Ephrin Receptor, Ephrin-Rezeptor), AXL (Tyro 3 PTK); TIE (Tyrosine Kinase Receptor in endothelial cells, Tyrosinkinase-Rezeptor in Endothelialzellen), TET (Rearranged During Transfection, neu angeordnet während Transfektion), ROS (RPTK, exprimiert in bestimmten Epithelzellen) und LTK (Leukocyte Tyrosine Kinase, Leukozyten-Tyrosinkinase).

**8.** Verfahren gemäß einem der Ansprüche 1 bis 7, **dadurch gekennzeichnet, dass** das Aptamer oder der Ligand einen Rezeptor Ret in aktivierter Form und insbesondere den Rezeptor Ret, der durch Mutation auf dem Niveau eines Cysteins, das in der extrazellulären Domäne lokalisiert ist, insbesondere auf dem Niveau der Codons 609, 611, 618, 620 oder 634, aktiviert ist, erkennt, wobei das Verfahren umfasst:

(a) In-Kontakt-Bringen eines Gemisches von Nukleinsäuren mit Zellen $C_N$, die den Rezeptor Ret nicht in aktivierter Form exprimieren,
(b) Wiedergewinnen einer ersten Untergruppe S1 von Nukleinsäuren, die während der Stufe (a) nicht an die Zellen $C_N$ binden,
(c) In-Kontakt-Bringen der ersten Untergruppe S1 mit Zellen $C_i$, die einen Rezeptor Ret exprimieren, der in seiner intrazellulären Domäne mutiert ist, insbesondere den mutierten Rezeptor $Ret^{M918T}$,
(d) Wiedergewinnen einer zweiten Untergruppe S2 von Nukleinsäuren, die nicht an die Zellen $C_i$ binden,
(e) In-Kontakt-Bringen der zweiten Untergruppe S2 mit den Zellen $C_{Te}$, die einen Rezeptor Ret exprimieren, der durch Mutation in der extrazellulären Domäne aktiviert wurde, wobei der Rezeptor aus der Gruppe ausgewählt wird, die aus den mutierten Ret-Rezeptoren besteht, die eine Mutation an einem der Cysteine tragen, die in der extrazellulären Domäne lokalisiert sind, vorzugsweise auf dem Niveau von Cys609, Cys611, Cys618, Cys620 oder Cys634, vorzugsweise den Rezeptor $Re^{C634Y}$,
(f) Wiedergewinnen der Nukleinsäuren, die an die Zellen $C_{Te}$ gebunden sind, das heißt, gleichzeitig eine erhöhte Affinität und eine Bindungsspezifität für die Zellen aufweisen, die einen mutierten Ret-Rezeptor exprimieren, wie er in Stufe (e) definiert ist,
(g) Amplifikation der in Stufe (f) erhaltenen Nukleinsäuren derart, dass ein Gemisch von Nukleinsäuren erhalten wird, das an Nukleinsäuren angereichert ist, die eine erhöhte Affinität für die Zellen $C_{Te}$ haben,
(h) Wiederholung der Stufen (a)-(g) bis zum Erhalt wenigstens eines Aptamers, dessen Affinität für die Zellen $C_{Te}$, die durch seine Dissoziationskonstante (Kd) definiert ist, messbar ist und das für eine pharmakologische Aktivität geeignet ist,
und
(i) Identifizierung der spezifischen Aptamere der Zellen, die einen Ret-Rezeptor in seiner aktivierten Form exprimieren, der aus dem in (h) erhaltenen Gemisch ausgewählt wurde.

**9.** Verfahren gemäß Anspruch 8, **dadurch gekennzeichnet, dass**:

- die Zellen $C_N$ insbesondere Wildtyp-Zellen PC 12 (Bezeichnung ECACC Nr. 88022) oder Wildtyp-Zellen NIH 3T3 (Bezeichnung ECACC Nr. 93061524) sind,
- die Zellen $C_i$ und $C_{Te}$ erhalten werden, indem ein Onkogen, das Träger einer intrazellulären bzw. extrazellulären Mutation ist, in Zellen $C_N$ in Kultur derart eingeführt wird, dass sie das Onkogen exprimieren.

**10.** Aptamer, **dadurch gekennzeichnet, dass** es durch ein Identifizierungsverfahren, wie es in den Ansprüchen 1 bis 9 definiert ist, erhalten werden kann, dass es für Zellen spezifisch ist, die einen Rezeptor mit Tyrosinkinase-Aktivität in aktivierter Form exprimieren, und dass es aus der Gruppe ausgewählt ist, die durch die Aptamere der Formel (I) gebildet wird:

$$R_1 - R - R_2 \qquad (I),$$

in der:

$R_1$ 5' GGGAGACAAGAAUAAACGCUCAA 3' (SEQ ID NO:1) oder ein Fragment aus 1 bis 23 Nucleotiden der SEQ ID NO:1 darstellt;
$R_2$ 5' AACGACAGGAGGCUCACAACAGGA 3' (SE ID NO:2) oder ein Fragment aus 1 bis 24 Nucleotiden der SEQ ID NO: 1 darstellt und
R eine zufällige Sequenz mit 10 bis 1000 Nucleotiden, vorzugsweise 50 Nucleotiden, darstellt.

**11.** Aptamer gemäß Anspruch 10, **dadurch gekennzeichnet, dass** R vorzugsweise aus den folgenden Sequenzen ausgewählt ist:

| | |
|---|---|
| **D4** | 5'GCGCGGGAAUAGUAUGGAAGGAUACGUAUACCGUGCAAUCCAGGGCAACG 3' (SEQ ID NO:3) |
| **D12** | 5'GGGCUUCAUAAGCUAGACCGGCCAACGCAGAAAGCCUUAAGCCCGAGUU 3' (SEQ ID NO:4) |
| **D14** | 5'GCCCAUAGCCCACCACCAAGAGGAAAUCCCUAAGCGCGAGUCGAGUGAGC 3' (SEQ ID. NO:5) |
| **D20** | 5'GGGCCAAUCGAAGQCGGUAAUUCCCAAACUAACGUGCAAACUGCACCCGC3.'(SEQ ID NO:6) |
| **D24** | 5'GGGGUAUGUAGGGAAUAGCAGUUUUUUUGCGUAUACCUACACCGCAGCG 3' (SEQ ID NO:7) |
| **D30** | 5'AGGCGAGCGCGACCACGUCAGUAUGCUAGACAACAACGCGCGCGUGGUAC 3' (SEQ ID NO:8) |
| **D32** | 5'CCCCGCUUUUUUGACGUGAUCGAACGCGUAUCAACGCAGCAGCAGUCGAGC 3' (SEQ ID NO: 9) |
| **D33** | 5'CAAAGCGUGUAUUCUCGAGAGCCGACCAUCGUUGCGAACAUCCGCGGAAGG 3' (SEQ ID NO:10) |
| **D42** | 5'GACCCGUAUGAAGGUGGGGCAGGACACGACCGUCGCGGAAUGAGCGAGC3'(SEQ ID NO:11) |
| **D60** | 5'CCGACCUGUACAGCAGUUAGUUACACGUUUGAAACAACCGGGCUUCGUUCGAGC 3' (SEQ ID NO: 12) |
| **D76** | 5'GGCUUACACGGAGAAACAAGAGAGCGGCCCAAACUUGAUUGACACUGGCC 3'-(SEQ ID NO:13) |
| **D71** | 5'GGCCCUUAACGCAAAAACGAAGAAUCAUCGAUUGAUCGCCUUAUGGGCU 3' (SEQ ID NO:14) |
| **D87** | 5-'CCGCGGUCUGUGGGACCCUUCAGGAUGAAGCGGCAACCCAUGCGGGCC 3' (SEQ ID NO: 15) |

**12.** Aptamer gemäß Anspruch 10 oder Anspruch 11, **dadurch gekennzeichnet, dass** die Ribosen der Purine Träger einer Hydroxyl-Funktion an dem Kohlenstoff in Position 2' sind, während die Ribosen der Pyrimidine ein Fluoratom an dem Kohlenstoff in Position 2' tragen.

**13.** Aptamer gemäß einem der Ansprüche 10 bis 12, **dadurch gekennzeichnet, dass** es eine der folgenden Sequenzen aufweist: SEQ ID NO:31-33.

**14.** Aptamer gemäß einem der Ansprüche 10 bis 12, **dadurch gekennzeichnet, dass** es die folgende Formel (II) aufweist:

$$5'\ R_4X_6X_5X_4X_3GGAAUAGX_2X_1R_3X'_1X'_2CGUAUACX'_3X'_4X'_5X'_6R_5\ 3' \qquad (II)$$

deren Sekundärstruktur in Figur 10 dargestellt ist und in der:

- die Ribosen der Purine Träger einer HO-Gruppe in 2' sind und die Ribosen der Pyrimidine Träger eines Fluoratoms in 2' sind,
- $R_3$ vorhanden ist oder nicht vorhanden ist und eine apikale Schleife (oder Loop) darstellt, die umfasst:

  • eine lineare oder verzweigte Kohlenstoffkette, die aus der Gruppe, bestehend aus $C_6$-$C_{30}$-Alkyl-Gruppen

oder $C_6$-$C_{30}$-Aryl-Gruppen, ausgewählt ist;
• ein Polymer wie PEG oder PEI oder anderes;
• funktionelle Gruppen wie Biotin, Streptavidin, Peroxidase,
• andere Moleküle von Interesse wie beispielsweise Wirkstoffe, Markierungen, insbesondere fluoreszierende, oder Chelatbildner für Radioisotope,
• eine natürliche oder modifizierte Nucleotidsequenz; wobei $R_3$ vorzugsweise die folgenden Schleifen oder Loops (1) bis (4) darstellt:

| | |
|---|---|
| Schleife (1) : | 5' UGGAAGGA 3' (SEQ ID NO:29) |
| Schleife (2) : | 5'CUUUUUU 3' (SEQ ID NO:30) |
| Schleife (3) : | 5' GNPuA 3' |
| Schleife (4) : | 5' UNCG 3' |

in denen die Ribosen der Purine Träger einer Hydroxyl-Funktion an dem Kohlenstoff in Position 2' sind, während die Ribosen der Pyrimidine ein Fluoratom an dem Kohlenstoff in Position 2' tragen,
- $X_1$, $X'_1$, $X_2$, $X'_2$, $X_3$, $X'_3$, $X_4$, $X'_4$, $X_5$, $X'_5$, $X_6$ und $X'_6$ Py oder Pu darstellen mit der Präferenz, dass:

$X_1$-$X'_1$ C-G, A-U, G-C oder U-A entspricht,
$X_2$-$X'_2$ C-G, A-U, G-C oder U-A entspricht,
$X_3$-$X'_3$ C-G, A-U, G-C oder U-A entspricht,
$X_4$-$X'_4$ C-G, A-U, G-C oder U-A entspricht,
$X_5$-$X'_5$ C-G, A-U, G-C oder U-A entspricht,
$X_6$-$X'_6$ CG, A-U, G-C oder U-A entspricht,
N G oder C oder A oder U entspricht,
Pu G oder A entspricht, in denen die Ribosen Träger einer HO-Gruppierung in 2' sind,
Py U oder C entspricht, in denen die Ribosen Träger eines Fluoratoms in 2' sind, und

- $R_4$ und $R_5$ vorhanden oder nicht vorhanden sind und darstellen:

• eine natürliche oder modifizierte Nucleotidsequenz, die zwischen 1 oder mehreren Tausend Nucleotiden, vorzugsweise zwischen 1 und 39 Nucleotiden, umfasst; wobei ein Teil der Nucleotid-Sequenz oder die Sequenz vorzugsweise eine der folgenden Sequenzen umfasst:

$R_4$:

5'-$R_1$-$Z_1$-3', wobei $Z_1$ = G: 5'GGGAGACAAGAAUAAACGCUCAAG 3' (SEQ ID NO: 18) oder
5'-$R_1$-$Z_1$-3', wobei $Z_1$ = GCGGUAU (SEQ ID NO:26):

5' GGGAGACAAGAAUAAACGCUCAAGCGGUAU (SEQ ID NO:19) und

$R_5$:

5'-$Z_2$-$R_2$ 3', wobei $Z_1$ = CAAUCCAGGGCAACG (SEQ ID NO:27):

5' CAAUCCAGGGCAACGAACGACAGGAGGCUCACAACAGGA 3' (SEQ ID NO:20) oder
5'-$Z_2$-$R_2$-3', wobei $Z_2$ = ACCGCAGCG (SEQ ID NO.28):

5' ACCGCAGCGAACGACAGGAGGCUCACAACAGGA 3' (SEQ ID NO:21);
5' GGGAGACAAGAAUAAACGCUCAAG 3' (SEQ ID NO:18) oder
5' GGGAGACAAGAAUAAACGCUCAAGCGGUAU (SEQ ID NO:19) für $R_4$ und
5' CAAUCCAGGGCAACGAACGACAGGAGGCUCACAACAGGA 3' (SEQ ID NO:20) oder
5' ACCGCAGCGAACGACAGGAGGCUCACAACAGGA 3' (SEQ ID NO:21) für $R_5$;

• eine lineare oder verzweigte Kohlenstoffkette, ausgewählt aus der Gruppe, bestehend aus $C_6$-$C_{30}$-Alkyl-Gruppen und $C_6$-$C_{30}$-Aryl-Gruppen;

- ein Polymer wie PEG oder PEI oder ein anderes;
- funktionelle Gruppierungen wie Biotin, Streptavidin, Peroxidase,
- andere Moleküle von Interesse wie zum Beispiel Wirkstoffe, Markierungssubstanzen, insbesondere fluoreszierende, oder Chelatbildner für Radioisotope.

**15.** Aptamer gemäß Anspruch 14, **dadurch gekennzeichnet, dass** $R_3$ 5' UGGAAGGA 3' (Schleife (1)) darstellt, $R_4$ SEQ ID NO:18 darstellt und $R_5$ SEQ ID NO:20 darstellt, wobei das Aptamer eine Struktur (Familie D4) aufweist, die gleichzeitig Bindungseigenschaften für den Rezeptor Ret in seiner aktivierten Form und Inhibierungseigenschaften der Aktivität des Rezeptors besitzt.

**16.** Aptamer gemäß Anspruch 15, **dadurch gekennzeichnet, dass** es die Sequenz SEQ ID NO:22 aufweist.

**17.** Aptamer gemäß Anspruch 14, **dadurch gekennzeichnet, dass** $R_3$ 5' CUUUUUU 3' (Schleife (2)), 5' GNPuA 3' (Schleife (3)) oder 5' UNCG 3' (Schleife (4)) darstellt, $R_4$ 1 bis 30 Nucleotide, ausgewählt aus SEQ ID NO:19, oder 1 bis 24 Nucleotide, ausgewählt aus SEQ ID NO:18, umfasst und $R_5$ 1 bis 33 Nucleotide der SEQ ID NO:21 oder 1 bis 39 Nucleotide, ausgewählt aus SEQ ID NO:20, umfasst, wobei das Aptamer eine Struktur aufweist, die nur Bindungseigenschaften an den Rezeptor Ret in seiner aktivierten Form und insbesondere den Rezeptor Ret, der in seiner extrazellulären Domäne mutiert ist, besitzt.

**18.** Aptamer gemäß Anspruch 17, **dadurch gekennzeichnet, dass** $R_3$ 5' CUUUUUU 3' (Schleife (2)) darstellt, $R_4$ SEQ ID NO:19 darstellt und $R_5$ SEQ ID NO:21 darstellt.

**19.** Aptamer gemäß Anspruch 17 oder Anspruch 18, **dadurch gekennzeichnet, dass** es SEQ ID NO:25 aufweist.

**20.** Aptamer gemäß Anspruch 14, **dadurch gekennzeichnet, dass** $R_3$ 5' UGGAAGGA 3' (Schleife (1)) darstellt, $R_4$ und $R_{5'}$ nicht vorhanden sind, wobei das Aptamer eine Struktur aufweist, die nur Bindungseigenschaften an den Rezeptor Ret in seiner aktivierten Form besitzt und dass es die Sequenz SEQ ID NO:23 aufweist.

**21.** Diagnose-Reagens für einen Tumor, **dadurch gekennzeichnet, dass** es aus einem Aptamer gemäß einem der Ansprüche 10 bis 20 besteht.

**22.** Reagens gemäß Anspruch 21, **dadurch gekennzeichnet, dass** es einem Aptamer der Formel (II) entspricht:

$$5'\ R_4X_6X_5X_4X_3GGAAUAGX_2X_1R_3X'_1X'_2CGUAUACX'_3X'_4X'_5X'_6R_5\ 3'$$

in der $R_3$, $R_4$ und $R_5$ nicht vorhanden sind.

**23.** Reagens gemäß Anspruch 22, **dadurch gekennzeichnet, dass** es einem Aptamer der Sequenz:

5' GUAGGGAAUAGCACGUAUACCUAC 3' (SEQ ID NO:24)

entspricht.

**24.** Reagens gemäß Anspruch 21, **dadurch gekennzeichnet, dass** es einem Aptamer der Formel II entspricht, in der $R_3$ 5' CUUUUUU 3' darstellt, und dadurch, dass es der Sequenz SEQ ID NO:25 entspricht.

**25.** Diagnose- oder Detektions-Reagens für den Rezeptor Ret in aktivierter oder nicht aktivierter Form, **dadurch gekennzeichnet, dass** es aus wenigstens einem Aptamer gemäß einem der Ansprüche 10 bis 20 besteht.

**26.** Medikament, **dadurch gekennzeichnet, dass** es ein Aptamer gemäß einem der Ansprüche 10 bis 20 umfasst, das einerseits Bindungsvermögen an einen Rezeptor RPTK und andererseits inhibitorische Wirkung gegenüber dem Rezeptor in aktivierter Form aufweist.

**27.** Medikament, das zur Behandlung eines Tumors bestimmt ist, **dadurch gekennzeichnet, dass** es ein Aptamer gemäß einem der Ansprüche 10 bis 20 umfasst, das einerseits Bindungsaktivität für einen aktivierten Rezeptor RPTK und insbesondere einen in der extrazellulären Domäne mutierten Rezeptor und insbesondere den Rezeptor Ret, der auf dem Niveau eines der Cysteine mutiert ist, die in der extrazellulären Domäne lokalisiert sind (Codons 609, 611, 618, 620 und 634), und andererseits eine inhibitorische Wirkung gegenüber diesem mutierten Rezeptor aufweist.

**28.** Medikament gemäß Anspruch 26 oder Anspruch 27, **dadurch gekennzeichnet, dass** es einem Aptamer der D4-Familie der Aptamere entspricht, wie sie in den Ansprüchen 11, 14 oder 15 definiert sind.

**29.** Pharmazeutische Zusammensetzung, **dadurch gekennzeichnet, dass** sie ein Aptamer gemäß einem der Ansprüche 10 bis 20 umfasst, der einerseits eine Bindungsaffinität für einen RPTK-Rezeptor und andererseits inhibitorische Wirkung gegenüber dem Rezeptor in seiner aktivierten Form aufweist.

**30.** Pharmazeutische Zusammensetzung, **dadurch gekennzeichnet, dass** sie umfasst:

- ein Aptamer gemäß einem der Ansprüche 10 bis 20, das einerseits Bindungsaffinität für einen aktivierten RPTK-Rezeptor und insbesondere einen Rezeptor, der in der extrazellulären Domäne mutiert ist, und insbesondere den Rezeptor Ret, der auf dem Niveau eines der Cysteine, die in der extrazellulären Domäne lokalisiert sind (Codons 609, 611, 618, 620 und 634) mutiert ist, und eine inhibitorische Wirkung gegenüber diesem mutierten Rezeptor aufweist,
- ein anderes Antikrebsmolekül und
- wenigstens ein pharmazeutisch verträgliches Vehikel.

**31.** Verwendung eines Aptamers, das gleichzeitig Bindungsaktivität für einen RPTK-Rezeptor und inhibitorische Wirkung gegenüber diesem RPTK-Rezeptor zeigt, für das Screening von Produkten, die mit dem RPTK-Rezeptor wechselwirken und ihn inhibieren können oder nicht.

**32.** Verwendung eines Aptamers, das gleichzeitig Bindungsvermögen für einen aktivierten RPTK-Rezeptor und insbesondere den Rezeptor Ret, der auf dem Niveau eines der Cysteine, die in der extrazellulären Domäne lokalisiert sind (Codons 609, 611, 618, 620 und 634) mutiert ist, und inhibitorische Wirkung gegenüber diesem aktivierten RPTK-Rezeptor aufweist, für das Screening von Produkten, die mit dem RPTK-Rezeptor wechselwirken.

**33.** Verfahren zum Screening von Produkten, die mit einem RPTK-Rezeptor wechselwirken, oder Targets, die einen Komplex mit RPTK in aktivierter oder nicht aktivierter Form bilden, wobei das Verfahren **dadurch gekennzeichnet ist, dass** es umfasst:

- In-Kontakt-Bringen der Zellen, die RPTK in aktivierter oder nicht aktivierter Form exprimieren, mit der zu testenden Substanz,
- Zusetzen eines Aptamers gemäß einem der Ansprüche 10 bis 20 unter zweckmäßigen Bedingungen vor, gleichzeitig mit oder nach der zu testenden Substanz,
- Evaluieren der kompetitiven Bindung zwischen dem Aptamer und dem zu testenden Molekül (zum Beispiel durch Messung von Radioaktivität, Fluoreszenz, Lumineszenz, Oberflächen-Plasmonresistenz, BRET, FRET oder jede andere Technik zum Beweis einer molekularen Wechselwirkung).

**34.** Verfahren gemäß Anspruch 33, **dadurch gekennzeichnet, dass** nach Identifizierung der Substanzen, die in kompetitiver Weise mit dem Aptamer an Zellen, die RPTK aufweisen, binden, die Wirkung dieser Substanzen auf die biologische Aktivität der Zellen evaluiert werden kann, um Substanzen zu finden, die die biologischen Aktivitäten der Zellen, die RPTK aufweisen, inhibieren oder aktivieren.

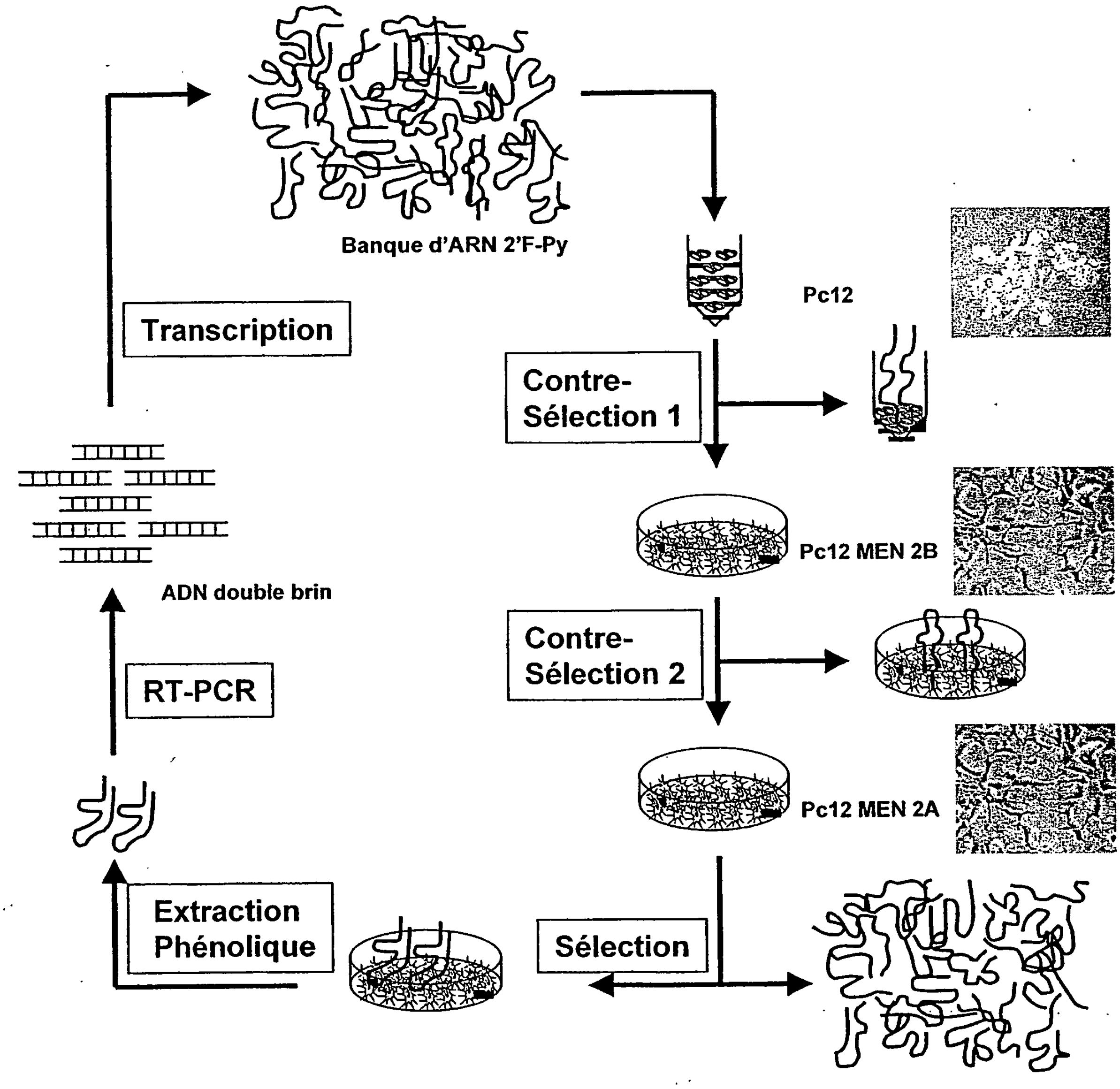
Banque d'ARN 2'F-Py
Transcription
Pc12
Contre-
Sélection 1
Pc12 MEN 2B
ADN double brin
Contre-
Sélection 2
RT-PCR
Pc12 MEN 2A
Extraction
Phénolique
Sélection

FIGURE 1

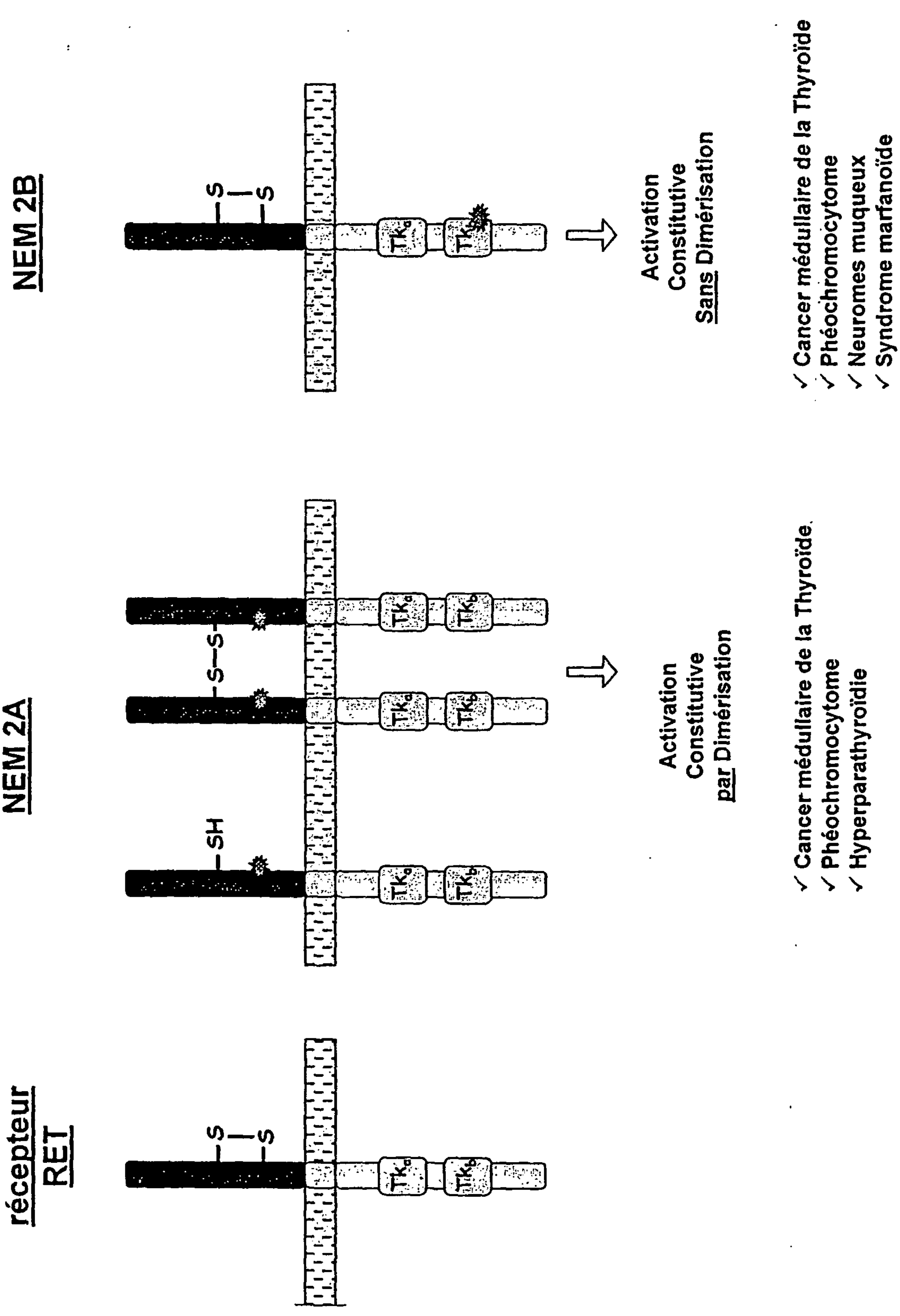
récepteur RET
S—S
TKa
TKb
NEM 2A
SH
S—S
Activation
Constitutive
par Dimérisation
✓ Cancer médullaire de la Thyroïde
✓ Phéochromocytome
✓ Hyperparathyroïdie
NEM 2B
Activation
Constitutive
Sans Dimérisation
✓ Cancer médullaire de la Thyroïde
✓ Phéochromocytome
✓ Neuromes muqueux
✓ Syndrome marfanoïde

FIGURE 2

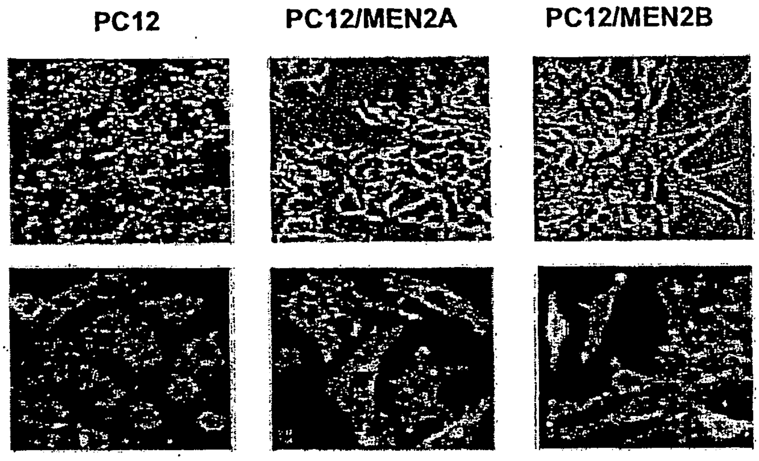


**Cellules PC12 transfectées de manière stable avec un vecteur d'expression des récepteurs mutés humains Ret$^{C634Y}$ (PC12/MEN2A) ou Ret$^{M918T}$ (PC12/MEN2B).**

FIGURE 3

**Représentation schématique de l'activation du récepteur RET dépendant du GDNF**

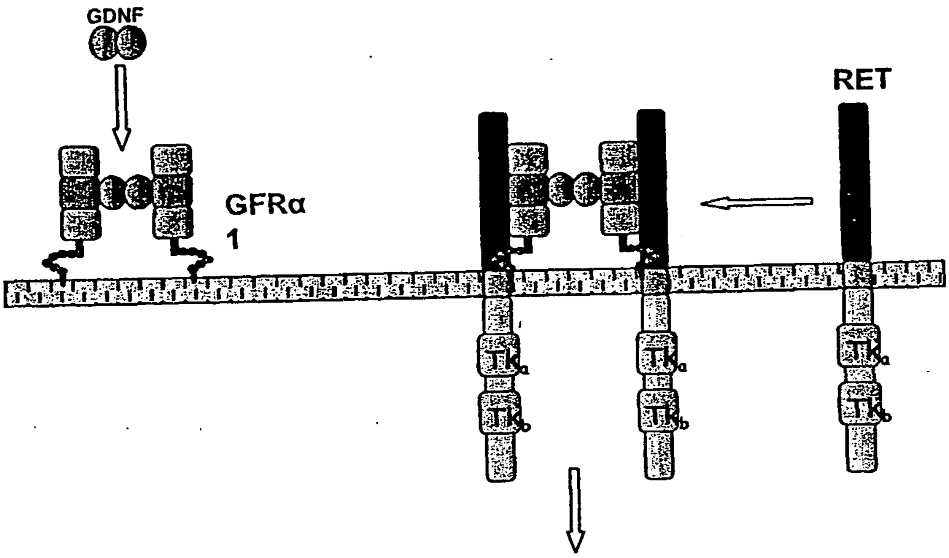


**Activation de la tyrosine kinase**

FIGURE 4

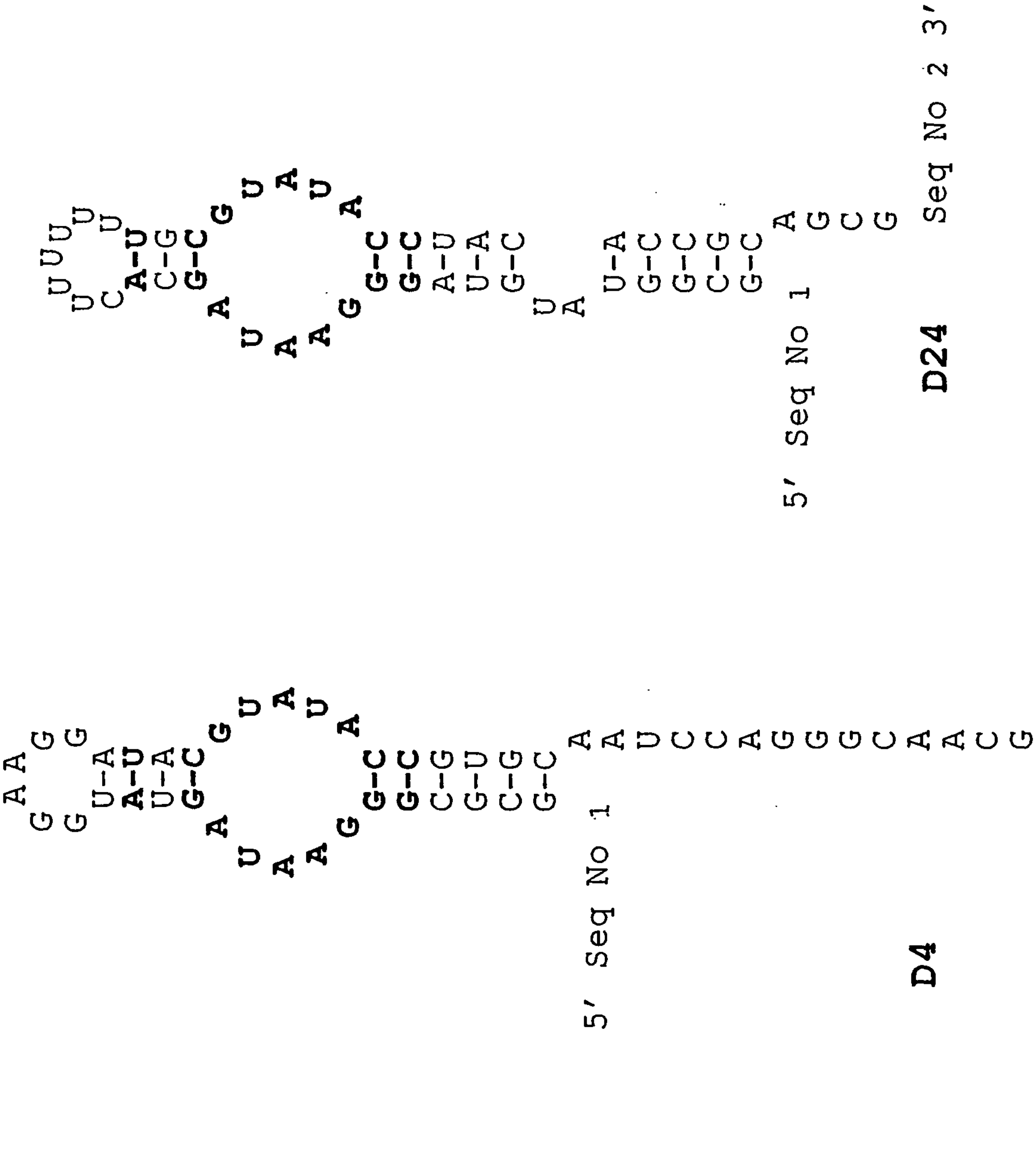


*Fig. 5 A*

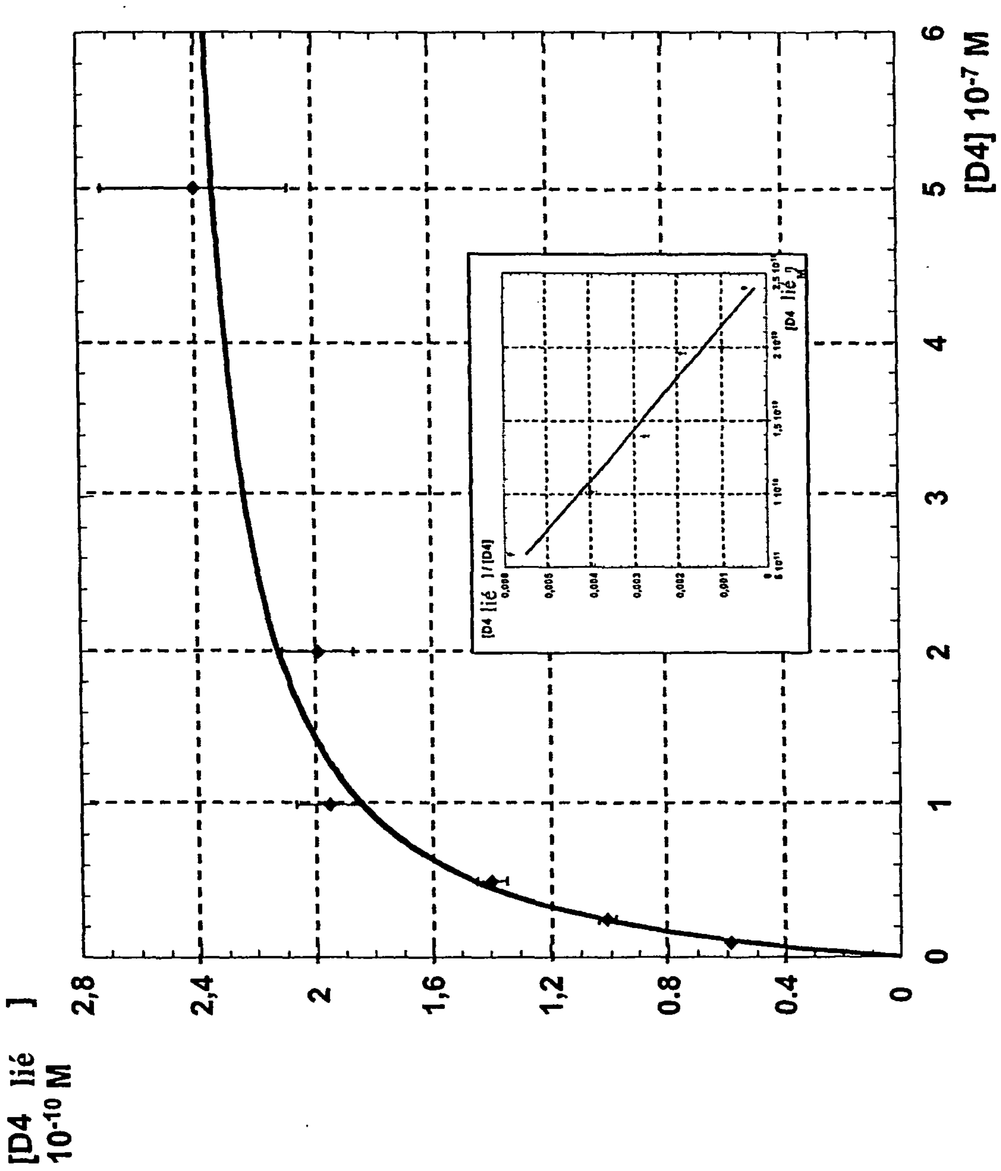


FIGURE 5B

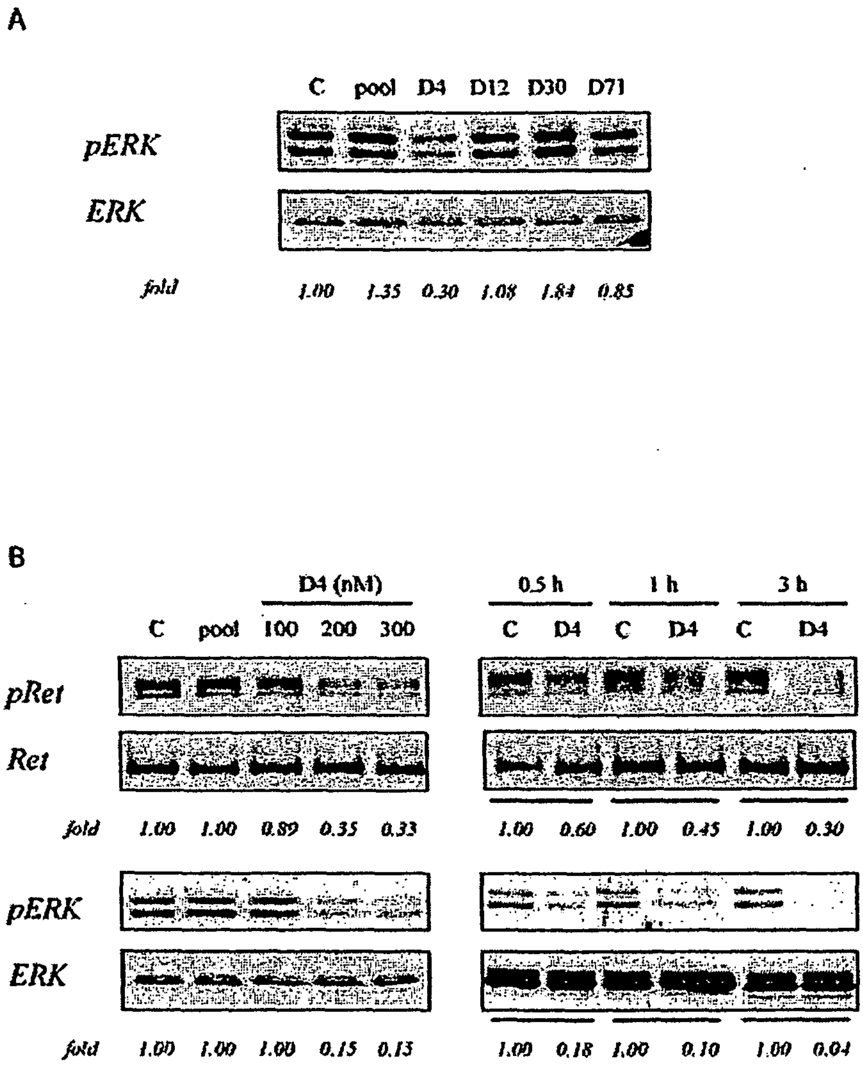
A
C pool D4 D12 D30 D71
pERK
ERK
fold 1.00 1.35 0.30 1.08 1.84 0.85
B
D4 (nM)
C pool 100 200 300
0.5 h 1 h 3 h
C D4 C D4 C D4
pRet
Ret
fold 1.00 1.00 0.89 0.35 0.33
1.00 0.60 1.00 0.45 1.00 0.30
pERK
ERK
fold 1.00 1.00 1.00 0.15 0.13
1.00 0.18 1.00 0.10 1.00 0.04

FIGURE 6

A

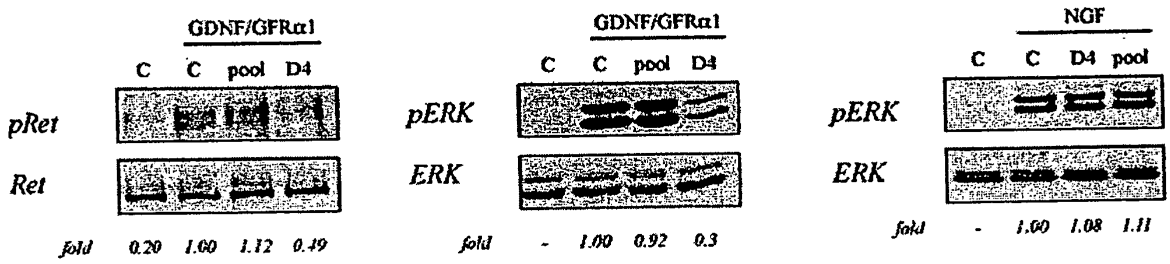
GDNF/GFRα1
C C pool D4
pRet
Ret
fold 0.20 1.00 1.12 0.49
GDNF/GFRα1
C C pool D4
pERK
ERK
fold - 1.00 0.92 0.3
NGF
C C D4 pool
pERK
ERK
fold - 1.00 1.08 1.11

B

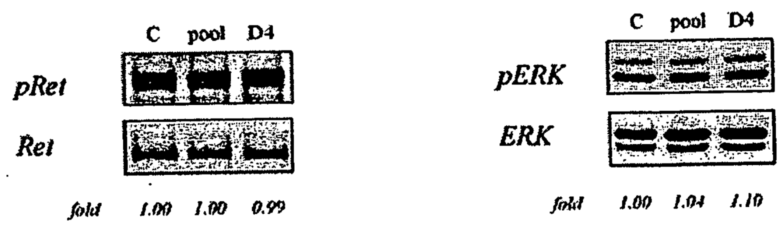
C pool D4
pRet
Ret
fold 1.00 1.00 0.99
C pool D4
pERK
ERK
fold 1.00 1.04 1.10

FIGURE 7

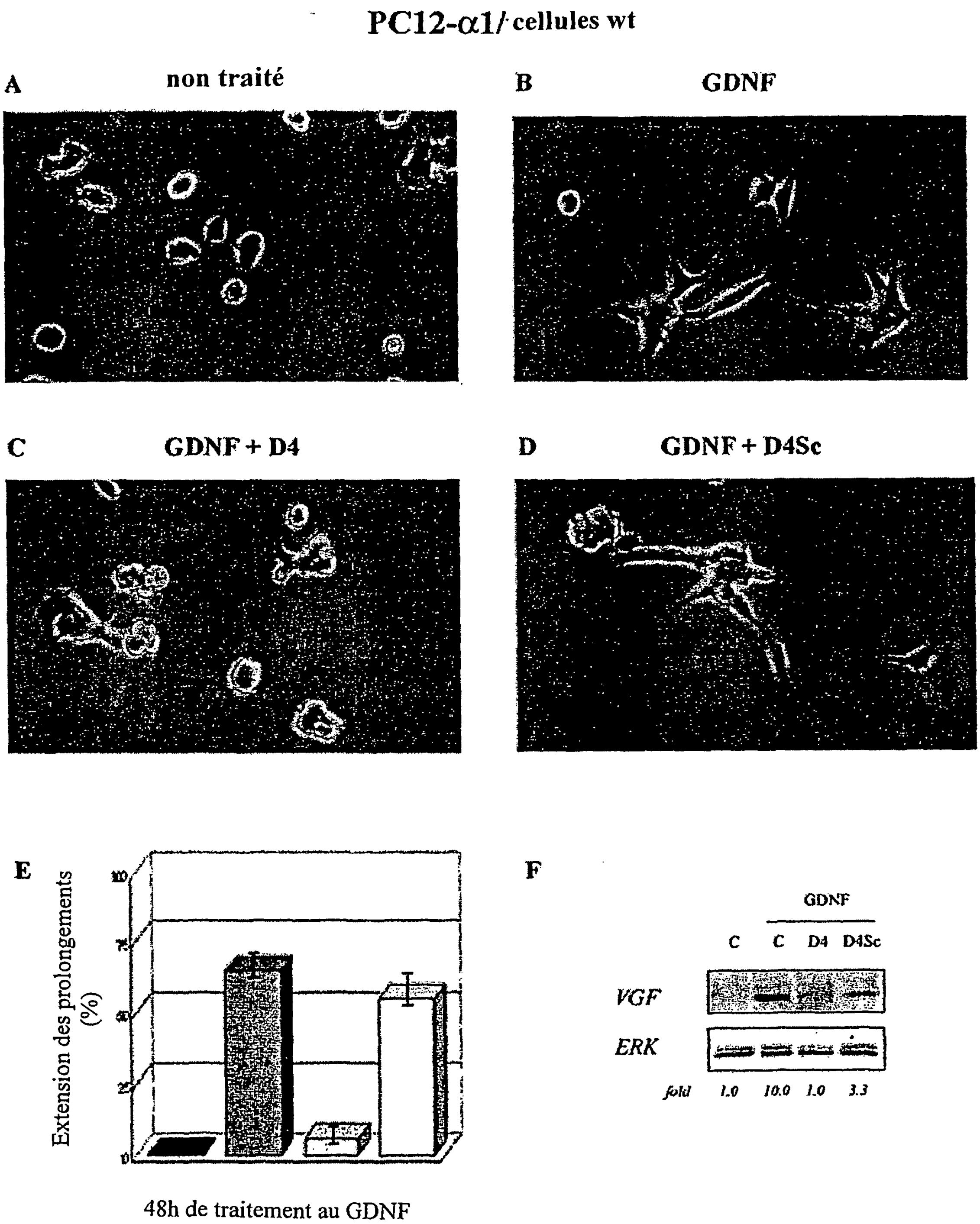
PC12-α1/ cellules wt
A
non traité
B
GDNF
C
GDNF + D4
D
GDNF + D4Sc
E
Extension des prolongements (%)
48h de traitement au GDNF
F
GDNF
C
C
D4
D4Sc
VGF
ERK
fold
1.0
10.0
1.0
3.3

FIGURE 8

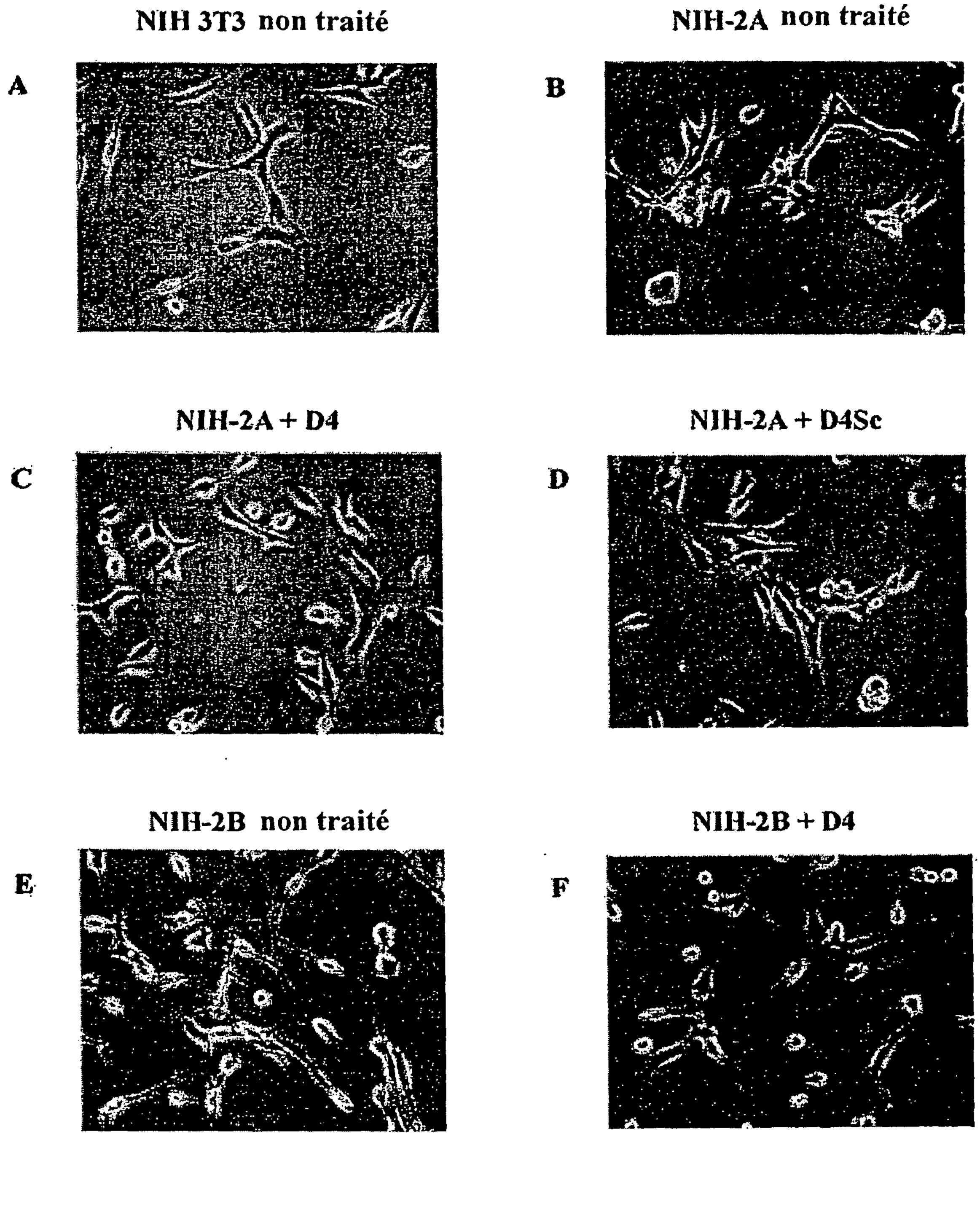
NIH 3T3 non traité
A
NIH-2A non traité
B
NIH-2A + D4
C
NIH-2A + D4Sc
D
NIH-2B non traité
E
NIH-2B + D4
F

FIGURE 9

FIGURE 10

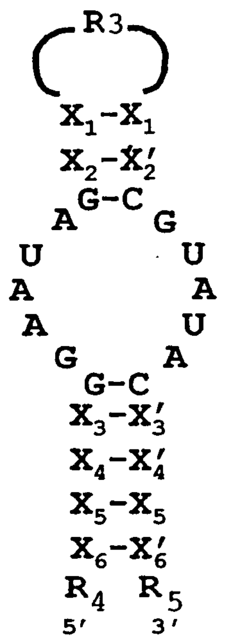


Formule II

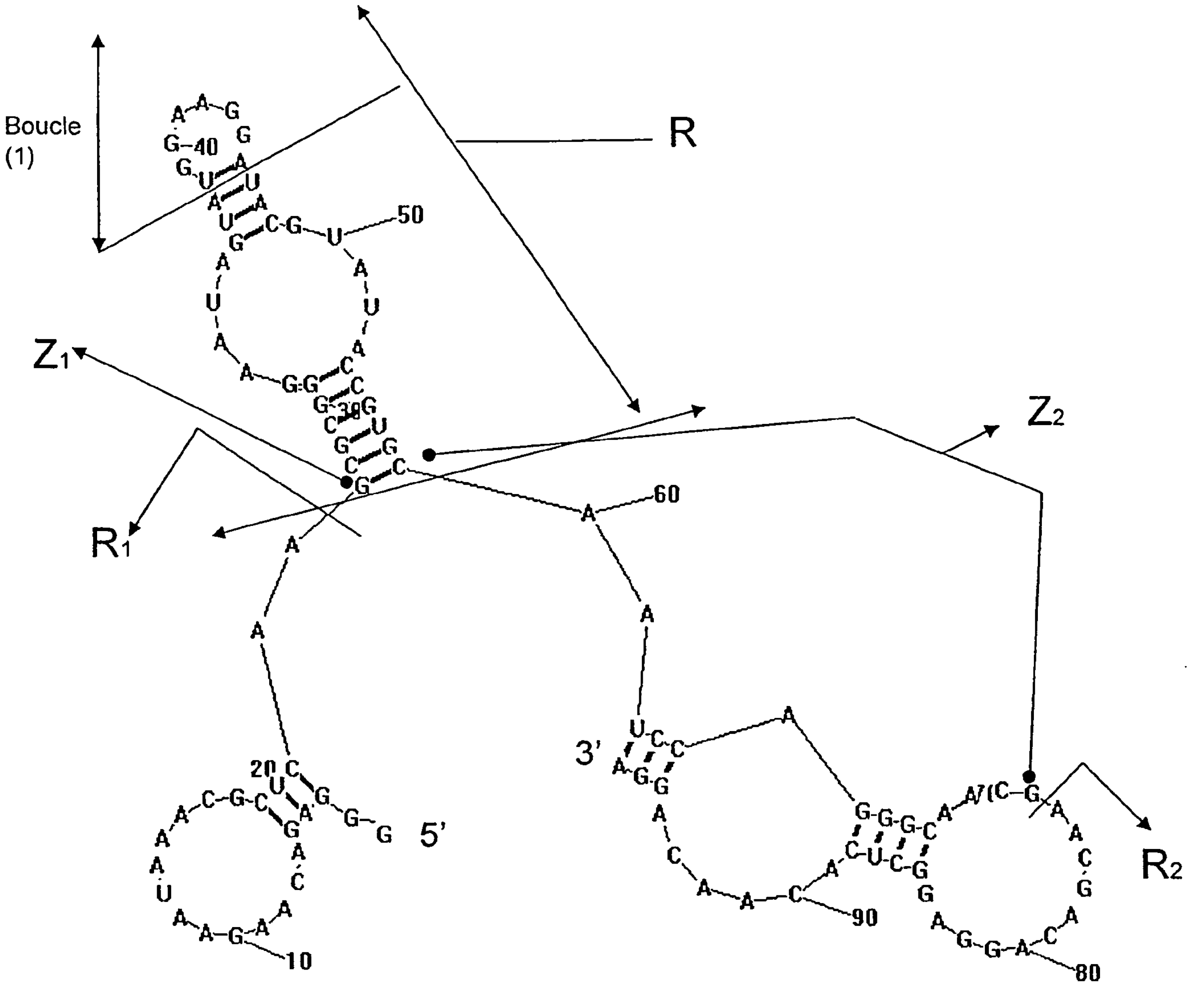


D4 - 97nt ENERGY -17.5

FIGURE 11

5' → 3'

$R_1 + Z_1 = R_4$

5' → 3'

$R_2 + Z_2 = R_5$

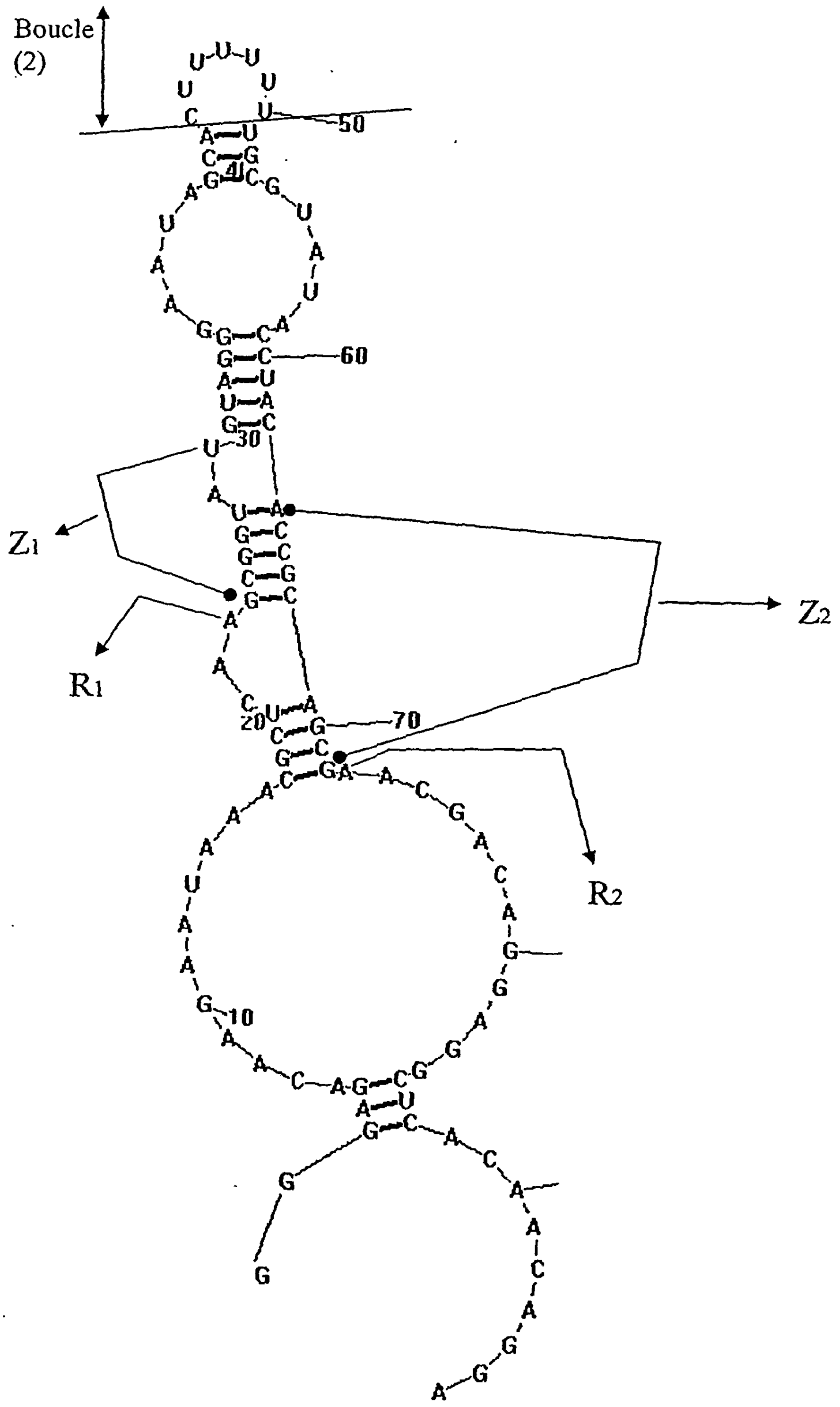


D24 - 96nt ENERGY -24

FIGURE 12

5' → 3'
$R_1 + Z_1 = R_4$

5' → 3'
$R_2 + Z_2 = R_5$

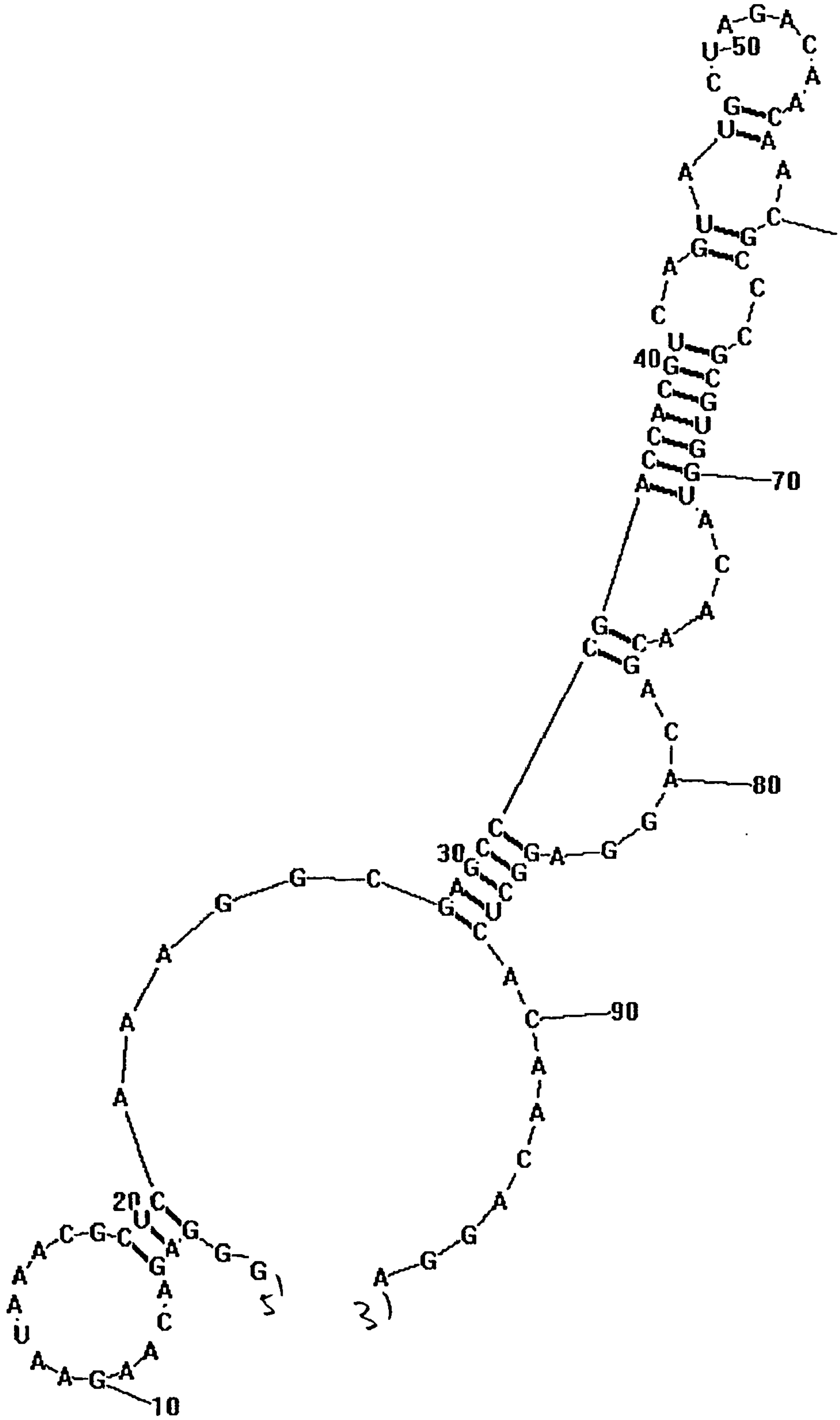


D30 - 97nt ENERGY -17

FIGURE 13

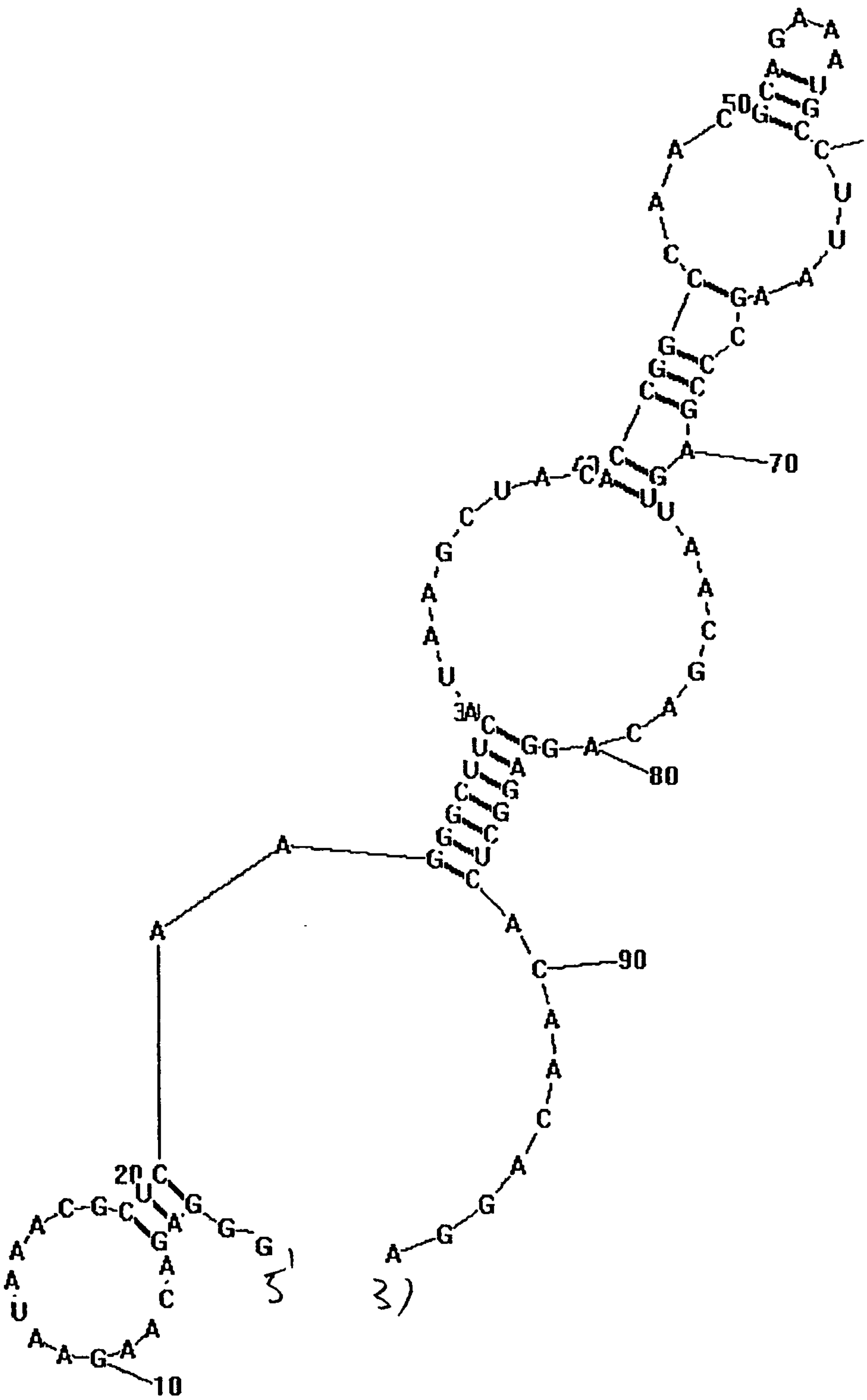

D12 - 97nt ENERGY -19.1

FIGURE 14

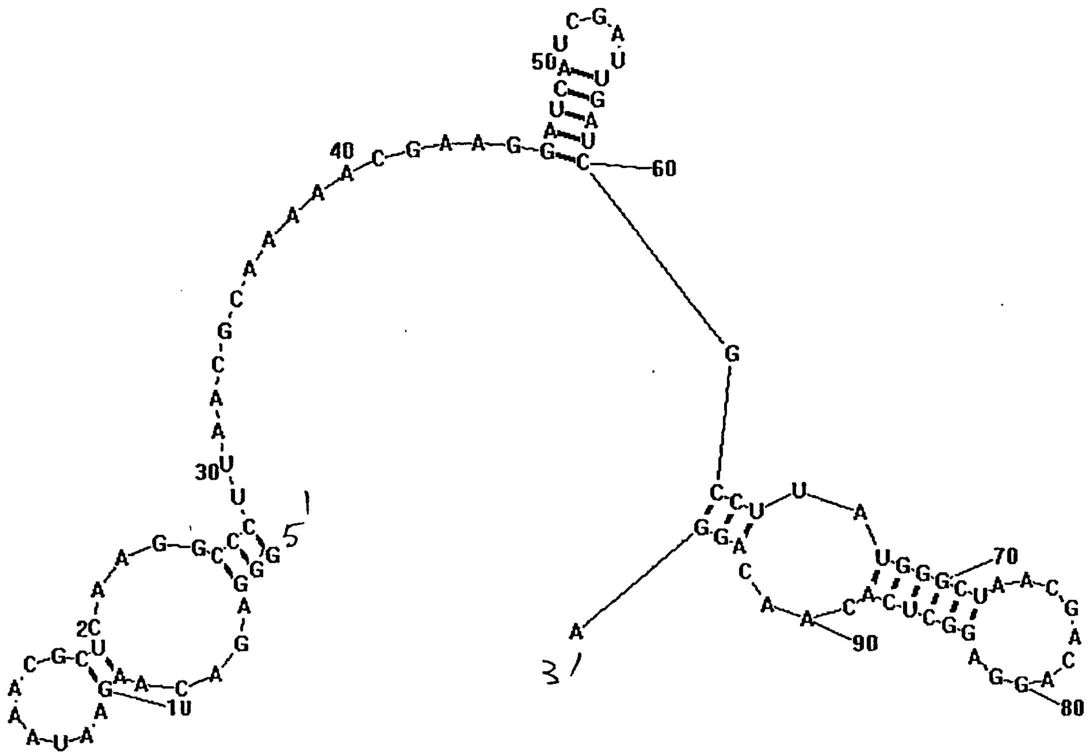


D71 - 96nt ENERGY -16.6

FIGURE 15

Fig. 16: criblage d'aptamères interagissant avec RET sur les cellules PC12 MEN 2A par fixation compétitive avec l'aptamère D4

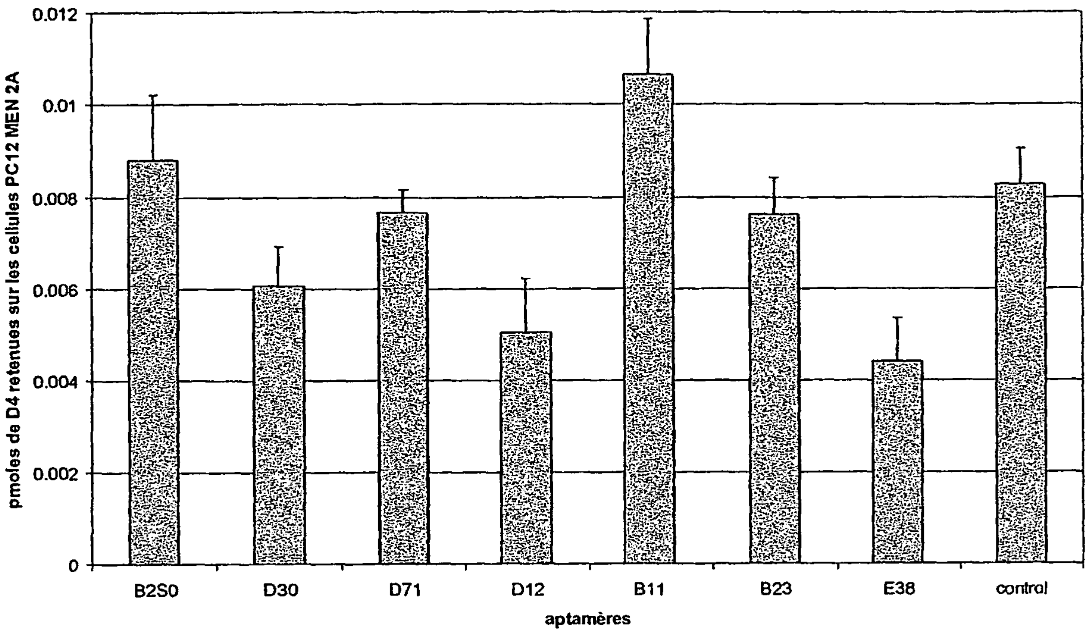


Fig 17 : Fixation compétitive de l'aptamère E38 sur les cellules PC12 MEN 2A en présence d'une concentration croissante d'aptamère D4

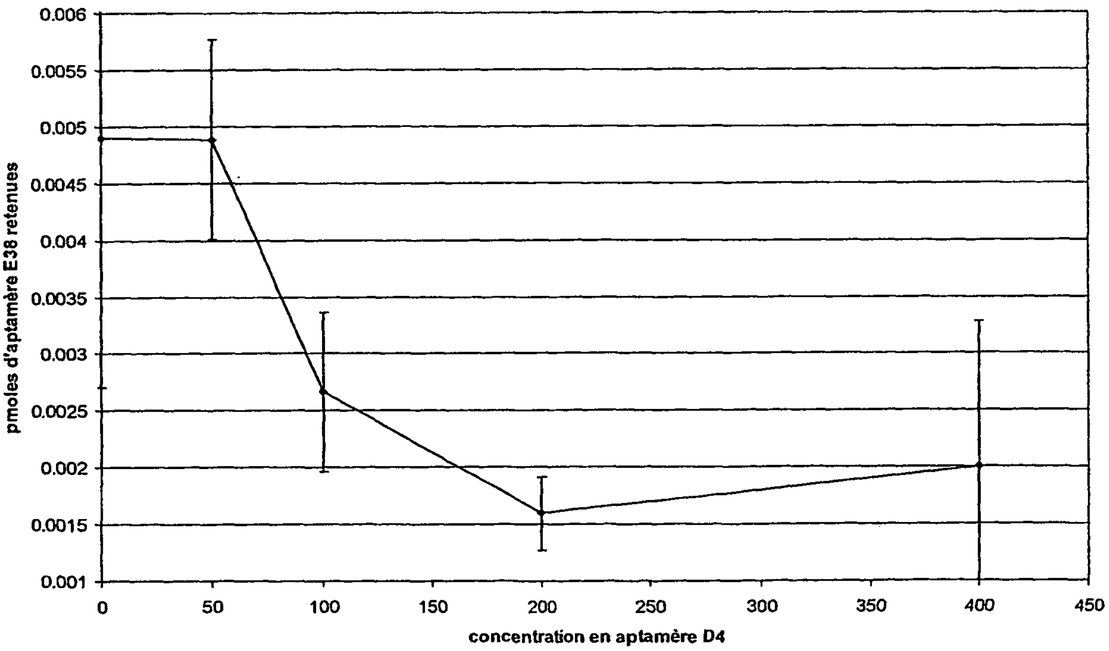

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5270163 A
- US 5475096 A
- US 5496938 A
- US 5567588 A
- US 5580737 A
- US 5637459 A
- US 5660985 A
- US 5683867 A
- US 5707796 A
- US 5763177 A
- US 5789157 A
- US 6232071 B


### Littérature non-brevet citée dans la description


- **Blume-Jensen P. et al.** *Nature,* 2001, vol. 411, 355-365
- **Jhiang SM.** *Oncogene,* 2000, vol. 19, 5590-5597
- **Califano D et al.** *PNAS,* 1996, vol. 93, 7933-7937
- **D.H. Mathews et al.** *J. Mol. Biol.,* 1999, vol. 288, 911-940
- **D.H. Mathews et al.** *Journal of Molecular Biology,* 1999, vol. 288, 911-940
- **D'Alessio A. et al.** *Endocrinology,* 2003, vol. 144 (10), 4298-4305
- **Cerchia L. et al.** *Biochem. J.,* 2003, vol. 372, 897-903
- **Tuerk C et al.** *Science,* 1990, vol. 249 (4968), 505-510
- **Ellington AD et al.** *Nature,* 1990, vol. 346 (6287), 818-22
- **Padilla, R et al.** *Nucleic Acids Res,* 1999, vol. 27 (6), 1561-1563
- **Padilla, R et al.** *N.A.R.,* 1999
- **Bartel DP et al.** *Science,* 1993, vol. 261 (5127), 1411-8
- **Santoro M et al.** *Science,* 1995, vol. 267 (5196), 381-383
- **Colucci-D'Amato et al.** *J. Biol. Chem.,* 2000, vol. 275, 19306-19314
- **Carlomagno F et al.** *Endocrinology,* 1998, vol. 139 (8), 3613-3619
- **Salton SR.** *Mt Sinai J Med.,* 2003, vol. 70 (2), 93-100
- **Santoro et al.** *Science,* 1995